# EUROPÄISCHE PATENTSCHRIFT

(11) **EP 2 406 215 B1**
(45) Veröffentlichungstag und Bekanntmachung des Hinweises auf die Patenterteilung: **16.04.2014**
(21) Anmeldenummer: 10706154.1
(22) Anmeldetag: 24.02.2010
(51) Int. Cl.: C07D 209/46, C07D 237/14, C07D 273/04, A61K 31/4035, A61K 31/50, A61K 31/5395, A61P 9/00

(54) **OXO-HETEROCYCLISCH SUBSTITUIERTE ALKYLCARBONSÄUREN UND IHRE VERWENDUNG**
OXO-HETEROCYCLICALLY SUBSTITUTED ALKYL CARBOXYLIC ACIDS AND USE THEREOF
ACIDES ALKYLCARBOXYLIQUES À SUBSTITUTION OXO-HÉTÉROCYCLIQUE ET LEUR UTILISATION

(30) Priorität: 09.03.2009 DE 102009012314
(43) Veröffentlichungstag der Anmeldung: 18.01.2012
(73) Patentinhaber: Bayer Intellectual Property GmbH, 40789 Monheim (DE)
(72) Erfinder: LAMPE, Thomas, 40545 Düsseldorf (DE); HAHN, Michael, 40764 Langenfeld (DE); STASCH, Johannes-Peter, 42651 Solingen (DE); SCHLEMMER, Karl-Heinz, 42113 Wuppertal (DE); WUNDER, Frank, 42117 Wuppertal (DE); HEITMEIER, Stefan, 42489 Wülfrath (DE); GRIEBENOW, Nils, 41541 Dormagen (DE); EL SHEIKH, Sherif, 45219 Essen (DE); LI, Volkhart, Min-Jian, 42553 Velbert (DE); BECKER, Eva-Maria, 42327 Wuppertal (DE); STOLL, Friederike, 40215 Düsseldorf (DE); KNORR, Andreas, 40699 Erkrath (DE)
(74) Vertreter: BIP Patents
(86) Internationale Anmeldenummer: PCT/EP2010/001124
(87) Internationale Veröffentlichungsnummer: WO 2010/102717

(56) Entgegenhaltungen:
- EP-A1- 0 608 709
- WO-A1-00/64888
- WO-A1-2009/127338
- DE-A1- 19 821 483
- EVGENOV OLEG V ET AL: "NO-independent stimulators and activators of soluble guanylate cyclase: discovery and therapeutic potential" NATURE REVIEWS. DRUG DISCOVERY, NATURE PUBLISHING GROUP, GB LNKD- DOI:10.1038/NRD2038, Bd. 5, Nr. 9, 1. September 2006 (2006-09-01), Seiten 755-768, XP002534424 ISSN: 1474-1776 in der Anmeldung erwähnt

## Beschreibung

Die vorliegende Anmeldung betrifft neue Alkylcarbonsäuren mit einer oxo-substituierten azaheterocyclischen Partialstruktur, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat YC-1 wurde erstmals ein NO-unabhängiger, jedoch Häm-abhängiger Stimulator der sGC beschrieben [Evgenov et al., *ibid*.]. Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521. Diese Verbindungen bilden gemeinsam mit den kürzlich publizierten, strukturell diversen Substanzen CMF-1571 und A-350619 die neue Klasse der sGC-Stimulatoren [Evgenov et al., *ibid*.]. Gemeinsames Charakteristikum dieser Substanzklasse ist eine NO-unabhängige und selektive Aktivierung der hämhaltigen sGC. Darüber hinaus zeigen die sGC-Stimulatoren in Kombination mit NO einen synergistischen Effekt auf die sGC-Aktivierung, welcher auf einer Stabilisierung des Nitrosyl-Häm-Komplexes basiert. Die genaue Bindungsstelle der sGC-Stimulatoren an der sGC ist bis heute Gegenstand der Diskussion. Entfernt man von der löslichen Guanylatcyclase die HämGruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid*.].

Darüber hinaus wurden NO- und Häm-unabhängige sGC-Aktivatoren, mit BAY 58-2667 als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid*.; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Pro-pionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552].

Die in der vorliegenden Erfindung beschriebenen Verbindungen sind nun ebenfalls wie die Verbindungen der WO 2009127338 in der Lage, die Häm-freie Form der löslichen Guanylatcyclase zu aktivieren. Dies wird auch dadurch belegt, dass diese neuartigen Aktivatoren einerseits am Häm-haltigen Enzym keine synergistische Wirkung mit NO zeigen und andererseits sich ihre Wirkung nicht durch den Häm-abhängigen Inhibitor der löslichen Guanylatcyclase, 1*H*-1,2,4-Oxadiazolo[4,3-a]chinoxalin-1-on (ODQ), blockieren lässt, sondern durch diesen sogar potenziert wird [vgl. O.V. Evgenov et al., Nature Rev. Drug Disc. 5 (2006), 755; J.P. Stasch et al., J. Clin. Invest. 116 (2006), 2552].

Aufgabe der vorliegenden Erfindung war somit die Bereitstellung neuer Verbindungen, welche in der oben beschriebenen Weise als Aktivatoren der löslichen Guanylatcyclase wirken und als solche insbesondere zur Behandlung und zur Prävention kardiovaskulärer Erkrankungen eingesetzt werden können.

Die Verbindungen der vorliegenden Erfindung zeichnen sich strukturell durch eine terminale Alkylcarbonsäure-Gruppierung aus, welche auf die im Folgenden dargestellte Weise mit einem oxo-substituierten Aza-Heterocyclus als Kopfgruppe verknüpft ist.

In EP 0 719 763-A1, EP 0 779 279-A1 und EP 0 802 192-A1 werden verschiedene Phenylacetamid-Derivate mit azaheterocyclischer Partialstruktur als Apolipoprotein B-Inhibitoren zur Behandlung von Atherosklerose und koronaren Herzerkrankungen beschrieben, und in EP 0 608 709-A1 werden 2-Oxochinolinylmethyl-substituierte Phenylacetamide als Angiotensin II-antagonisten zur Behandlung von arterieller Hypertonie und Atherosklerose offenbart. In EP 0 842 943-A2, EP 0 842 944-A2, EP 0 842 945-A2, EP 0 918 059-A1 und WO 99/60015-A1 werden unter anderem oxo-heterocyclisch substituierte Alkylcarbonsäuren als VLA-4-Antagonisten und Inhibitoren der Leukozyten-Adhäsion beansprucht. Ferner werden in WO 01/57002-A1 bestimmte kondensierte Azol-Derivate als hypoglykämisch wirksame Agentien beschrieben.

Gegenstand der vorliegenden Erfindung sind Verbindungen der allgemeinen Formel (I) 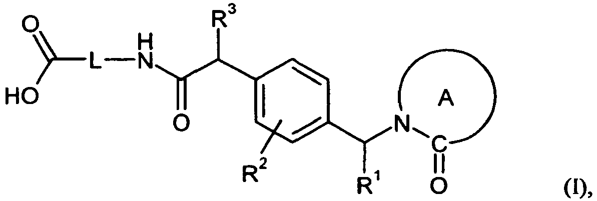 in welcher
der Ring A für einen N-verknüpften 5- bis 7-gliedrigen, gesättigten oder partiell ungesättigten, oxo-substituierten Azaheterocyclus steht,
der (*i*) ein oder zwei weitere Heteroatome aus der Reihe N, O und/oder S als Ringglieder enthalten kann,
der (*ii*) mit einem Rest ausgewählt aus der Reihe Fluor, Chlor, (C₁-C₆)-Alkyl, Trifluormethyl, (C₃-C₇)Cycloalkyl, 4- bis 7-gliedriges Heterocyclyl und Phenyl substituiert ist oder der benzo-anelliert ist,
wobei der Phenyl-Substituent und der anellierte Phenylring ihrerseits bis zu zweifach, gleich oder verschieden, mit einem Rest ausgewählt aus der Reihe Halogen, Cyano, (C₁-C₄)-Alkyl, (C₂-C₄)-Alkenyl, Trifluormethyl, (C₁-C₄)-Alkoxy und Trifluormethoxy substituiert sein können,
und
der (*iii*) darüber hinaus bis zu zweifach, gleich oder verschieden, mit weiteren Resten ausgewählt aus der Reihe Fluor, Chlor, (C₁-C₆)-Alkyl, Trifluormethyl, Oxo, (C₃-C₇)-Cycloalkyl, 4- bis 7-gliedriges Heterocyclyl und Phenyl substituiert sein kann,
wobei Phenyl seinerseits bis zu zweifach, gleich oder verschieden, mit einem Rest ausgewählt aus der Reihe Halogen, Cyano, (C₁-C₄)-Alkyl, (C₂-C₄)-Alkenyl, Trifluormethyl, (C₁-C₄)-Alkoxy und Trifluormethoxy substituiert sein kann,
- R¹: für Wasserstoff, (C₁-C₄)-Alkyl oder Cyclopropyl steht,
- R²: für Wasserstoff, Halogen, Cyano, (C₁-C₄)-Alkyl oder Trifluormethyl steht,
- R³: für (C₃-C₆)-Alkyl oder (C₃-C₆)-Alkenyl, welche jeweils mit Cyano, (C₁-C₄)-Alkoxy oder Trifluormethoxy und bis zu sechsfach mit Fluor substituiert sein können,
oder
für (C₃-C₇)-Cycloalkyl oder (C₃-C₇)-Cycloalkenyl, welche jeweils bis zu zweifach, gleich oder verschieden, mit (C₁-C₄)-Alkyl, Trifluormethyl und (C₁-C₄)-Alkoxy sowie bis zu vierfach mit Fluor substituiert sein können,
oder
für Oxetanyl, Tetrahydrofuranyl oder Tetrahydropyranyl steht,
und
- L: für geradkettiges (C₃-C₇)-Alkandiyl oder (C₃-C₇)-Alkendiyl steht, welche jeweils bis zu vierfach, gleich oder verschieden, mit einem Rest R⁴ substituiert sein können, worin
R⁴ Fluor, Trifluormethyl oder (C₁-C₄)-Alkyl bedeutet
oder
zwei an dasselbe Kohlenstoffatom gebundene Reste R⁴ miteinander verknüpft sind und zusammen mit diesem Kohlenstoffatom einen (C₃-C₆)-Cycloalkan-1,1-diyl-Ring bilden,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem beschreibt die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Insbesondere beschreibt die vorliegende Erfindung hydrolysierbare Ester-Derivate der erfindungsgemäßen Carbonsäuren der Formel (I). Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkylester, in welchen die Alkylgruppe geradkettig oder verzweigt sein kann, bevorzugt. Besonders bevorzugt sind Methyl-, Ethyl- oder *tert*.-Butylester.

Im Rahmen der vorliegenden Erfindung haben die Substituenten, soweit nicht anders spezifiziert, die folgende Bedeutung:

(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert*.-Butyl, *n*-Pentyl, 2-Pentyl, 3-Pentyl, *n*-Hexyl, 2-Hexyl und 3-Hexyl.

(C₃-C₆)-Alkyl steht im Rahmen der Erfindung für einen geradkettigen oder verzweigten Alkylrest mit 3 bis 6 Kohlenstoffatomen. Bevorzugt ist ein geradkettiger oder verzweigter Alkylrest mit 3 bis 5 Kohlenstoffatomen. Beispielhaft und vorzugsweise seien genannt: *n*-Propyl, Isopropyl, *n*-Butyl, *iso*-Butyl, *sec*.-Butyl, *tert*.-Butyl, *n*-Pentyl, 2-Pentyl, 3-Pentyl, *n*-Hexyl, 2-Hexyl und 3-Hexyl.

(C₃-C₆)-Alkenyl und (C₂-C₄)-Alkenyl stehen im Rahmen der Erfindung für einen geradkettigen oder verzweigten Alkenylrest mit einer Doppelbindung und 3 bis 6 bzw. 2 bis 4 Kohlenstoffatomen. Bevorzugt ist ein geradkettiger oder verzweigter Alkenylrest mit 3 bis 5 Kohlenstoffatomen bzw. ein geradkettiger Alkenylrest mit 2 oder 3 Kohlenstoffatomen. Beispielhaft und vorzugsweise seien genannt: Vinyl, Allyl, Isopropenyl, *n*-But-2-en-1-yl, 2-Methylprop-2-en-1-yl und *n*-But-3-en-1-yl.

(C₃-C₇)-Alkandiyl und (C₃-C₆)-Alkandiyl stehen im Rahmen der Erfindung für einen geradkettigen, α,ω-divalenten Alkylrest mit 3 bis 7 bzw. 3 bis 6 Kohlenstoffatomen. Beispielhaft und vorzugsweise seien genannt: Propan-1,3-diyl (1,3-Propylen), Butan-1,4-diyl (1,4-Butylen), Pentan-1,5-diyl (1,5-Pentylen), Hexan-1,6-diyl (1,6-Hexylen) und Heptan-1,7-diyl (1,7-Heptylen).

(C₃-C₇)-Alkendiyl und C₃-C₆)-Alkendiyl stehen im Rahmen der Erfindung für einen geradkettigen, α,ω-divalenten Alkenylrest mit 3 bis 7 bzw. 3 bis 6 Kohlenstoffatomen und einer Doppelbindung. Beispielhaft und vorzugsweise seien genannt: Propen-1,3-diyl, But-2-en-1,4-diyl, Pent-2-en-1,5-diyl, Hex-2-en-1,6-diyl, Hex-3-en-1,6-diyl, Hept-2-en-1,7-diyl und Hept-3-en-1,7-diyl.

(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy und *tert*.-Butoxy.

(C₃-C₇)-Cycloalkyl und (C₃-C₆)-Cycloalkyl stehen im Rahmen der Erfindung für eine monocyclische, gesättigte Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.

(C₃-C₇)-Cycloalkenyl und C₄-C₆)-Cycloalkenyl stehen im Rahmen der Erfindung für eine monocyclische Cycloalkylgruppe mit 3 bis 7 bzw. 4 bis 6 Ring-Kohlenstoffatomen und einer Ring-Doppelbindung. Bevorzugt ist ein Cycloalkenylrest mit 4 bis 6, besonders bevorzugt mit 5 oder 6 Kohlenstoffatomen. Beispielhaft und vorzugsweise seien genannt: Cyclopropenyl, Cyclobutenyl, Cyclopentenyl, Cyclohexenyl und Cycloheptenyl.

(C₃-C₆)-Cycloalkan-1,1-diyl steht im Rahmen der Erfindung für eine 1,1-divalente monocyclische, gesättigte Cycloalkylgruppe mit 3 bis 6 Kohlenstoffatomen. Beispielhaft und vorzugsweise seien genannt: Cyclopropan-1,1-diyl, Cyclobutan-1,1-diyl, Cyclopentan-1,1-diyl und Cyclohexan-1,1-diyl.

4- bis 7-gliedriges Heterocyclyl und 4- bis 6 gliedriges Heterocyclyl stehen im Rahmen der Erfindung für einen monocyclischen, gesättigten Heterocyclus mit insgesamt 4 bis 7 bzw. 4 bis 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Bevorzugt ist 4-bis 6-gliedriges Heterocyclyl mit ein oder zwei Ring-Heteroatomen aus der Reihe N und/oder O. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.

Halogen schließt im Rahmen der Erfindung Fluor, Chlor, Brom und Iod ein. Bevorzugt sind Chlor, Fluor oder Brom, besonders bevorzugt Fluor oder Chlor.

Ein Oxo-Substituent steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Besonders bevorzugt ist die Substitution mit einem oder zwei Substituenten.

Gegenstand der vorliegenden Erfindung sind insbesondere Verbindungen der Formel (I), in welcher

der Ring A für einen oxo-substituierten Azaheterocyclus der Formel 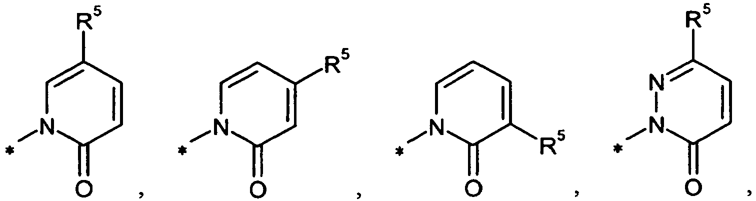 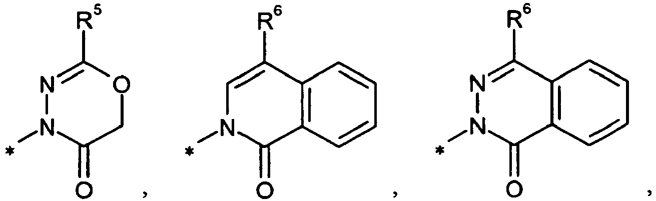 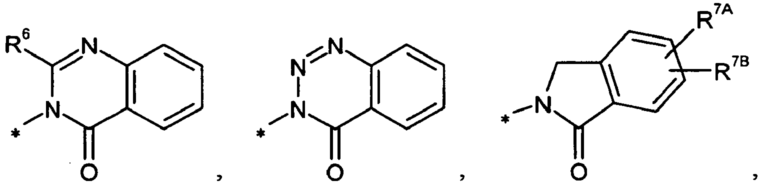 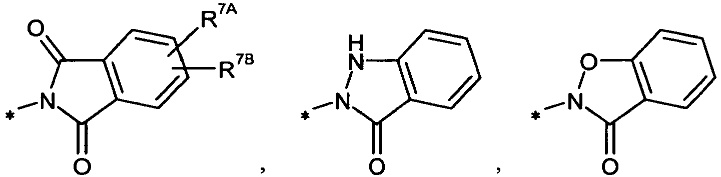 oder 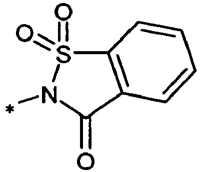 steht, worin
- *: die Verknüpfungsposition mit dem restlichen Teil des Moleküls kennzeichnet,
- R⁵: Chlor, (C₁-C₆)-Alkyl, Trifluormethyl, (C₃-C₆)-Cycloalkyl, 4- bis 6-gliedriges Heterocyclyl oder Phenyl bedeutet, wobei Phenyl seinerseits bis zu zweifach, gleich oder verschieden, mit einem Rest ausgewählt aus der Reihe Fluor, Chlor, Brom, Cyano, (C₁-C₄)-Alkyl, Vinyl, Trifluormethyl, (C₁-C₄)-Alkoxy und Trifluormethoxy substituiert sein kann,
- R⁶: Wasserstoff bedeutet oder die oben genannte Bedeutung von R⁵ hat
und
R^{7A} und R^{7B} unabhängig voneinander Wasserstoff, Fluor oder Chlor bedeuten,
sowie ihre Salze, Solvate und Solvate der Salze.

Bevorzugt im Rahmen der vorliegenden Erfindung sind Verbindungen der Formel (I), in welcher der Ring A für einen oxo-substituierten Azaheterocyclus der Formel 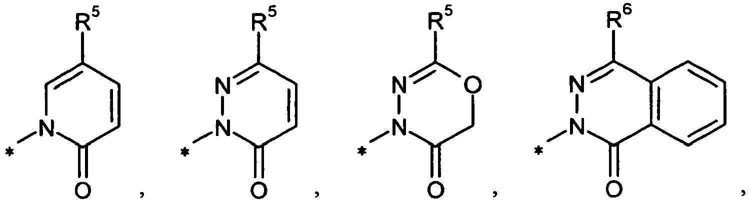 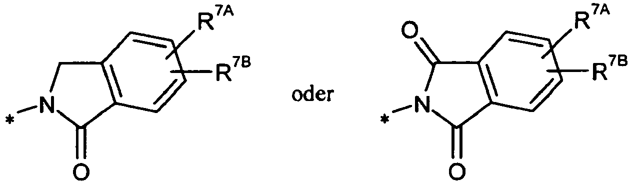 steht, worin
- *: die Verknüpfungsposition mit dem restlichen Teil des Moleküls kennzeichnet,
- R⁵: Chlor, (C₁-C₆)-Alkyl, Trifluormethyl, (C₃-C₆)-Cycloalkyl oder Phenyl bedeutet, wobei Phenyl seinerseits bis zu zweifach, gleich oder verschieden, mit einem Rest ausgewählt aus der Reihe Fluor, Chlor, Cyano, (C₁-C₄)-Alkyl, Trifluormethyl, (C₁-C₄)-Alkoxy und Trifluormethoxy substituiert sein kann,
- R⁶: Wasserstoff bedeutet oder die zuvor genannte Bedeutung von R⁵ hat
und
R^{7A} und R^{7B} unabhängig voneinander Wasserstoff, Fluor oder Chlor bedeuten,
- R¹: für Wasserstoff oder (C₁-C₄)-Alkyl steht,
- R²: für Wasserstoff, Fluor, Chlor oder Trifluormethyl steht,

- R³: für (C₃-C₆)-Alkyl oder (C₃-C₆)-Alkenyl, welche jeweils mit Cyano, Methoxy, Ethoxy oder Trifluormethoxy und bis zu sechsfach mit Fluor substituiert sein können,
oder
für (C₃-C₆)-Cycloalkyl oder (C₄-C₆)-Cycloalkenyl, welche jeweils bis zu zweifach, gleich oder verschieden, mit Methyl, Ethyl und Trifluormethyl sowie bis zu vierfach mit Fluor substituiert sein können,
oder
für Oxetanyl steht,
und
- L: für geradkettiges (C₃-C₆)-Alkandiyl oder (C₃-C₆)-Alkendiyl steht, welche jeweils bis zu vierfach, gleich oder verschieden, mit einem Rest R⁴ substituiert sein können, worin
R⁴ Fluor, Trifluormethyl, Methyl oder Ethyl bedeutet oder
zwei an dasselbe Kohlenstoffatom gebundene Reste R⁴ miteinander verknüpft sind und zusammen mit diesem Kohlenstoffatom einen Cyclopropan-1,1-diyl- oder Cyclobutan-1,1-diyl-Ring bilden,
sowie ihre Salze, Solvate und Solvate der Salze.

Besonders bevorzugt im Rahmen der vorliegenden Erfindung sind Verbindungen der Formel (I), in welcher
der Ring A für einen oxo-substituierten Azaheterocyclus der Formel 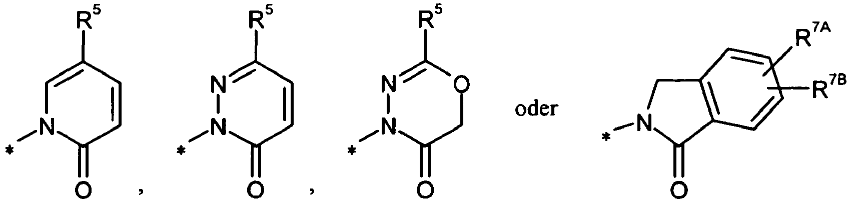 steht, worin
- *: die Verknüpfungsposition mit dem restlichen Teil des Moleküls kennzeichnet,
- R⁵: Chlor, Trifluormethyl oder Phenyl bedeutet, wobei Phenyl seinerseits bis zu zweifach, gleich oder verschieden, mit einem Rest ausgewählt aus der Reihe Fluor, Chlor, Methyl und Trifluormethyl substituiert sein kann,
und
R^{7A} und R^{7B} unabhängig voneinander Wasserstoff oder Fluor bedeuten,
- R¹: für Wasserstoff steht,
- R²: für Wasserstoff steht,
- R³: für Propan-2-yl, Butan-2-yl, Pentan-2-yl, 3,3,3-Trifluorpropan-1-yl, 1,1,1-Trifluorpropan-2-yl, 1,1,1-Trifluorbutan-2-yl, 4,4,4-Trifluorbutan-2-yl, 4,4,4-Trifluor-2-methylbutan-1-yl, Cyclopentyl oder 3,3-Difluorcyclopentyl steht,
und
- L: für geradkettiges (C₃-C₆)-Alkandiyl oder (C₃-C₆)-Alkendiyl steht, welche jeweils bis zu vierfach, gleich oder verschieden, mit einem Rest R⁴ substituiert sein können, worin
R⁴ Methyl darstellt
oder
zwei an dasselbe Kohlenstoffatom gebundene Reste R⁴ miteinander verknüpft sind und zusammen mit diesem Kohlenstoffatom einen Cyclopropan-1,1-diyl-Ring bilden,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man zunächst entweder
[A] eine Verbindung der Formel (II) 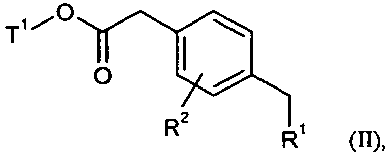 in welcher R¹ und R² die oben angegebenen Bedeutungen haben
   und
   - T¹: für (C₁-C₄)-Alkyl steht,
   in einem inerten Lösungsmittel in Gegenwart einer Base mit einer Verbindung der Formel (III)

   R³-X (III),

   in welcher R³ die oben angegebene Bedeutung hat
   und
   - X: für eine Abgangsgruppe wie beispielsweise Halogen, Mesylat, Tosylat oder Triflat steht,
   in eine Verbindung der Formel (IV) 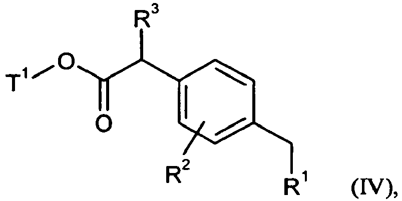 in welcher R¹, R², R³ und T¹ jeweils die oben angegebenen Bedeutungen haben,
   überführt
   oder
[B] eine Verbindung der Formel (V) 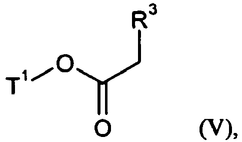 in welcher R³ und T¹ die oben angegebenen Bedeutungen haben,
   in einem inerten Lösungsmittel, nach Deprotonierung mittels einer Base, mit einer Verbindung der Formel (VI) 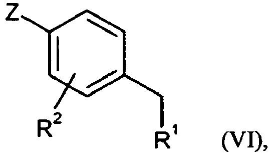 in welcher R¹ und R² die oben angegebenen Bedeutungen haben
   und
   - Z: für Chlor, Brom oder Iod steht,
   in Gegenwart eines geeigneten Palladium-Katalysators gleichfalls zu einer Verbindung der Formel (IV) 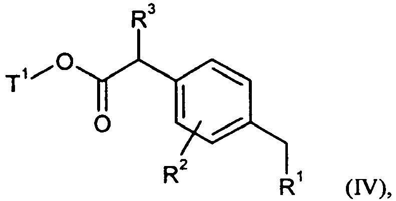 in welcher R¹, R², R³ und T¹ jeweils die oben angegebenen Bedeutungen haben,
   umsetzt,
   die Verbindung der Formel (IV) dann in einem inerten Lösungsmittel mit elementarem Brom oder mit N-Bromsuccinimid zu einer Verbindung der Formel (VII) 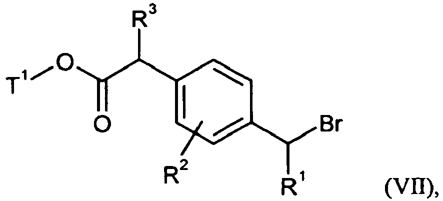 in welcher R¹, R², R³ und T¹ jeweils die oben angegebenen Bedeutungen haben,
   bromiert, anschließend in einem inerten Lösungsmittel in Gegenwart einer Base mit einer Verbindung der Formel (VIII) 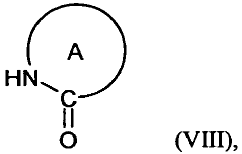 in welcher der Ring A für einen oxo-substituierten Azaheterocyclus, wie oben definiert, steht,
   zu einer Verbindung der Formel (IX) 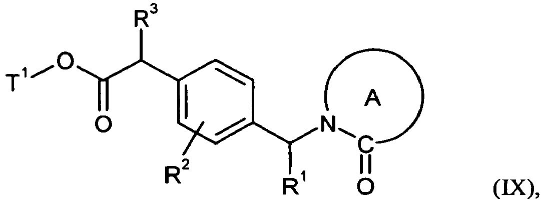 in welcher der Ring A, R¹, R², R³ und T¹ jeweils die oben angegebenen Bedeutungen haben,
   umsetzt, nachfolgend den Ester-Rest T¹ in (IX) unter basischen oder sauren Bedingungen abspaltet, die resultierende Carbonsäure der Formel (X) 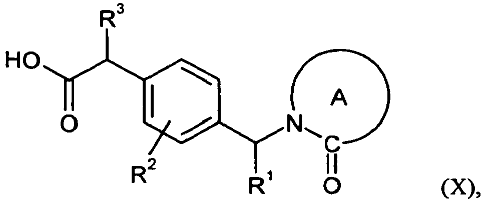 in welcher der Ring A, R¹, R² und R³ jeweils die oben angegebenen Bedeutungen haben,
   dann in einem inerten Lösungsmittel in Gegenwart eines Kondensationsmittels oder über die Zwischenstufe des entsprechenden Carbonsäurechlorids in Gegenwart einer Base mit einem Amin der Formel (XI) 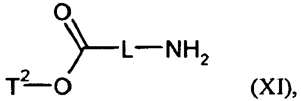 in welcher L die oben angegebene Bedeutung hat
   und
   - T²: für (C₁-C₄)-Alkyl steht,
   zu einer Verbindung der Formel (XII) 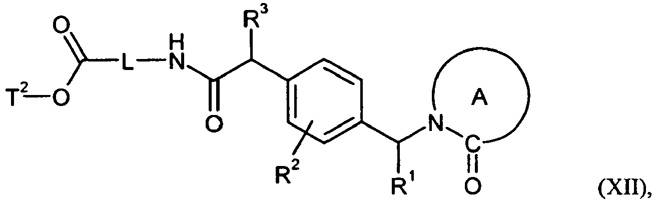 in welcher der Ring A, R¹, R², R³, L und T² jeweils die oben angegebenen Bedeutungen haben,
   kuppelt und abschließend den Ester-Rest T² in (XII) durch erneute basische oder saure Solvolyse zur Carbonsäure der Formel (I) abspaltet
   und die Verbindungen der Formel (I) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Bei der zuvor beschriebenen Reaktionssequenz kann es gegebenenfalls zweckmäßig sein, die Reihenfolge einzelner Transformationen zu vertauschen. So ist es beispielsweise möglich, die Verbindung der Formel (VII-A) [T¹ in (VII) = *tert*.-Butyl] 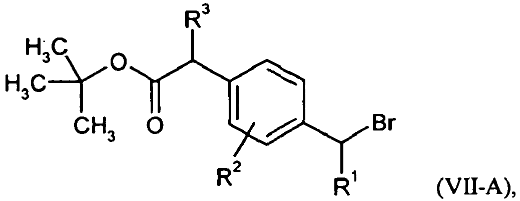 in welcher R¹, R² und R³ die oben angegebenen Bedeutungen haben,
zunächst durch Behandlung mit einer Säure in eine Carbonsäure der Formel (XIII) 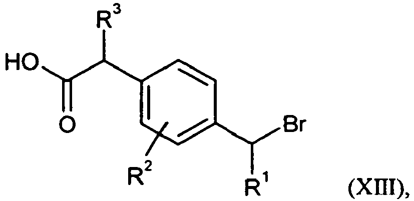 in welcher R¹, R² und R³ die oben angegebenen Bedeutungen haben,
zu überführen und diese anschließend in einem inerten Lösungsmittel in Gegenwart eines Kondensationsmittels oder über die Zwischenstufe des entsprechenden Carbonsäurechlorids in Gegenwart einer Base mit einem Amin der Formel (XI) 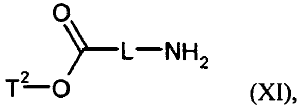 in welcher L die oben angegebene Bedeutung hat
und
T² für (C₁-C₄)-Alkyl steht,
zu einer Verbindung der Formel (XIV) 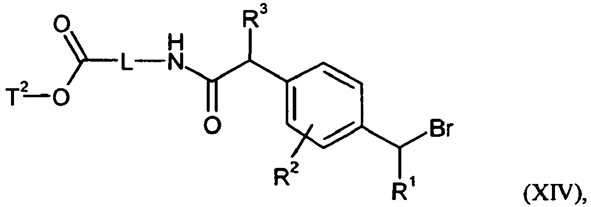 in welcher R¹, R², R³, L und T² jeweils die oben angegebenen Bedeutungen haben,
zu kuppeln, welche dann in einem inerten Lösungsmittel in Gegenwart einer Base mit einer Verbindung der Formel (VIII) 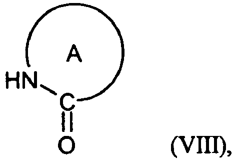 in welcher der Ring A für einen oxo-substituierten Azaheterocyclus, wie oben definiert, steht,
zu der Verbindung der Formel (XII) 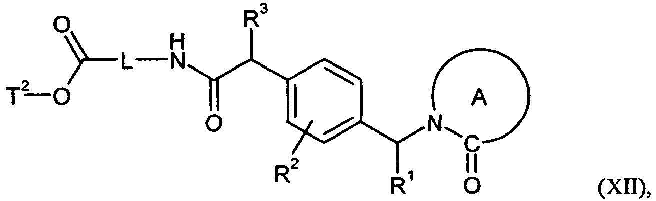 in welcher der Ring A, R¹, R², R³, L und T² jeweils die oben angegebenen Bedeutungen haben,
umgesetzt und durch Abspaltung des Ester-Restes T² in (XII) in die Carbonsäure der Formel (I) überführt wird.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (IX), (X) oder (XII) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen; vorzugsweise werden chromatographische Verfahren oder eine Trennung über diastereomere Salze verwendet.

Inerte Lösungsmittel für den Verfahrensschritt (II) + (III) → (IV) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder dipolar-aprotische Lösungsmittel wie Dimethylformamid (DMF), Dimethylsulfoxid (DMSO), *N,N'*-Dimethylpropylenharnstoff (DMPU) oder *N*-Methylpyrrolidinon (NMP). Ebenso ist es möglich, Gemische der genannten Lösungsmittel einzusetzen. Bevorzugt werden Tetrahydrofuran, Dimethylformamid oder Gemische hiervon verwendet.

Als Basen für den Verfahrensschritt (II) + (III) → (IV) eignen sich übliche starke anorganische oder organische Basen. Hierzu gehören insbesondere Alkalialkoholate wie Natrium- oder Kaliummethanolat, Natrium- oder Kaliumethanolat oder Natrium- oder Kalium-*tert*.-butylat, Alkalihydride wie Natrium- oder Kaliumhydrid, oder Amide wie Lithium-, Natrium- oder Kalium-bis-(trimethylsilyl)amid oder Lithiumdiisopropylamid. Bevorzugt wird Kalium-*tert*.-butylat, Natriumhydrid oder Lithiumdiisopropylamid verwendet.

Die Umsetzung (II) + (III) → (IV) erfolgt im Allgemeinen in einem Temperaturbereich von 100°C bis +30°C, bevorzugt bei -78°C bis 0°C.

Die Ester-Arylierung im Verfahrensschritt (V) + (VI) → (IV) wird vorzugsweise in Toluol oder Toluol/Tetrahydrofuran-Gemischen in einem Temperaturbereich von +20°C bis +100°C durchgeführt. Als Base zur Deprotonierung eignet sich für diese Reaktion insbesondere Lithium-bis(trimethylsilyl)amid. Geeignete Palladium-Katalysatoren sind beispielsweise Palladium(II)acetat oder Tris-(dibenzylidenaceton)-dipalladium in Kombination mit elektronenreichen, sterisch anspruchsvollen Phosphin-Liganden wie 2-Dicyclohexylphosphino-2'-(*N,N*-dimethylamino)biphenyl oder 2-Di-tert.-butylphosphino-2'-(*N,N*-dimethylamino)biphenyl [vgl. z.B. W.A. Moradi, S.L. Buchwald, J. Am. Chem. Soc. 123, 7996-8002 (2001)].

Die Bromierung im Verfahrensschritt (IV) → (VII) wird vorzugsweise in einem Halogenkohlenwasserstoff als Lösungsmittel, insbesondere in Dichlormethan oder Tetrachlormethan, in einem Temperaturbereich von +40°C bis +100°C durchgeführt. Als Bromierungsmittel eignen sich elementares Brom in Gegenwart von Licht sowie insbesondere *N*-Bromsuccinimid (NBS) unter Zusatz von α,α'-Azobis(isobutyronitril) (AIBN) oder Dibenzoylperoxid als Initiator [vgl. z.B. R.R. Kurtz, D.J. Houser, J. Org. Chem. 46, 202 (1981); Z.-J. Yao et al., Tetrahedron 55, 2865 (1999)].

Inerte Lösungsmittel für die Verfahrensschritte (VII) + (VIII) → (IX) und (XIV) + (VIII) → (XII) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Chlorbenzol oder Chlortoluol, oder andere Lösungsmittel wie Dimethylformamid (DMF), Dimethylsulfoxid (DMSO), *N,N*'-Dimethylpropylenharnstoff (DMPU), *N*-Methylpyrrolidinon (NMP), Acetonitril oder Pyridin. Ebenso ist es möglich, Gemische der genannten Lösungsmittel einzusetzen. Bevorzugt werden Tetrahydrofuran, Dimethylformamid oder Gemische hiervon verwendet.

Als Basen für diese Umsetzungen eignen sich die üblichen anorganischen oder organischen Basen. Hierzu gehören insbesondere Alkalihydroxide wie beispielsweise Lithium-, Natrium- oder Kaliumhydroxid, Alkalicarbonate wie Lithium-, Natrium-, Kalium- oder Cäsiumcarbonat, Alkalialkoholate wie Natrium- oder Kaliummethanolat, Natrium- oder Kaliumethanolat oder Natrium- oder Kalium-*tert*.-butylat, Alkalihydride wie Natrium- oder Kaliumhydrid, oder Amide wie Lithium-, Natrium- oder Kalium-bis-(trimethylsilyl)amid oder Lithiumdiisopropylamid. Bevorzugt wird Cäsiumcarbonat oder Natriumhydrid eingesetzt.

Die Reaktionen (VII) + (VIII) → (IX) und (XIV) + (VIII) → (XII) erfolgen im Allgemeinen in einem Temperaturbereich von -20°C bis +120°C, bevorzugt im Bereich von 0°C bis +80°C.

Die Abspaltung der Ester-Gruppen T¹ bzw. T² in den Verfahrensschritten (IX) → (X), (XII) → (I) und (VII-A) → (XIII) wird nach üblichen Methoden durchgeführt, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert.-*Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydro-furan, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von -20°C bis +100°C, bevorzugt bei 0°C bis +60°C.

Inerte Lösungsmittel für die Verfahrensschritte (X) + (XI) → (XII) und (XIII) + (XI) → (XIV) [Amid-Kupplung] sind beispielsweise Ether wie Diethylether, *tert*.-Butyl-methylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Acetonitril, Ethylacetat, Pyridin, Dimethylsulfoxid (DMSO), Dimethylformamid (DMF), *N,N'*-Dimethylpropylenharnstoff (DMPU) oder *N-*Methylpyrrolidinon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel bei diesen Kupplungsreaktionen eignen sich beispielsweise Carbodiimide wie *N,N'*-Diethyl-, *N,N'*-Dipropyl-, *N,N'*-Diisopropyl-, *N,N'*-Dicyclohexylcarbodiimid (DCC) oder *N*-(3-Dimethylaminoisopropyl)-*N'*-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-*tert*.-Butyl-5-methylisoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris-(pyrrolidino)phosphonium-hexafluorophosphat (PyBOP), *O*-(Benzotriazol-1-yl)-*N,N,N',N'*-tetramethyluronium-tetrafluoroborat (TBTU), *O*-(Benzotriazol-1-yl)-*N,N,N',N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N'*-tetramethyluronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder organische Basen wie Triethylamin, *N*-Methylmorpholin, *N*-Methylpiperidin, *N,N*-Diisopropylethylamin, Pyridin oder 4-*N,N*-Dimethylaminopyridin. Bevorzugt eingesetzt werden *O*-(7-Azabenzotriazol-1-yl)-*N,N,N',N'*-tetramethyluronium-hexafluorophosphat (HATU) oder *O*-(Benzotriazol-1-yl)-*N,N,N',N'*-tetramethyluronium-tetrafluoroborat (TBTU), jeweils in Kombination mit Pyridin oder *N,*N-Diisopropylethylamin, oder *N*-(3-Dimethylaminoisopropyl)-*N'-*ethylcarbodiimid-Hydrochlorid (EDC) in Verbindung mit 1-Hydroxybenzotriazol (HOBt) und Triethylamin.

Die Kupplungen (X) + (XI) → (XII) und (XIII) + (XI) → (XIV) werden in der Regel in einem Temperaturbereich von 0°C bis +60°C, bevorzugt bei +10°C bis +40°C durchgeführt.

Beim Einsatz eines der Verbindung (X) bzw. (XIII) entsprechenden Carbonsäurechlorids wird die Kupplung mit der Amin-Komponente (XI) in Gegenwart einer üblichen organischen Hilfsbase wie Triethylamin, *N*-Methylmorpholin, *N*-Methylpiperidin, *N,N*-Diisopropylethylamin, Pyridin, 4-*N,N-*Dimethylaminopyridin, 1,8-Diazabicyclo[5.4.0]undec-7-en (DBU) oder 1,5-Diazabicyclo[4.3.0]-non-5-en (DBN) durchgeführt. Bevorzugt wird Triethylamin oder *N,N*-Diisopropylethylamin verwendet.

Die Reaktion des Amins (XI) mit dem Carbonsäurechlorid erfolgt im Allgemeinen in einem Temperaturbereich von -20°C bis +60°C, bevorzugt im Bereich von 0°C bis +40°C.

Die Herstellung der Carbonsäurechloride selbst erfolgt auf übliche Weise durch Behandlung der Carbonsäuren (X) bzw. (XIII) mit Thionylchlorid.

Die genannten Umsetzungen können bei normalem, bei erhöhtem oder bei erniedrigtem Druck durchgeführt werden (z.B. von 0.5 bis 5 bar). Im Allgemeinen arbeitet man jeweils bei Normaldruck.

Die Verbindungen der Formeln (II), (III), (V), (VI), (VIII) und (XI) sind kommerziell erhältlich, als solche in der Literatur beschrieben oder können in Analogie zu üblichen, literaturbekannten Verfahren hergestellt werden [zu Verbindungen der Formel (XI) siehe auch nachfolgende Syntheseschemata 3-5].

Die Herstellung der erfindungsgemäßen Verbindungen kann durch die folgenden Reaktionsschemata beispielhaft veranschaulicht werden:

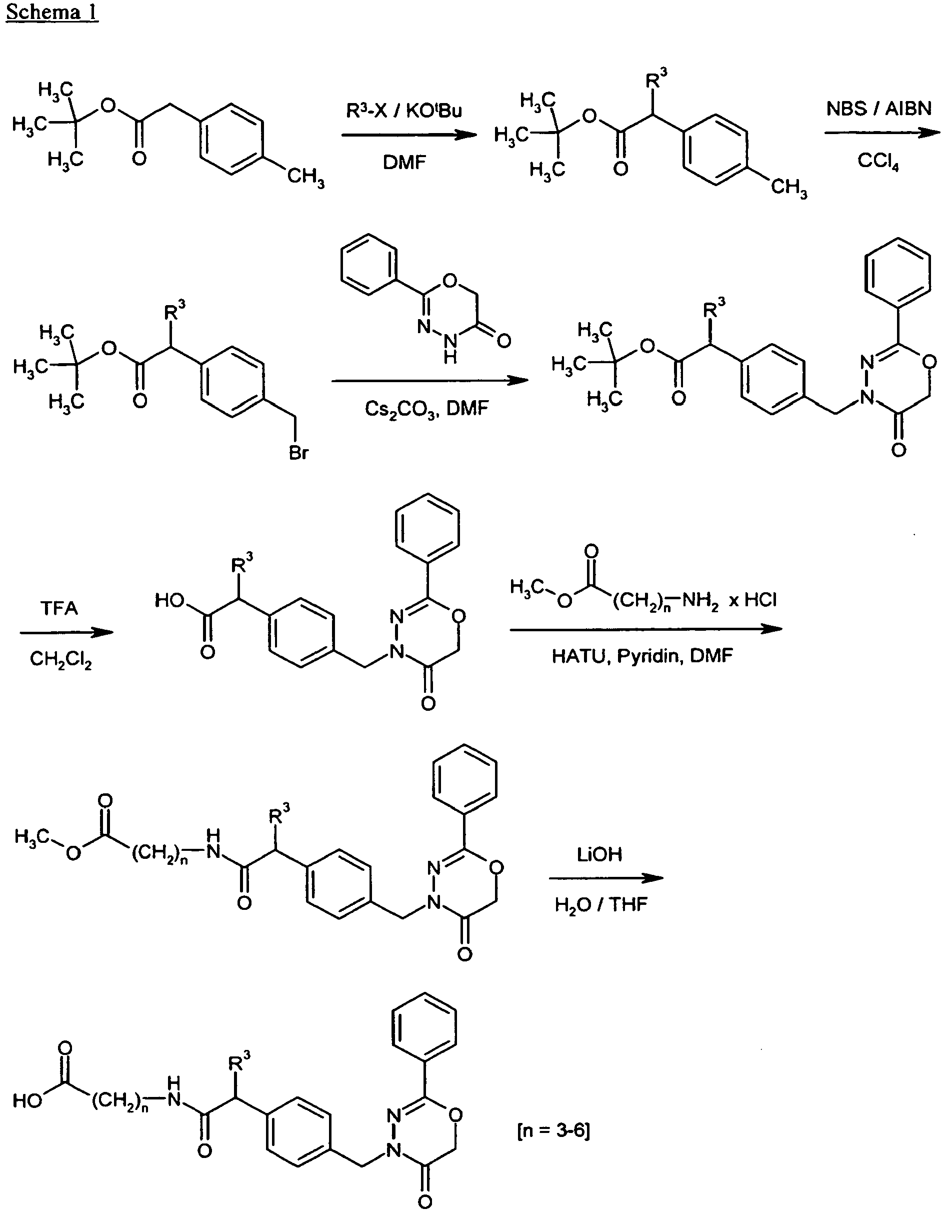

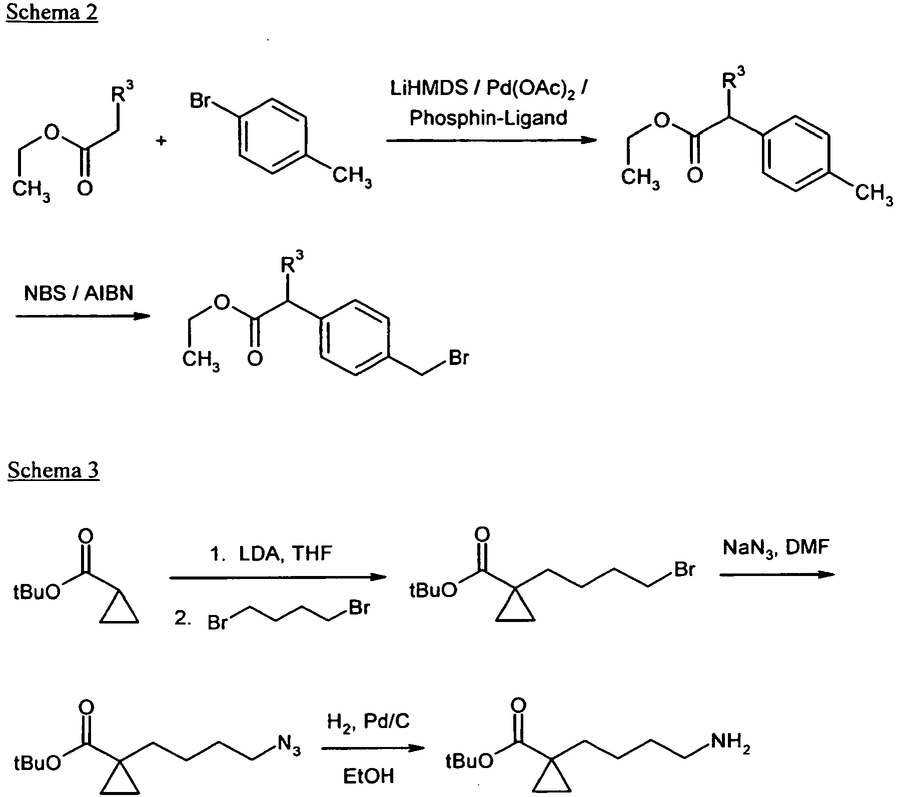

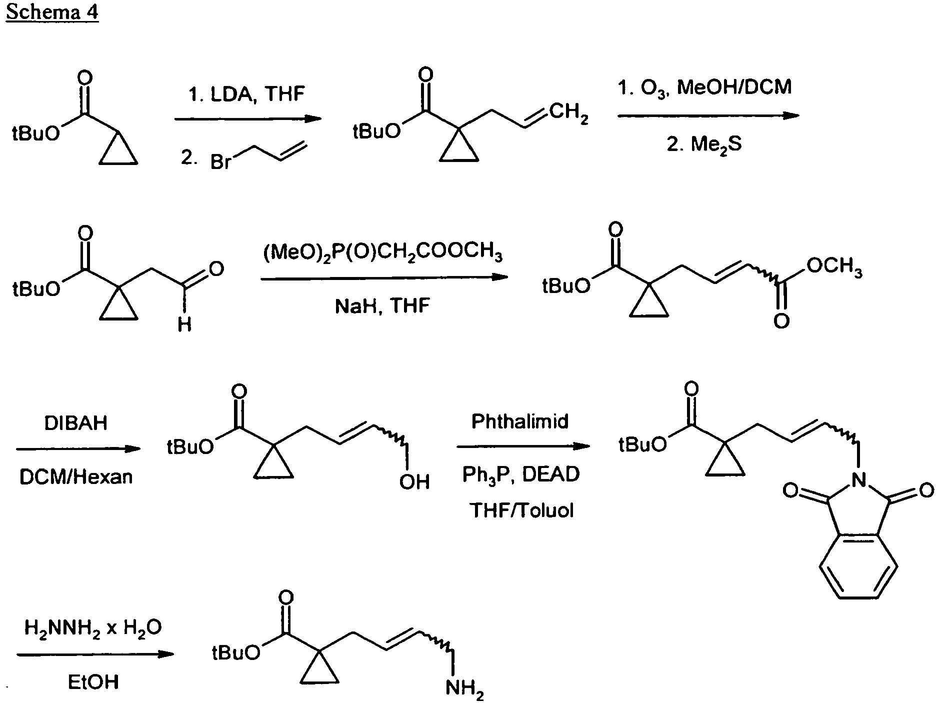

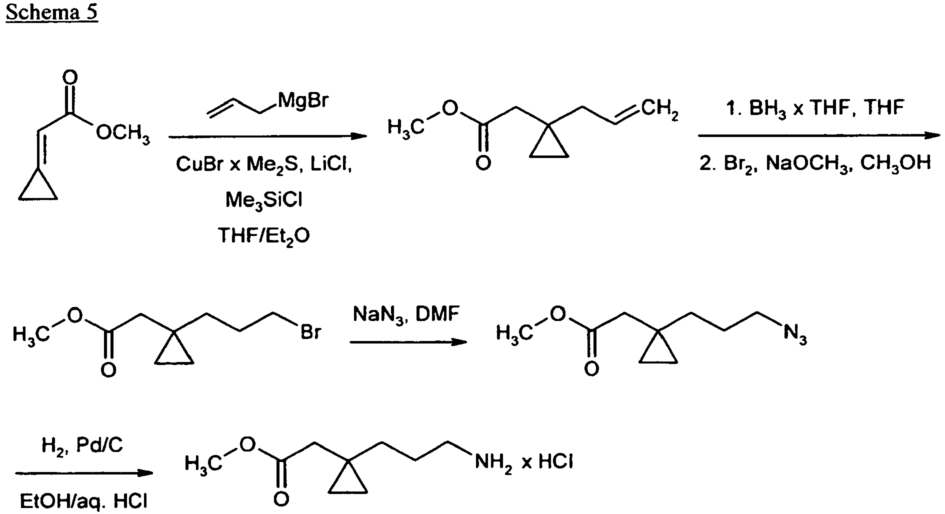

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente Aktivatoren der löslichen Guanylatcyclase dar. Sie führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte, Häm-unabhängige Aktivierung der löslichen Guanylatcyclase und einen intrazellulären cGMP-Anstieg vermittelt.

Die erfindungsgemäßen Verbindungen können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, pulmonaler Hypertonie, renaler Hypertonie, peripheren und kardialen Gefäßerkrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, zur Verhinderung von Restenosen wie nach Thrombolysetherapien, percutan-transluminalen Angioplastien (PTA), percutan-transluminalen Koronarangioplastien (PTCA) und Bypass sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz, von Osteoporose, Glaukom und Gastroparese eingesetzt werden.

Außerdem können die erfindungsgemäßen Verbindungen zur Behandlung von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, CREST-Syndrom, Erythematose, Onychomykose sowie von rheumatischen Erkrankungen verwendet werden.

Die erfindungsgemäßen Verbindungen können darüber hinaus zur Verhinderung von ischämie- und/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs insbesondere bei chirurgischen Eingriffen oder im Bereich der Transplantationsmedizin Verwendung finden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Him-Trauma, Schlaganfall, Demenz, die nach Schlaganfällen auftritt ("post stroke dementia"), post-traumatischem Schädel-Him-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel eingesetzt werden.

Denkbar ist daher die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Beschrieben wird ferner die Verwendung der erfindungsgemäßen Verbindungen in einem Verfahren zur Behandlung und/oder Prävention von Erkrankungen, insbesondere der zuvor genannten Erkrankungen.

Ebenfalls offenbart wird ein mögliches Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur verwendung in der Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- AIBN: 2,2'-Azobis-(2-methylpropionitril)
- aq.: wässrig, wässrige Lösung
- ATP: Adenosin-5'-triphosphat
- Brij^{®}: Polyethylenglycol-dodecylether
- BSA: bovines Serumalbumin
- Bsp.: Beispiel
- Bu: Butyl
- c: Konzentration
- CI: chemische Ionisation (bei MS)
- d: Tag(e)
- DBU: 1,8-Diazabicyclo[5.4.0]undec-7-en
- DC: Dünnschichtchromatographie
- DCI: direkte chemische Ionisation (bei MS)
- DCM: Dichlormethan
- de: Diastereomerenüberschuss
- DEAD: Diethylazodicarboxylat
- DIBAH: Diisobutylaluminiumhydrid
- DIEA: Diisopropylethylamin
- DMF: Dimethylformamid
- DMSO: Dimethylsulfoxid
- d. Th.: der Theorie (bei Ausbeute)
- DTT: Dithiothreitol
- EDC: *N'*-(3-Dimethylaminopropyl)-*N*-ethylcarbodiimid-Hydrochlorid
- ee: Enantiomerenüberschuss
- EI: Elektronenstoß-Ionisation (bei MS)
- ent: enantiomerenrein, Enantiomer
- eq.: Äquivalent(e)
- ESI: Elektrospray-Ionisation (bei MS)
- Et: Ethyl
- GC: Gaschromatographie
- GTP: Guanosin-5'-triphosphat
- h: Stunde(n)
- HATU: *O*-(7-Azabenzotriazol-1-yl)-*N,N,N',N'*-tetramethyluronium-Hexafluorophosphat
- HOBt: 1-Hydroxy-1*H*-benzotriazol-Hydrat
- HPLC: Hochdruck-, Hochleistungsflüssigchromatographie
- ⁱPr: Isopropyl
- KO^{t}Bu: Kalium-*tert*.-butylat
- LC-MS: Flüssigchromatographie-gekoppelte Massenspektroskopie
- LDA: Lithiumdiisopropylamid
- LiHDMS: Lithiumhexamethyldisilazid [Lithium-bis(trimethylsilyl)amid]
- Me: Methyl
- min: Minute(n)
- MS: Massenspektroskopie
- NBS: *N*-Bromsuccinimid
- NMR: Kernresonanzspektroskopie
- Pd/C: Palladium auf Aktivkohle
- PDC: Pyridiniumdichromat
- Ph: Phenyl
- Pr: Propyl
- rac: racemisch, Racemat
- R_{f}: Retentionsindex (bei DC)
- RP: reverse phase (Umkehrphase, bei HPLC)
- RT: Raumtemperatur
- Rₜ: Retentionszeit (bei HPLC)
- s.o.: siehe oben
- ^{t}Bu: *tert*.-Butyl
- TBTU: *O*-(Benzotriazol-1-yl)-*N,N,N',N'*-tetramethyluronium-Tetrafluoroborat
- TCTU: *O*-(1*H*-6-Chlorbenzotriazol-1-yl)-1,1,3,3-tetramethyluronium-Tetrafluoroborat
- TEA: Triethanolamin
- TFA: Trifluoressigsäure
- THF: Tetrahydrofuran
- UV: Ultraviolett-Spektroskopie
- v/v: Volumen zu Volumen-Verhältnis (einer Lösung)
- zus.: zusammen

### GC-MS- und LC-MS-Methoden:

### Methode 1 (GC-MS)

Instrument: Micromass GCT, GC 6890; Säule: Restek RTX-35, 1 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 2 (LC-MS)

Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 3 (LC-MS)

Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen:50°C; UV-Detektion: 210 nm.

### Methode 4 (LC-MS)

Gerätetyp MS: Micromass ZQ; Gerätetyp HPLC: HP 1100 Series; UV DAD; Säule: Phenomenex Gemini 3 µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (LC-MS)

Instrument: Waters Acquity SQD UPLC System; Säule: Waters Acquity UPLC HSS T3 1.8 µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 6 (LC-MS)

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Ausgangsverbindungen und Intermediate:

### Beispiel 1A

### Cyclopropancarbonsäure-tert.-butylester

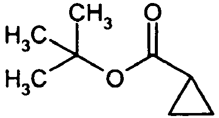

50.99 g (454.4 mmol) Kalium-*tert*.-butylat wurden in 454 ml abs. THF gelöst und auf 0°C gekühlt. Die kräftig gerührte Lösung wurde tropfenweise mit 50 g (478.3 mmol) Cyclopropancarbonsäurechlorid versetzt, so dass die Reaktionstemperatur nicht über 50°C stieg (Kühlung erforderlich). Nach Ende der Zugabe wurde die entstandene Suspension noch 30 min gerührt. Nach Abkühlen wurde die Reaktionsmischung im Vakuum auf ca. ein Drittel des Ausgangsvolumens eingeengt und dann auf 2 Liter gesättigte wässrige Ammoniumchlorid-Lösung gegeben. Nach Einstellen des pH-Wertes auf 8 durch Zugabe von gesättigter Natriumhydrogencarbonat-Lösung wird dreimal mit Diethylether extrahiert. Die vereinigten organischen Phasen wurden über Magnesiumsulfat getrocknet und im Vakuum ohne Erhitzen eingeengt (kaltes Wasserbad). Der Rückstand wurde bei ca. 85°C Badtemperatur und 42 mbar destilliert. Es wurden 43.1 g (63.2% d. Th.) der Zielverbindung als klare Flüssigkeit erhalten.
GC-MS (Methode 1): Rₜ = 1.8 min.
¹H-NMR (400 MHz, DMSO-d₆): δ = 1.52-1.48 (m, 1H), 1.41 (s, 9H), 0.82-0.72 (m, 4H).

### Beispiel 2A

### 1-(4-Brombutyl)cyclopropancarbonsäure-tert.-butylester

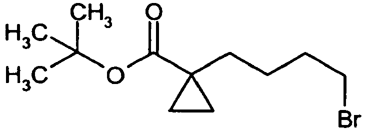

Zu einer auf -78°C gekühlten Lösung von 7.4 ml (52.7 mmol) Diisopropylamin in 20 ml abs. THF wurden 21.2 ml (52.7 mmol) einer 2.5 M Lösung von n-Butyllithium in n-Hexan getropft, wobei die Reaktionstemperatur unter -60°C gehalten wurde. Nach 30 min Rühren bei -60°C bis -70°C wurde diese Lösung zu einer auf -78°C gekühlten Lösung von 5.0 g (35.2 mmol) Cyclopropancarbonsäure-*tert*.-butylester und 15.2 g (70.3 mmol) 1,4-Dibrombutan in 20 ml abs. THF getropft. Nach Ende der Zugabe wurde die Kühlung entfernt und die Mischung unter Rühren langsam auf RT erwärmt. Nach weiteren 5 h Rühren bei RT wurde das Reaktionsgemisch auf gesättigte wässrige Ammoniumchlorid-Lösung gegeben. Es wurde dreimal mit Dichlormethan extrahiert. Die vereinigten organischen Phasen wurden über Magnesiumsulfat getrocknet und im Vakuum eingeengt. Das Produkt wurde durch Chromatographie an Silicagel gereinigt (Laufmittel Cyclohexan/Dichlormethan 50:1). Es wurden 4.62 g (44.6% d. Th.) der Zielverbindung erhalten.
MS (DCI): m/z = 294/296 (M+NH₄)⁺.
GC-MS (Methode 1): Rₜ = 4.70 min; m/z = 220 (M-C₄H₈)⁺.
¹H-NMR (400 MHz, DMSO-d₆): δ = 3.54 (t, 2H), 1.72-1.65 (m, 2H), 1.57-1.42 (m, 4H), 1.39 (s, 9H), 0.98 (m, 2H), 0.66 (m, 2H).

### Beispiel 3A

### 6-Brom-2,2-dimethylhexansäure-tert.-butylester

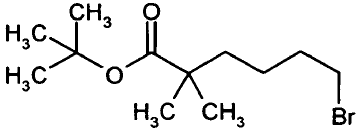

Die Titelverbindung wurde auf zu Beispiel 2A analoge Weise ausgehend von 2-Methylpropansäure-*tert*.-butylester und 1,4-Dibrombutan erhalten.
GC-MS (Methode 1): Rₜ = 4.25 min; m/z = 205 (M-75)⁺.
¹H-NMR (400 MHz, DMSO-d₆): δ = 3.54 (t, 2H), 1.81-1.72 (m, 2H), 1.49-1.39 (m, 2H), 1.40 (s, 9H), 1.35-1.28 (m, 2H), 1.08 (s, 6H).

### Beispiel 4A

### (+/-)-1-(4-Brompentyl)cyclopropancarbonsäure-tert.-butylester

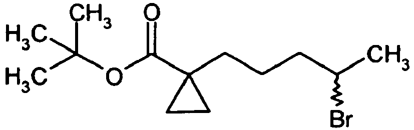

Zu einer auf -78°C gekühlten Lösung von 7.4 ml (52.7 mmol) Diisopropylamin in 20 ml abs. THF wurden 21.2 ml (52.7 mmol) einer 2.5 M Lösung von n-Butyllithium in *n*-Hexan getropft, wobei die Reaktionstemperatur unter -60°C gehalten wurde. Nach 30 min Rühren bei -60°C bis -70°C wurde diese Lösung zu einer auf -78°C gekühlten Lösung von 5.0 g (35.2 mmol) Cyclopropancarbonsäure-*tert*.-butylester und 16.2 g (70.3 mmol) 1,4-Dibrompentan in 20 ml abs. THF getropft. Nach Ende der Zugabe wurde die Kühlung entfernt und die Mischung unter Rühren langsam auf RT erwärmt. Nach weiteren 4 h Rühren bei RT wurde das Reaktionsgemisch auf gesättigte wässrige Ammoniumchlorid-Lösung gegeben. Es wurde dreimal mit Dichormethan extrahiert. Die vereinigten organischen Phasen wurden über Magnesiumsulfat getrocknet und im Vakuum eingeengt. Das Produkt wurde durch Chromatographie an Silicagel gereinigt (Laufmittel-Gradient Cyclohexan/Dichlormethan 50:1 bis 5:1). Es wurden in zwei Chargen zusammen 5.73 g (53.6% d. Th.) der Zielverbindung erhalten.
MS (DCI): m/z = 308/310 (M+NH₄)⁺.
GC-MS (Methode 1): Rₜ = 4.82 min; m/z = 234 (M-C₄H₈)⁺.
¹H-NMR (400 MHz, DMSO-d₆): δ = 4.29 (q, 1H), 1.78-1.71 (m, 2H), 1.67 (d, 3H), 1.65-1.43 (m, 4H), 1.39 (s, 9H), 0.98 (m, 2H), 0.67 (m, 2H).

### Allgemeine Vorschrift 1: Darstellung von Aziden ausgehend von aliphatischen Bromiden

Zu einer Lösung des entsprechenden Bromids in DMF (ca. 0.2 bis 1 mol/l) wird bei RT ein Überschuss an Natriumazid (ca. 4-6 eq.) gegeben. Die Suspension wird bei 50-80°C für 2-18 h kräftig gerührt. Nach Abkühlen auf RT wird die Reaktionsmischung verdünnt (z.B. mit Ethylacetat oder Dichlormethan) und nacheinander mit gesättigter Natriumhydrogencarbonat-Lösung und gesättigter Natriumchlorid-Lösung gewaschen. Nach vorsichtigem Einengen im Vakuum kann das Rohprodukt, falls erforderlich, durch Chromatographie an Silicagel gereinigt werden (typisches Laufmittelgemisch z.B. Cyclohexan/Ethylacetat 100:1 bis 10:1).

Die folgenden Beispiele wurden gemäß der Allgemeinen Vorschrift 1 hergestellt:

| **Beispiel** | **Struktur** | **Analytische Daten** |
|---|---|---|
| **5A** | 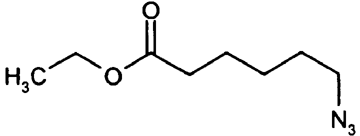 | GC-MS (Methode 1): Rₜ = 4.01 min; m/z = 157 |
| | | MS (DCI): m/z = 203 (M+NH₄)⁺, 186 (M+H)⁺ |
| | | ¹H-NMR (400 MHz, DMSO-d₆): δ = 4.05 (q, 2H), 3.22 (t, 2H), 2.29 (t, 2H), 1.58-1.49 (m, 4H), 1.47-1.39 (m, 2H), 1.18 (t, 3H). |
| **6A** | 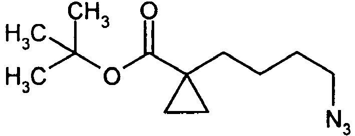 | GC-MS (Methode 1): Rₜ = 4.63 min; m/z = 154 |
| | | MS (DCI): m/z = 240 (M+H)⁺ |
| | | ¹H-NMR (400 MHz, DMSO-d₆): δ = 3.31 (m, 2H), 1.55-1.42 (m, 6H), 1.39 (s, 9H), 0.98 (m, 2H), 0.65 (m, 2H). |
| **7A** | 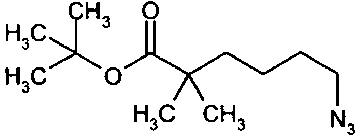 | ¹H-NMR (400 MHz, DMSO-d₆): δ = 3.32 (t, 2H), 1.53-1.41 (m, 4H), 1.39 (s, 9H), 1.30-1.20 (m, 2H), 1.06 (s, 6H). |
| **8A** | 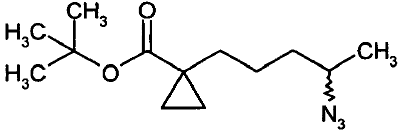 | GC-MS (Methode 1): Rₜ = 4.74 min; m/z = 154 |
| | | MS (DCI): m/z = 254 (M+H)⁺ |
| | | ¹H-NMR (400 MHz, DMSO-d₆): δ = 3.54 (q, 1H), 1.51-1.40 (m, 6H), 1.39 (s, 9H), 1.19 (d, 3H), 0.98 (m, 2H), 0.67 (m, 2H). |

### Beispiel 9A

### (+/-)-6-Azido-2-methylhexansäureethylester

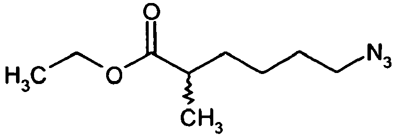

Zu einer auf -78°C gekühlten Lösung von 0.32 ml (2.28 mmol) Diisopropylamin in 2 ml abs. THF wurden 0.91 ml (2.28 mmol) einer 2.5 M Lösung von n-Butyllithium in n-Hexan getropft, wobei die Reaktionstemperatur unter -60°C gehalten wurde. Nach 30 min Rühren bei -60°C bis -70°C wurde diese Lösung zu einer auf -78°C gekühlten Lösung von 352 mg (1.9 mmol) 6-Azidohexansäureethylester in 2 ml abs. THF getropft. Nach Ende der Zugabe wurde die Mischung auf -20°C erwärmt und 20 min nachgerührt, bevor 0.18 ml (2.85 mmol) Methyliodid zugetropft wurden. Nach Ende der Zugabe wurde die Mischung langsam auf RT erwärmt und weitere 2 h gerührt. Das Reaktionsgemisch wurde dann auf gesättigte wässrige Ammoniumchlorid-Lösung gegeben. Man extrahierte dreimal mit Dichlormethan, trocknete die vereinigten organischen Phasen über Magnesiumsulfat und konzentrierte im Vakuum. Das Produkt wurde durch Chromatographie an Silicagel gereinigt (Laufmittel Cyclohexan/Dichlormethan 60:1). Es wurden 96.4 mg (25.5% d. Th.) der Zielverbindung erhalten.
MS (DCI): m/z = 200 (M+H)⁺.
GC-MS (Methode 1): Rₜ = 4.05 min.
¹H-NMR (400 MHz, DMSO-d₆): δ = 4.05 (q, 2H), 3.31 (t, 2H), 2.45-2.39 (m, 1H), 1.58-1.49 (m, 4H), 1.34-1.28 (m, 2H), 1.19 (t, 3H), 1.07 (d, 3H).

### Beispiel 10A

### 1-[4-(1,3-Dioxo-1,3-dihydro-2H-isoindol-2-yl)butyl]cyclopropancarbonsäure-tert.-butylester

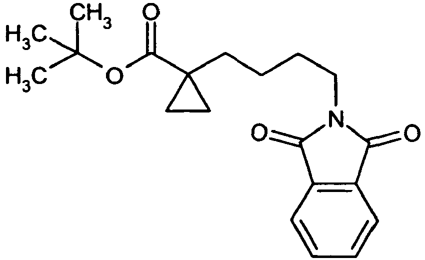

4.2 g (15.15 mmol) 1-(4-Brombutyl)cyclopropancarbonsäure-*tert*.-butylester wurden unter Argon in 50 ml DMF vorgelegt, mit 3.34 g (22.72 mmol) Phthalimid sowie 4.19 g (30.3 mmol) Kaliumcarbonat versetzt und 2 h bei 90°C gerührt. Das Reaktionsgemisch wurde danach filtriert, das Filtrat mit Wasser verdünnt und dreimal mit Ethylacetat extrahiert. Die vereinigten organischen Phasen wurden über Magnesiumsulfat getrocknet und im Vakuum konzentriert. Das Produkt wurde durch Chromatographie an Silicagel gereinigt (Laufmittel Cyclohexan/Ethylacetat 10:1). Es wurden 4.23 g (81.3% d. Th.) der Zielverbindung erhalten.
LC-MS (Methode 2): Rₜ = 2.46 min; m/z = 342 (M-H)⁻.
¹H-NMR (400 MHz, DMSO-d₆): δ = 7.86 (m, 4H), 3.57 (t, 2H), 1.56 (m, 2H), 1.40 (m, 4H), 1.27 (s, 9H), 0.94 (q, 2H), 0.64 (q, 2H).

### Beispiel 11A

### 6-Oxoheptansäure-tert.-butylester

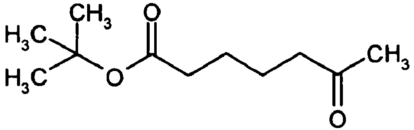

10.0 g (ca. 90%-ig, 62.4 mmol) 6-Oxoheptansäure wurden in 71.8 ml Cyclohexan vorgelegt und mit 20.46 g (93.6 mmol) *tert*.-Butyl-2,2,2-trichloracetimidat und 15 ml Dichlormethan versetzt. Zu der Lösung wurden bei -10°C langsam 0.55 ml (6.24 mmol) Trifluormethansulfonsäure getropft. Die resultierende Suspension wurde über Nacht unter Erwärmung auf RT gerührt. Der unlösliche Niederschlag wurde dann durch Filtration entfernt. Das Filtrat wurde zweimal mit Natriumhydrogencarbonat-Lösung und mit gesättigter Natriumchlorid-Lösung gewaschen, über Magnesiumsulfat getrocknet und im Vakuum eingeengt. Der Rückstand wurde durch Chromatographie an Silicagel gereinigt (Laufmittel Cyclohexan/Ethylacetat 5:1). Aus dem so erhaltenen Produkt fiel beim Stehen über Nacht ein Feststoff aus, der durch Absaugen entfernt und verworfen wurde. Das Zielprodukt in Form des Filtrats wurde nicht weiter aufgereinigt. Es wurden 4.51 g (36.1 % d. Th.) der Titelverbindung erhalten.
GC-MS (Methode 1): Rₜ = 4.1 min; m/z = 144 (M-C₄H₈)⁺.
MS (DCI): m/z = 218 (M+NH₄)⁺.
¹H-NMR (400 MHz, DMSO-d₆): δ = 2.46-2.42 (m, 2H), 2.20-2.15 (m, 2H), 2.08 (s, 3H), 1.47-1.40 (m, 4H), 1.41 (s, 9H).

### Beispiel 12A

### (+/-)6-Aminoheptansäure-tert.-butylester

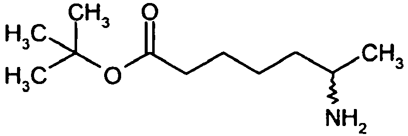

Zu einer Lösung von 2.0 g (9.99 mmol) 6-Oxoheptansäure-*tert*.-butylester in 10 ml Methanol wurden bei RT 7.70 g (99.86 mmol) Ammoniumacetat und 941 mg (14.98 mmol) Natriumcyanoborhydrid gegeben. Die Mischung wurde über Nacht bei RT gerührt und dann auf Wasser gegeben. Mithilfe von 10%-iger Natronlauge wurde der pH auf ca. 10 eingestellt und die Mischung dreimal mit Ethylacetat extrahiert. Man vereinigte die organischen Phasen, trocknete über Magnesiumsulfat und engte im Vakuum ein. Nach Trocknen im Hochvakuum wurden 1.95 g (ca. 80% Reinheit, ca.78% d. Th.) der Zielverbindung erhalten.
MS (DCI): m/z = 202 (M+H)⁺.
¹H-NMR (500 MHz, DMSO-d₆): δ = 2.95-2.89 (m, 1H), 2.19 (t, 2H), 1.52-1.43 (m, 2H), 1.41 (s, 9H), 1.35-1.25 (m, 4H), 1.04 (d, 3H).

### Allgemeine Vorschrift 2: Reduktion von Aziden zu primären Aminen

Zu einer Lösung des entsprechenden Azids in Ethanol oder Methanol (gegebenenfalls unter Zusatz von Wasser) wird Hydrier-Katalysator (z.B. 5% oder 10% Palladium auf Kohle) gegeben. Die Reaktionsmischung wird kräftig bis zum vollständigen Umsatz unter einer Wasserstoffatmosphäre bei Normaldruck gerührt und dann über Kieselgur abfiltriert. Der Filter-Rückstand wird mit Ethanol oder Methanol gewaschen, die erhaltenen Filtrate vereinigt, vorsichtig im Vakuum eingeengt und der Rückstand kurz im Hochvakuum getrocknet. Das so erhaltene Amin kann ohne weitere Reinigung in den Folgereaktionen eingesetzt werden.

Die folgenden Beispiele wurden gemäß der Allgemeinen Vorschrift 2 hergestellt:

| **Beispiel** | **Struktur** | **Analytische Daten** |
|---|---|---|
| **13A** | 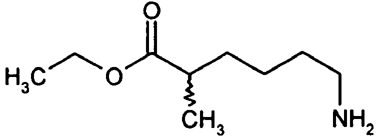 | GC-MS (Methode 1): Rₜ = 3.63 min; m/z = 173 (M)⁺ |
| | | ¹H-NMR (400 MHz, DMSO-d₆): δ = 4.04 (q, 2H), 3.15 (br. s, 2H), 2.39 (q, 1H), 1.57-1.21 (m, 6H), 1.19 (t, 3H), 1.05 (d, 3H). |
| **14A** | 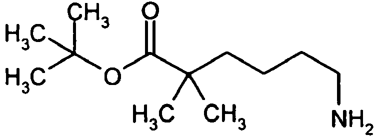 | MS (DCI): m/z = 216 (M+H)⁺. |
| **15A** | 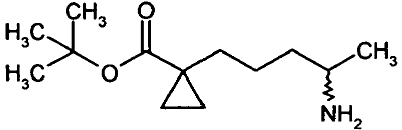 | GC-MS (Methode 1): Rₜ = 4.31 min; m/z = 171 (M-C₄H₈)⁺. |
| **16A** | 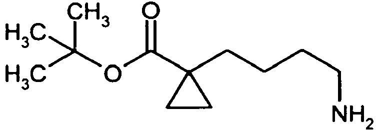 | MS (DCI): m/z = 214 (M+H)⁺. |

### Beispiel 16A

### 1-(4-Aminobutyl)cyclopropancarbonsäure-tert.-butylester

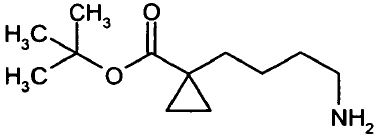

Die Titelverbindung konnte alternativ zum obigen Verfahren auch ausgehend von 1-[4-(1,3-Dioxo-1,3-dihydro-2*H*-isoindol-2-yl)butyl]cyclopropancarbonsäure-*tert*.-butylester hergestellt werden:

Zu einer Lösung von 1.0 g (2.91 mmol) 1-[4-(1,3-Dioxo-1,3-dihydro-2*H*-isoindol-2-yl)butyl]cyclo-propancarbonsäure-*tert*.-butylester in 20 ml Ethanol wurden bei RT 0.29 g (5.8 mmol) Hydrazinhydrat gegeben. Die Mischung wurde 45 min unter Rückfluss gerührt. Das Reaktionsgemisch wurde danach filtriert und das Filtrat bei 20°C am Rotationsverdampfer eingeengt. Der Rückstand wurde in Dichlormethan aufgenommen und zweimal mit gesättigter wässriger Natriumhydrogencarbonat-Lösung gewaschen. Man trocknete die organische Phase über Magnesiumsulfat und engte bei 20°C im Vakuum ein. Es wurden 0.6 g (97.2% d. Th.) der Zielverbindung erhalten. Die Substanz wurde bei -20°C gelagert oder direkt weiter umgesetzt.
GC-MS (Methode 1): Rₜ = 4.2 min; m/z = 138.
MS (DCI): m/z = 214 (M+H)⁺.
¹H-NMR (400 MHz, CDCl₃): δ = 2.69 (t, 2H), 1.44 (m, 15H), 1.25 (s, 2H), 1.1 (q, 2H), 0.6 (q, 2H).

### Beispiel 17A

### [3-(1,3-Dioxo-1,3-dihydro-2H-isoindol-2-yl)propyl](triphenyl)phosphoniumbromid

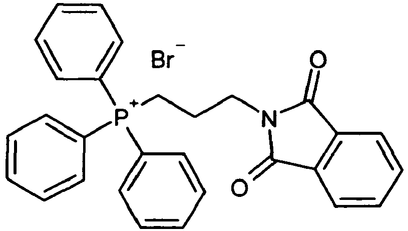

2.50 g (9.33 mmol) 2-(3-Brompropyl)-1*H*-isoindol-1,3(2*H*)-dion in 25 ml Xylol wurden mit Argon entgast und mit 2.45 g (9.33 mmol) Triphenylphosphin versetzt. Es wurde 24 h unter Rückfluss gerührt und die Mischung anschließend bei 70°C filtriert. Der Filterkuchen wurde mit etwas Di-ethylether nachgewaschen und im Hochvakuum getrocknet. Es wurden 3.50 g (70.8% d. Th.) der Titelverbindung erhalten.
¹H-NMR (400 MHz, DMSO-d₆): δ = 7.86 (m, 7H), 7.76 (m, 12H), 3.76 (t, 2H), 3.7 (m, 2H), 1.94 (m, 2H).

### Beispiele 18A

### 1-Formylcyclopropancarbonsäureethylester

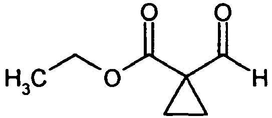

Eine Lösung von 1.225 g (8.5 mmol) 1-Hydroxymethyl-cyclopropancarbonsäueethylester [Herstellung siehe z.B. T.A. Ayers, Tetrahedron Lett. 40 (30), 5467-5470 (1999)] in 43 ml Dichlormethan wurde bei 0°C mit 5.05 g (11.9 mmol) Dess-Martin-Periodan-Reagens versetzt und anschließend 6 h bei RT gerührt. Zu dem Reaktionsgemisch wurde dann eine Lösung von 6.7 g (42.5 mmol) Natriumthiosulfat in 60 ml gesättigter wässriger Natriumhydrogencarbonat-Lösung gegeben. Es wurde 20 min bei RT gerührt und dann die Phasen getrennt. Die organische Phase wurde zweimal mit Wasser gewaschen, über Magnesiumsulfat getrocknet und bei 20°C im Vakuum eingeengt. Es wurden 1.139 g (80.0% d. Th.) der Zielverbindung erhalten.
¹H-NMR (400 MHz, DMSO-d₆): δ = 10.17 (s, 1H), 4.20 (q, 2H), 1.58 (q, 2H), 1.47 (q, 2H), 1.24 (t, 3H).

### Beispiel 19A

### 1-[(1E/Z)-4-(1,3-Dioxo-1,3-dihydro-2H-isoindol-2-yl)but-1-en-1-yl]cyclopropancarbonsäure-ethylester

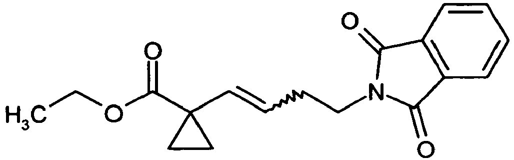

Unter Argon wurden 600 mg (3.377 mmol) 1-Formylcyclopropancarbonsäureethylester und 1.79 g (3.377 mmol) [3-(1,3-Dioxo-1,3-dihydro-2*H*-isoindol-2-yl)propyl](triphenyl)phosphoniumbromid in 12 ml trockenem DMSO vorgelegt und bei 6°C mit einer Lösung von 379 mg (3.377 mmol) Kalium-*tert*.-butylat in 3 ml DMSO versetzt. Es wurde 25 min bei 6°C gerührt, dann auf RT erwärmt und weitere 4 h nachgerührt. Das Reaktionsgemisch wurde danach mit 25 ml Wasser und 35 ml Essigsäureethylester versetzt und extrahiert. Die organische Phase wurde je zweimal mit Wasser und gesättigter Natriumchlorid-Lösung gewaschen. Es wurde über Magnesiumsulfat getrocknet und im Vakuum eingeengt. Das Produkt wurde durch Chromatographie an Silicagel gereinigt (Laufmittel Cyclohexan/Ethylacetat 5:1). Es wurden 504 mg (47.6% d. Th.) der Titelverbindung als *E*/*Z*-Isomerengemisch (ca. 1:2.5) erhalten.
LC-MS (Methode 3): Rₜ = 1.24 min; m/z = 314 (M+H)⁺.

### Beispiel 20A

### 1-[(1E/Z)-4-Aminobut-1-en-1-yl]cyclopropancarbonsäureethylester

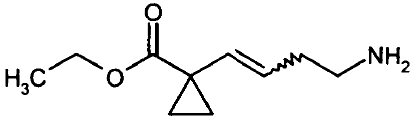

Zu einer Lösung von 255 mg (0.18 mmol) 1-[(1*E*/*Z*)4-(1,3-Dioxo-1,3-dihydro-2H-isoindol-2-yl)-but-1-en-1-yl]cyclopropancarbonsäureethylester in 5.1 ml Ethanol wurden 48 µl (0.99 mmol) Hydrazinhydrat gegeben und die Mischung für 1 h unter Rückfluss gerührt. Der resultierende Niederschlag wurde abgesaugt, mit Ethanol nachgewaschen und das Filtrat bei 20°C im Vakuum eingeengt. Man erhielt 220 mg der rohen Titelverbindung (*E*/*Z*-Isomerengemisch), welche ohne weitere Aufreinigung in Folgereaktionen eingesetzt wurde.
MS (DCI): m/z = 184 (M+H)⁺.

### Beispiel 21A

### Methyl-[1-(prop-2-en-1-yl)cyclopropyl]acetat

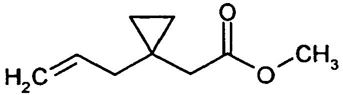

45.69 g (222.2 mmol) Kupfer(I)bromid-Dimethylsulfid-Komplex und 9.42 g (222.2 mmol) wasserfreies Lithiumchlorid wurden in 300 ml THF gelöst, auf -78°C gekühlt und langsam mit 100 ml (200 mmol) einer 2 M Lösung von Allylmagnesiumbromid in Diethylether versetzt. Anschließend wurden zu der Reaktionslösung nacheinander 28.2 ml (222.2 mmol) Chlortrimethylsilan und 11.21 g (100 mmol) Methylcyclopropylidenacetat [CAS Registry-Nr. 110793-87-8] getropft und die Mischung für ca. 5 min nachgerührt (DC-Kontrolle, Laufmittel Cyclohexan/Essigsäureethylester 20:1). Die Reaktionslösung wurde dann mit 50 ml einer wässrigen Lösung von Ammoniak/Ammoniumchlorid (1:9) versetzt und über Kieselgur filtriert. Die organische Phase wurde abgetrennt und die wässrige Phase noch zweimal mit Diethylether extrahiert. Die vereinigten organischen Phasen wurden anschließend mit gesättigter Natriumchlorid-Lösung gewaschen, über Magnesiumsulfat getrocknet und im Vakuum eingeengt. Der Rückstand wurde in 100 ml THF gelöst und mit 222 ml (222 mmol) einer 1 M Lösung von Tetrabutylammoniumfluorid in THF versetzt. Die Reaktionslösung wurde noch 10 min nachgerührt und dann bis zur Trockene eingeengt. Das erhaltene Rohprodukt wurde über Kieselgel chromatographisch gereinigt (Laufmittel Cyclohexan/Essigsäureethylester 20:1). Es wurden 6.92 g (45 mmol, 45% d. Th.) der Titelverbindung erhalten.
GC-MS (Methode 1): Rₜ = 2.48 min; m/z = 155 (M+H)⁺.
¹H-NMR (400 MHz, DMSO-d₆, δ/ppm): 5.84-5.69 (1H, m), 5.02 (2H, d), 3.58 (3H, s), 2.24 (2H, s), 2.05 (2H, d), 0.45-0.35 (4H, m).

### Beispiel 22A

### Methyl-[1-(3-brompropyl)cyclopropyl]acetat

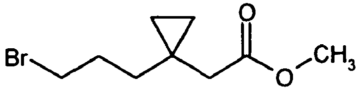

Unter Argon wurden 1542 mg (10 mmol) Methyl-[1-(prop-2-en-1-yl)cyclopropyl]acetat in 10 ml wasserfreiem THF bei 0°C tropfenweise mit 3.4 ml (3.4 mmol) Boran-THF-Komplex-Lösung (1 M in THF) versetzt. Nach 30 min bei 0°C wurde der Ansatz für weitere 30 min bei RT gerührt und dann mit 22 µl (0.54 mmol) Methanol versetzt. Bei -5°C wurden anschließend sukzessive 0.62 ml (12 mmol) Brom sowie 2971 mg (16.5 mmol) Natriummethylat-Lösung (30% in Methanol) zur Reaktionsmischung getropft. Nachdem der Ansatz Raumtemperatur erreicht hatte, wurden 10 ml gesättigte Natriumhydrogencarbonat-Lösung hinzugefügt, die organische Phase abgetrennt und die wässrige Phase noch dreimal mit *tert*.-Butylmethylether extrahiert. Die vereinigten organischen Phasen wurden mit gesättigter Natriumchlorid-Lösung gewaschen und über wasserfreiem Magnesiumsulfat getrocknet. Nach Entfernen des Lösungsmittels im Vakuum wurden 683 mg (2.9 mmol, 29% d. Th.) der Titelverbindung als gelbes Öl erhalten.
GC-MS (Methode 1): Rₜ = 4.29 min; m/z = 205 (M-OCH₃+H)⁺, 155 (M-Br)⁺.
¹H-NMR (400 MHz, DMSO-d₆, δ/ppm): 3.68 (3H, s), 3.41 (2H, t), 2.24 (2H, s), 2.01-1.91 (2H, m), 1.51-1.44 (2H, m), 0.50-0.38 (4H, m).

### Beispiel 23A

### Methyl-[1-(3-azidopropyl)cyclopropyl]acetat

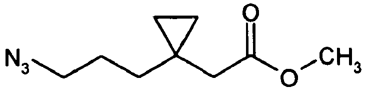

680 mg (2.89 mmol) Methyl-[1-(3-brompropyl)cyclopropyl]acetat und 1128 mg (17.35 mmol) Natriumazid in 5 ml DMF wurden 2 h bei 60°C gerührt. Die Reaktionsmischung wurde dann im Vakuum eingeengt. Der Rückstand wurde in Essigsäureethylester aufgenommen und die Lösung mit gesättigter Natriumchlorid-Lösung gewaschen und über wasserfreiem Magnesiumsulfat getrocknet. Nach Entfernen des Lösungsmittels im Vakuum wurden 389 mg (1.97 mmol, 68% d. Th.) der Titelverbindung als gelbes Öl erhalten.
MS (DCI): m/z = 198 (M+H)⁺, 215 (M+NH₄)⁺.
¹H-NMR (400 MHz, DMSO-d₆, δ/ppm): 3.59 (3H, s), 3.30 (2H, t), 2.26 (2H, s), 1.64-1.53 (2H, m), 1.36-1.29 (2H, m), 0.43-0.30 (4H, m).

### Beispiel 24A

### Methyl-[1-(3-aminopropyl)cyclopropyl]acetat-Hydrochlorid

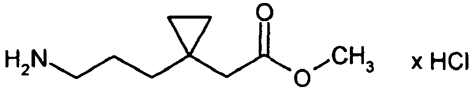

Eine Mischung aus 389 mg (1.97 mmol) Methyl-[1-(3-azidopropyl)cyclopropyl]acetat, 40 mg 10% Palladium auf Kohle und 1.97 ml (1.97 mmol) 1 M Salzsäure in 10 ml Ethanol wurde über Nacht bei Raumtemperatur unter Normaldruck hydriert. Nach Beendigung der Umsetzung wurde der Ansatz filtriert und das Filtrat bis zur Trockene eingeengt. Es wurden 313 mg (1.51 mmol, 76% d. Th.) der Titelverbindung als farbloses Öl erhalten.
MS (DCI): m/z = 172 (M+H)⁺.
¹H-NMR (400 MHz, DMSO-d₆, δ/ppm): 3.60 (3H, s), 2.79-2.66 (2H, m), 2.26 (2H, s), 1.68-1.55 (2H, m), 1.39-1.27 (2H, m), 0.45-0.28 (4H, m).

### Beispiel 25A

### 1-(Prop-2-en-1-yl)cyclopropancarbonsäure-tert.-butylester

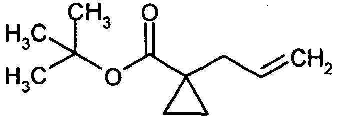

In 35 ml THF wurden 9.9 ml (70.32 mmol) Diisopropylamin vorgelegt, bei -40°C mit 28.1 ml (70.32 mmol) einer 2.5 M Lösung von *n*-Butyllithium in *n*-Hexan versetzt und 30 min gerührt. Man kühlte die Reaktionsmischung dann auf -78°C herunter und tropfte eine Lösung von 10 g (70.32 mmol) Cyclopropancarbonsäure-*tert*.-butylester in 5 ml THF hinzu. Es wurde 4 h bei -78°C gerührt und anschließend eine Lösung von 5.8 ml (66.81 mmol) Allylbromid in 5 ml THF zugetropft. Die Reaktionsmischung wurde über Nacht langsam auf RT erwärmt und dann vorsichtig mit wässriger Ammoniumchlorid-Lösung versetzt. Es wurde dreimal mit Methyl-*tert*.-Butylether extrahiert. Die vereinigten organischen Phasen wurden über Natriumsulfat getrocknet und im Vakuum eingeengt. Es wurden 10.7 g (83.5% d. Th.) der Zielverbindung erhalten.
GC-MS (Methode 1): Rₜ = 2.5 min; m/z = 126 (M-C₄H₈)⁺.
¹H-NMR (400 MHz, DMSO-d₆): δ = 5.82 (m, 1H), 4.98 (d, 2H), 2.21 (d, 2H), 1.37 (s, 9H), 0.99 (q, 2H), 0.69 (q, 2H).

### Beispiel 26A

### 1-(2-Oxoethyl)cyclopropancarbonsäure-tert.-butylester

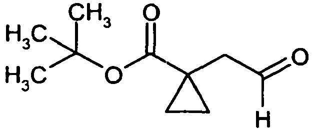

4.0 g (21.9 mmol) 1-(Prop-2-en-1-yl)cyclopropancarbonsäure-*tert*.-butylester wurden in 70 ml Methanol und 30 ml Dichlormethan gelöst. Bei -78°C wurde mit Hilfe eines Ozon-Generators für 45 min Ozon im O₂-Strom durch die Reaktionslösung geleitet. Nachdem die Lösung leicht blau geworden war, wurde reiner Sauerstoff zum Spülen durch die Reaktionslösung geleitet, bis die Lösung sich wieder entfärbt hatte. Die Reaktionslösung wurde dann mit 6.5 ml (88.9 mmol) Dimethylsulfid versetzt und langsam auf RT erwärmt. Es wurde am Rotationsverdampfer eingeengt, und der Rückstand wurde durch Chromatographie an Silicagel gereinigt (Laufmittel-Gradient Cyclohexan/Ethylacetat 50:1, 30:1, 20:1, 10:1). Es wurden 1.87 g (44.1% d. Th.) der Zielverbindung erhalten.
GC-MS (Methode 1): Rₜ = 3.3 min; m/z = 184 (M)⁺.

### Beispiel 27A

### cis/trans-1-[4-Methoxy-4-oxobut-2-en-1-yl]cyclopropancarbonsäure-tert.-butylester

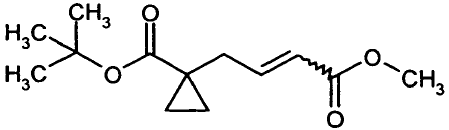

0.425 g (10.63 mmol) Natriumhydrid wurden in 40 ml THF vorgelegt und bei 0°C mit 1.6 ml (11.11 mmol) Phosphonoessigsäuretrimethylester versetzt. Es wurde 1 h bei 0°C gerührt, dann mit 1.78 g (9.66 mmol) 1-(2-Oxoethyl)cyclopropancarbonsäure-*tert*.-butylester versetzt und die Reaktionsmischung langsam auf RT erwärmt. Es wurde 2 h bei RT nachgerührt und der Ansatz anschließend mit Wasser versetzt und dreimal mit Dichlormethan extrahiert. Die vereinigten organischen Phasen wurden mit gesättigter Natriumchlorid-Lösung gewaschen, über Magnesiumsulfat getrocknet und im Vakuum eingeengt. Der Rückstand wurde durch Chromatographie an Silicagel gereinigt (Laufmittel-Gradient Dichlormethan/Ethylacetat 100:0 → 100:0.1). Es wurden 1.32 g (56.7% d. Th.) der Titelverbindung erhalten (*trans*-Isomer im deutlichen Überschuss).
GC-MS (Methode 1): Rₜ = 4.57 min, m/z = 184 (M-C₄H₈)⁺ *cis*-Isomer; Rₜ = 4.82 min, m/z = 184 (M-C₄H₈)⁺ *trans*-Isomer.
¹H-NMR (400 MHz, DMSO-d₆): δ = 6.91 (dt, 1H), 6.88 (d, 1H), 3.65 (s, 3H), 2.37 (d, 2H), 1.36 (s, 9H), 1.05 (q, 2H), 0.77 (q, 2H).

### Beispiel 28A

### cis/trans-1-[4-Hydroxybut-2-en-1-yl]cyclopropancarbonsäure-tert.-butylester

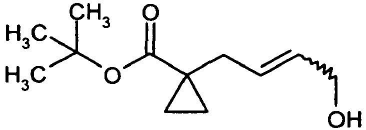

Zu einer Lösung von 1.185 g (4.93 mmol) *cis*/*trans*-1-[4-Methoxy-4-oxobut-2-en-1-yl]cyclo-propancarbonsäure-*tert*.-butylester in 15 ml Dichlormethan wurden bei -90°C langsam 9.7 ml (9.86 mmol) Diisobutylaluminiumhydrid als 1 M Lösung in Hexan zugetropft. Es wurde 2 h bei -90°C gerührt und dann in der Kälte ca. 10 ml 20%-ige wässrige Kaliumtartrat-Lösung zugetropft. Man verdünnte anschließend mit Wasser und Dichlormethan, extrahierte nach Phasentrennung die organische Phase mehrmals mit Wasser und trocknete die organische Phase über Natriumsulfat. Es wurde im Vakuum eingeengt und der Rückstand durch Chromatographie an Silicagel gereinigt (Laufmittel-Gradient Cyclohexan/Ethylacetat 10:1, 8:1, 4:1, 2:1). Es wurden 0.279 g (26.7% d. Th.) der Zielverbindung erhalten (*trans*-Isomer im deutlichen Überschuss).
¹H-NMR (400 MHz, DMSO-d₆): δ = 5.55 (m, 2H), 3.88 (t, 2H), 2.18 (d, 2H), 1.37 (s, 9H), 0.97 (q, 2H), 0.68 (q, 2H).

### Beispiel 29A

### cis/trans-1-[4-(1,3-Dioxo-1,3-dihydro-2H-isoindol-2-yl)but-2-en-1-yl]cyclopropancarbonsäure-tert.-butylester

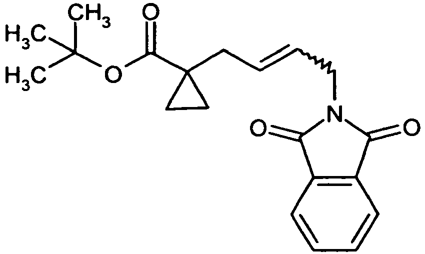

302.6 mg (1.43 mmol) *cis*/*trans*-1-[4-Hydroxybut-2-en-1-yl]cyclopropancarbonsäure-*tert*.-butylester wurden in 2 ml THF gelöst und mit 251.7 mg (1.71 mmol) Phthalimid sowie 411.3 mg (1.57 mmol) Triphenylphosphin versetzt. Die Reaktionsmischung wurde auf -10°C abgekühlt, und langsam wurden 713.7 mg (1.639 mmol) einer 40%-igen Lösung von Diethylazodicarboxylat in Toluol zugetropft. Anschließend wurde auf RT erwärmt und 1.5 h nachgerührt. Das Reaktionsgemisch wurde dann auf Wasser gegeben und mit Dichlormethan extrahiert. Die organische Phase wurde über Natriumsulfat getrocknet und im Vakuum eingeengt. Der Rückstand wurde durch Chromatographie an Silicagel gereinigt (Laufmittel-Gradient Cyclohexan/Ethylacetat 6:1, 4:1, 2:1). Es wurden 121 mg (24.8% d. Th.) der Zielverbindung erhalten (*trans*-Isomer im deutlichen Überschuss).
LC-MS (Methode 2): Rₜ = 2.35 min; m/z = 364 (M+Na)⁺.
¹H-NMR (400 MHz, DMSO-d₆): δ = 7.89 (m, 4H), 5.61 (m, 1H), 5.5 (m, 1H), 4.14 (d, 2H), 2.14 (d, 2H), 1.24 (s, 9H), 0.94 (q, 2H), 0.66 (q, 2H).

### Beispiel 30A

### cis/trans-1-[4-Aminobut-2-en-1-yl]cyclopropancarbonsäure-tert.-butylester

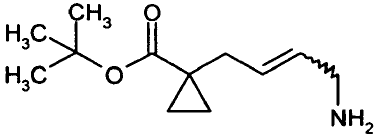

Zu einer Lösung von 120 mg (0.351 mmol) *cis*/*trans*-1-[4-(1,3-Dioxo-1,3-dihydro-2*H*-isoindol-2-yl)but-2-en-1-yl]cyclopropancarbonsäure-*tert*.-butylester in 2.4 ml Ethanol wurden bei RT 34 µl (0.703 mmol) Hydrazinhydrat gegeben. Die Mischung wurde 20 min unter Rückfluss gerührt. Das Reaktionsgemisch wurde dann filtriert und das Filtrat bei 20°C am Rotationsverdampfer eingeengt. Der Rückstand wurde in Dichlormethan aufgenommen und zweimal mit gesättigter wässriger Natriumhydrogencarbonat-Lösung gewaschen. Die organische Phase wurde über Magnesiumsulfat getrocknet und bei 20°C im Vakuum eingeengt. Erhalten wurden 81.2 mg leicht verunreinigtes Rohprodukt (ca. 109% d. Th.; *trans*-Isomer im deutlichen Überschuss). Die Substanz wurde bis zum Einsatz in Folgereaktionen im Tiefkühlschrank gelagert.
MS (DCI): m/z = 212 (M+H)⁺.
¹H-NMR (400 MHz, CDCl₃): δ = 8.06 (q, 1H), 7.82 (q, 1H), 5.51 (m, 2H), 3.11 (d, 2H), 2.17 (m, 2H), 1.37 (s, 9H), 0.97 (q, 2H), 0.67 (q, 2H).

### Beispiel 31A

### tert.-Butyl-5,5,5-trifluor-2-(4-methylphenyl)pentanoat

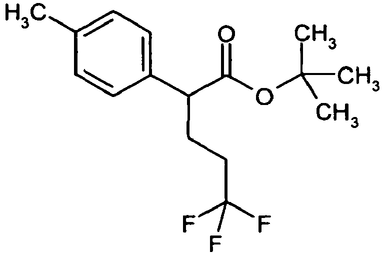

Unter Sauerstoffausschluss wurden 0.88 ml (6.3 mmol) Diisopropylamin in 20 ml THF vorgelegt, auf -78°C abgekühlt und langsam mit 2.52 ml (6.3 mmol) einer 2.5 M Lösung von n-Butyllithium in Hexan versetzt. Anschließend wurde die Reaktionslösung auf -10°C erwärmt und 10 min bei dieser Temperatur nachgerührt. Die Reaktionslösung wurde dann wieder auf -78°C abgekühlt und langsam mit 1 g (4.85 mmol) *tert*.-Butyl-(4-methylphenyl)acetat, gelöst in 10 ml THF, versetzt. Die Reaktionslösung wurde anschließend langsam auf-30°C erwärmt und danach erneut auf-78°C abgekühlt. Nach Erreichen dieser Temperatur wurden 0.62 ml (5.82 mmol) 3-Brom-1,1,1-trifluor-propan langsam zugetropft. Nach beendeter Zugabe wurde die Lösung langsam auf Raumtemperatur erwärmt und über Nacht gerührt. Nach DC-Kontrolle (Laufmittel Cyclohexan/Essigsäureethylester 10:1) wurde der Ansatz mit gesättigter Ammoniumchlorid-Lösung versetzt und in Essigsäureethylester aufgenommen. Die wässrige Phase wurde zweimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 10:1). Es wurden 542 mg (1.79 mmol, 37% d. Th.) eines gelblichen Öls isoliert.
GC-MS (Methode 1): Rₜ = 4.41 min; m/z = 246 (M-C₄H₉+H)⁺.

### Beispiel 32A

### tert.-Butyl-3-methyl-2-(4-methylphenyl)pentanoat

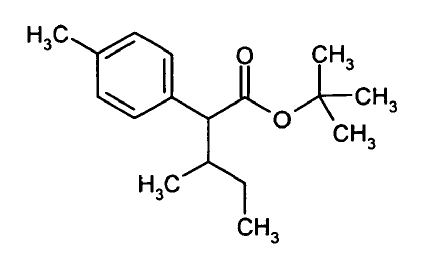

Unter Argon wurden 19.58 g (174.5 mmol) Kalium-*tert*.-butylat in 200 ml DMF vorgelegt, auf 0°C abgekühlt, langsam mit 30 g (145.4 mmol) *tert*.-Butyl-(4-methylphenyl)acetat, gelöst in 50 ml DMF, versetzt und anschließend 30 min bei 0°C gerührt. Nachfolgend wurden 18.95 ml (174.5 mmol) 2-Brombutan langsam zugetropft und die Lösung 4 h bei 0°C nachgerührt. Danach wurde die Reaktionslösung mit 200 ml Wasser und 200 ml Diethylether versetzt. Die wässrige Phase wurde zweimal mit Diethylether extrahiert. Die vereinigten organischen Phasen wurden über Magnesiumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 20:1). Es wurden 15.5 g (59.1 mmol, 40.6% d. Th.) einer farblosen Flüssigkeit isoliert.
¹H-NMR (400 MHz, DMSO-d₆, δ/ppm): 7.17 (2H, d), 7.11 (2H, d), 3.11 (1H, d), 2.27 (3H, s), 2.04-1.90 (1H, m), 1.55-1.42 (1H, m), 1.35 (9H, s), 1.24-1.10 (1H, m), 0.99-0.86 (3H, m), 0.77-0.51 (3H, m).
GC-MS (Methode 1): Rₜ = 5.04 min; m/z = 206 (M-C₄H₉+H)⁺.

Auf analoge Weise wurde die in der folgenden Tabelle aufgeführte Verbindung erhalten:

| **Beispiel** | **Name / Struktur** | **Analytische Daten** |
|---|---|---|
| **33A** | *tert*.-Butyl-cyclopentyl(4-methylphenyl)acetat | ¹H-NMR (400 MHz, DMSO-d₆, δ/ppm): 7.19 (2H, d), 7.11 (2H, d), 3.12 (1H, d), 2.45-2.29 (1H, m), 2.27 (3H, s), 1.89-1.71 (1H, m), 1.67-1.45 (3H, m), 1.44-1.15 (3H, m), 1.36 (9H, s), 1.02-0.84 (1H, m). |
| | 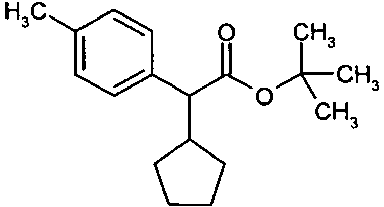 | |
| | | MS (DCI): m/z = 292 (M+NH₄)⁺; GC-MS (Methode 1): Rₜ = 5.89 min; m/z = 218 (M-C₄H₉+H)⁺. |

### Beispiel 34A

### Ethyl-4,4,4-trifluor-3-methyl-2-(4-methylphenyl)butanoat

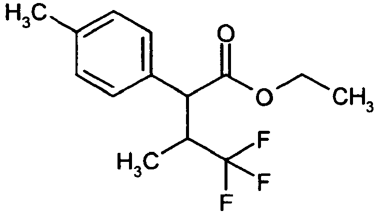

Unter Argon wurden 196.9 mg (0.88 mmol) Palladium(II)acetat und 724.8 mg (1.84 mmol) 2-Di-cyclohexylphosphino-2'-(*N,N*-dimethylamino)biphenyl in 50 ml wasserfreiem Toluol vorgelegt. Die Reaktionslösung wurde anschließend langsam mit 43.8 ml (43.8 mmol) einer 1 M Lösung von Lithiumhexamethyldisilazid in THF versetzt und 10 min bei Raumtemperatur gerührt. Nachfolgend wurde die Reaktionslösung auf -10°C abgekühlt, langsam mit 7 g (38.0 mmol) Ethyl-4,4,4-trifluor-3-methylbutanoat versetzt und 10 min bei -10°C nachgerührt. Danach wurden 5 g (29.2 mmol) 4-Bromtoluol, gelöst in 50 ml Toluol, zugetropft und die Reaktionslösung erst auf Raumtemperatur und dann auf 80°C erwärmt. Das Gemisch wurde 2 h bei dieser Temperatur gerührt, dann auf Raumtemperatur abgekühlt und über Nacht nachgerührt. Nach erfolgter Umsetzung (DC-Kontrolle, Laufmittel Cyclohexan/Dichlormethan 2:1) wurde die Reaktionsmischung über Kieselgur filtriert, der Rückstand mehrfach mit Essigsäureethylester und Dichlormethan gewaschen und die vereinigten Filtrate im Vakuum eingeengt. Das erhaltene Rohprodukt wurde über Kieselgel chromatographisch gereinigt (Laufmittel Petrolether/Dichlormethan 4:1 → 3:1). Es wurden 3.91 g (14.3 mmol, 48.8% d. Th.) der Titelverbindung als farblose Flüssigkeit isoliert.
¹H-NMR (400 MHz, DMSO-d₆, δ/ppm): 7.26 (2H, d), 7.20-7.12 (2H, m), 4.17-3.95 (2H, m), 3.74 (0.25H, d), 3.66 (0.75H, d), 3.35-3.07 (1H, m), 2.29 (2.25H, s), 2.28 (0.75H, s), 1.17 (0.75H, d), 1.11 (3H, t), 0.76 (2.25H, d) (Diastereomerengemisch).
GC-MS (Methode 1): Rₜ = 4.20 min, m/z = 275 (M+H)⁺ (Diastereomer 1); Rₜ = 4.23 min, m/z = 275 (M+H)⁺ (Diastereomer 2).

### Beispiel 35A

### tert.-Butyl-2-[4-(brommethyl)phenyl]-5,5,5-trifluorpentanoat

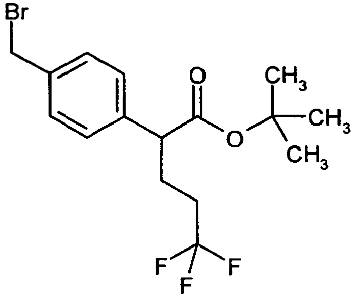

540 mg (1.79 mmol) *tert*.-Butyl-5,5,5-trifluor-2-(4-methylphenyl)pentanoat, 333.8 mg (1.78 mmol) N-Bromsuccinimid und 14.7 mg (0.09 mmol) 2,2'-Azobis-2-methylpropannitril in 10 ml Tetrachlormethan wurden 2 h unter Rückfluss gerührt. Nach vollständiger Umsetzung wurde das Succinimid abfiltriert und der Filterrückstand mit Dichlormethan nachgewaschen. Das Filtrat wurde im Vakuum eingeengt. Das Rohprodukt wurde über Kieselgel chromatographisch gereinigt (Laufmittel Cyclohexan/Essigsäureethylester 10:1). Es wurden 659 mg (1.72 mmol, 97% d. Th.) eines gelblichen Öls isoliert.
GC-MS (Methode 1): Rₜ = 5.91 min; m/z = 301 (M-Br)⁺.

### Beispiel 36A

### tert.-Butyl-2-[4-(brommethyl)phenyl]-3-methylpentanoat

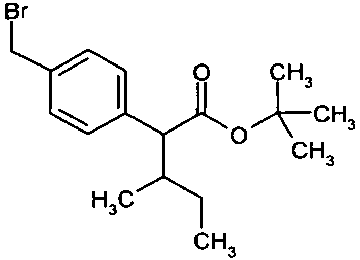

15 g (59.1 mmol) *tert*.-Butyl-3-methyl-2-(4-methylphenyl)pentanoat, 11 g (62 mmol) N-Bromsuccinimid und 97 mg (0.59 mmol) 2,2'-Azobis-2-methylpropannitril in 150 ml Dichlormethan wurden 2 h unter Rückfluss gerührt. Nach vollständiger Umsetzung wurde das Lösungsmittel im Vakuum entfernt. Das Rohprodukt wurde über Kieselgel chromatographisch gereinigt (Laufmittel Cyclohexan/Essigsäureethylester 20:1). Es wurden 16.22 g (47.5 mmol, 80% d. Th.) eines farblosen Öls isoliert.
GC-MS (Methode 1): Rₜ = 6.41 min; m/z = 261 (M-Br)⁺.
MS (DCI): m/z = 358/360 (M+NH₄)⁺.

Auf analoge Weise wurde die in der folgenden Tabelle aufgeführte Verbindung erhalten:

| **Beispiel** | **Name / Struktur** | **Analytische Daten** |
|---|---|---|
| **37A** | *tert*.-Butyl-[4-(brommethyl)phenyl](cyclopentyl)-acetat | ¹H-NMR (400 MHz, DMSO-d₆, δ/ppm): 7.39 (2H, d), 7.30 (2H, d), 4.68 (2H, s), 3.21 (1H, d), 2.45-2.31 (1H, m), 1.89-1.74 (1H, m), 1.69-1.45 (3H, m), 1.44-1.16 (3H, m), 1.35 (9H, s), 1.02-0.88 (1H, m). |
| | 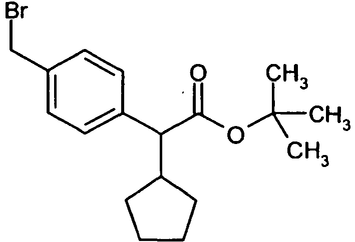 | |
| | | MS (DCI): m/z = 370/372 (M+NH₄)⁺. |

### Beispiel 38A

### Ethyl-2-[4-(brommethyl)phenyl]-4,4,4-trifluor-3-methylbutanoat

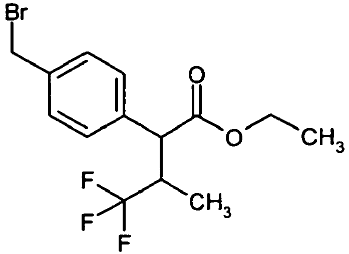

2.25 g (8.2 mmol) Ethyl-4,4,4-trifluor-3-methyl-2-(4-methylphenyl)butanoat, 1.53 g (8.6 mmol) N-Bromsuccinimid und 67 mg (0.41 mmol) 2,2'-Azobis-(2-methylpropannitril) in 36 ml Trichlormethan wurden über Nacht unter Rückfluss gerührt. Nach vollständiger Umsetzung wurde das Succinimid abfiltriert und der Filterrückstand mit Dichlormethan nachgewaschen. Das Filtrat wurde im Vakuum eingeengt. Das Rohprodukt wurde über Kieselgel chromatographisch gereinigt (Laufmittel Cyclohexan/Essigsäureethylester 40:1). Es wurden 2.667 g (7.5 mmol, 92% d. Th.) eines gelblichen Öls isoliert.
GC-MS (Methode 1): Rₜ = 5.72 min, m/z = 373 (M-Br)⁺ (Diastereomer 1); Rₜ = 5.74 min, m/z = 373 (M-Br)⁺ (Diastereomer 2).

### Beispiel 39A

### N'-(2-Chloracetyl)benzolcarbohydrazid

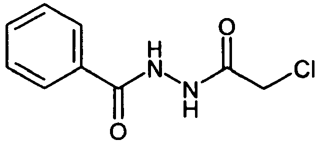

Eine Suspension von 500 g (3.67 mol) Benzolcarbohydrazid in 3.75 Liter THF wurde auf Rückfluss erhitzt, wobei sich das Benzolcarbohydrazid löste. Zu dieser Lösung wurden 497.7 g (4.41 mol) Chloracetylchlorid, gelöst in 125 ml THF, getropft und die Lösung 30 min unter Rückfluss nachgerührt. Nach vollständiger Umsetzung (DC-Kontrolle, Laufmittel Dichlormethan/Methanol 9:1) wurde der Ansatz mit 22.5 Liter Wasser und 10 Liter Essigsäureethylester versetzt und mit festem Natriumhydrogencarbonat auf pH 7 eingestellt. Die wässrige Phase wurde einmal mit 2.5 Liter Essigsäureethylester extrahiert. Die vereinigten organischen Phasen wurden getrocknet und die Lösung anschließend im Vakuum bis zur Trockene eingeengt. Der erhaltene weiße Feststoff wurde in einem 1: 1-Gemisch aus Dichlormethan und Methanol gelöst und auf 3 kg Kieselgel aufgezogen. An zwei Kieselgel-Portionen (jeweils 8 kg) wurde zunächst mit 50 Liter Dichlormethan/Essigsäureethylester 7:3 und dann mit 125 Liter Dichlormethan/Essigsäureethylester 1:1 als Laufmittel chromatographiert. Nach Einengen der Produktfraktionen wurden 424 g (1.99 mol, 54% d. Th.) der Titelverbindung als weißer Feststoff erhalten.
¹H-NMR (400 MHz, DMSO-d₆, δ/ppm): 10.56-10.32 (2H, breit), 7.88 (2H, d), 7.58 (1H, t), 7.50 (2H, t), 4.21 (2H, s).
MS (DCI): m/z = 213 (M+H)⁺, 230 (M+NH₄)⁺.

### Beispiel 40A

### 2-Phenyl-4H-1,3,4-oxadiazin-5(6H)-on

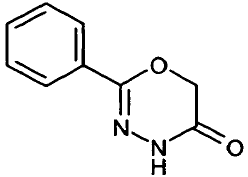

812 g (3.82 mol) *N*'-(2-Chloracetyl)benzolcarbohydrazid wurden in 13 Liter trockenem DMF gelöst und mit 384.95 g (4.58 mol) Natriumhydrogencarbonat versetzt. Anschließend wurde die Reaktionslösung auf 100°C erhitzt und über Nacht bei dieser Temperatur gerührt. Nach vollständiger Umsetzung (DC-Kontrolle, Laufmittel Dichlormethan/Essigsäureethylester 9:1) wurde die Reaktionslösung auf Raumtemperatur abgekühlt, auf 65 Liter Wasser gegossen und dreimal mit jeweils 17.5 Liter Essigsäureethylester extrahiert. Die vereinigten organischen Phasen wurden mit 13.8 Liter gesättigter wässriger Natriumhydrogencarbonat-Lösung gewaschen, getrocknet und im Vakuum bis zur Trockene eingeengt. Der erhaltene Feststoff wurde in einem 9:1-Gemisch aus Dichlormethan und Methanol gelöst und auf 17 kg Kieselgel aufgezogen. An zwei Kieselgel-Portionen (jeweils 8 kg) wurde mit 260 Liter Dichlormethan/Essigsäureethylester 9:1 als Laufmittel chromatographiert. Die vereinigten Produktfraktionen wurden eingeengt, und der erhaltene Feststoff wurde mit 3 Liter Diethylether ausgerührt. Nach Filtration wurden 247 g (1.40 mol, 35% d. Th.) der Titelverbindung als weißer Feststoff erhalten.
¹H-NMR (400 MHz, DMSO-d₆, δ/ppm): 11.04 (1H, s), 7.78 (2H, d), 7.53-7.41 (3H, m), 4.79 (2H, s).
MS (DCI): m/z = 177 (M+H)⁺.

### Beispiel 41A

### 6-Phenylpyridazin-3(2H)-on

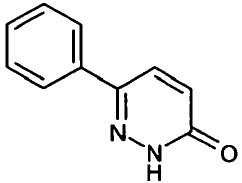

19.6 g (162.95 mmol) 1-Phenylethanon und 5 g (54.32 mmol) Oxoessigsäure-Monohydrat wurden 2 Stunden bei 100°C gerührt. Die Reaktionslösung wurde anschließend auf 40°C abgekühlt und mit 20 ml Wasser und 4 ml Ammoniak versetzt. Nachfolgend wurde das Gemisch zweimal mit 50 ml Dichlormethan extrahiert. Die erhaltene wässrige Phase wurde dann mit 2.64 ml (53.32 mmol) Hydrazin-Monohydrat versetzt und 2 Stunden bei 100°C gerührt. Nach Umsetzung wurde die Reaktionslösung auf Raumtemperatur abgekühlt. Die ausgefallenen Kristalle wurden abgesaugt, mit Wasser gewaschen und über Nacht im Vakuumtrockenschrank bei 50°C getrocknet. Es wurden 4.3 g (24.97 mmol, 15% d. Th.) der Titelverbindung als farblose Kristalle erhalten.
LC-MS (Methode 4): Rₜ = 1.39 min; m/z = 173 (M+H)⁺.
¹H-NMR (400 MHz, DMSO-d₆, δ/ppm): 13.2 (s, 1 H), 8.04 (d, 1 H), 7.86 (d, 2 H), 7.53-7.41 (m, 3 H), 7.00 (d, 1 H).

### Beispiel 42A

### tert.-Butyl-cyclopentyl{4-[(5-oxo-2-phenyl-5,6-dihydro-4H-1,3,4-oxadiazin-4-yl)methyl]phenyl}-acetat

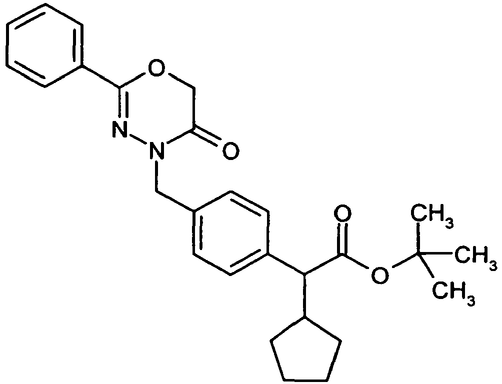

### Darstellungsmethode 1:

9.9 g (28.0 mmol) *tert*.-Butyl-[4-(brommethyl)phenyl](cyclopentyl)acetat, 5.92 g (33.6 mmol) 2-Phenyl-4*H-*1,3,4-oxadiazin-5(6*H*)-on und 13.70 g (42.03 mmol) Cäsiumcarbonat wurden in 100 ml DMF 12 h lang bei 60°C gerührt. Nach dem Abkühlen wurde der Ansatz auf Eiswasser gegeben und mit Diethylether extrahiert. Die organische Phase wurde über Magnesiumsulfat getrocknet und im Vakuum bis zur Trockene eingeengt. Das Rohprodukt wurde über Kieselgel chromatographisch gereinigt (Laufmittel Cyclohexan/Essigsäureethylester 20:1). Es wurden 6.6 g (14.7 mmol, 52% d. Th.) der Titelverbindung erhalten.

### Darstellungsmethode 2:

8.16 g (23.1 mmol) *tert*.-Butyl-[4-(brommethyl)phenyl](cyclopentyl)acetat, 3.7 g (21 mmol) 2-Phenyl-4*H*-1,3,4-oxadiazin-5(6*H*)-on und 7.53 g (23.1 mmol) Cäsiumcarbonat wurden in 147 ml DMF 12 h lang bei Raumtemperatur gerührt. Anschließend wurde die Reaktionslösung mit gesättigter wässriger Natriumhydrogencarbonat-Lösung verrührt und zweimal mit Essigsäureethylester extrahiert. Die vereinigten organischen Phasen wurden über Magnesiumsulfat getrocknet und im Vakuum bis zur Trockene eingeengt. Das erhaltene Rohprodukt wurde über Kieselgel chromatographisch gereinigt (Laufmittel Cyclohexan/Essigsäureethylester 5:1). Es wurden 6.51 g (14.5 mmol, 69% d. Th.) der Titelverbindung erhalten.

¹H-NMR (400 MHz, DMSO-d₆, δ/ppm): 7.76 (2H, d), 7.55-7.42 (3H, m), 7.31 (4H, s), 4.94 (2H, s), 4.87 (2H, s), 3.19 (1H, d), 2.45-2.31 (1H, m), 1.88-1.74 (1H, m), 1.69-1.46 (3H, m), 1.45-1.15 (3H, m), 1.34 (9H, s), 1.03-0.89 (1H, m).
LC-MS (Methode 4): Rₜ = 3.27 min; m/z = 449 (M+H)⁺.
Auf analoge Weise wurden die in der folgenden Tabelle aufgeführten Verbindungen erhalten:

| **Beispiel** | **Name / Struktur** | **Analytische Daten** |
|---|---|---|
| **43A** | *tert*.-Butyl-5,5,5-trifluor-2-{4-[(5-oxo-2-phenyl-5,6-dihydro-4*H*-1,3,4-oxadiazin-4-yl)methyl]phenyl}-pentanoat | LC-MS (Methode 2): Rₜ = 2.69 min; m/z = 477 (M+H)⁺. |
| | 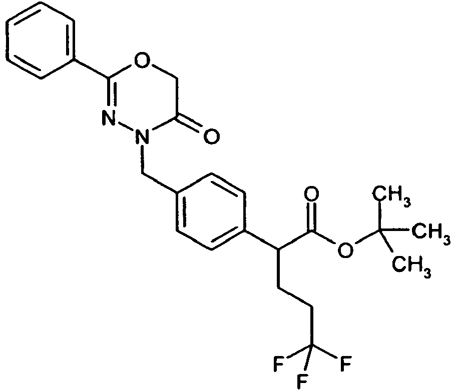 | |
| **44A** | *tert*.-Butyl-3-methyl-2-{4-[(5-oxo-2-phenyl-5,6-dihydro-4*H*-1,3,4-oxadiazin-4-yl)methyl]phenyl}-pentanoat | LC-MS (Methode 4): Rₜ = 3.24 min; m/z = 437 (M+H)⁺. |
| | 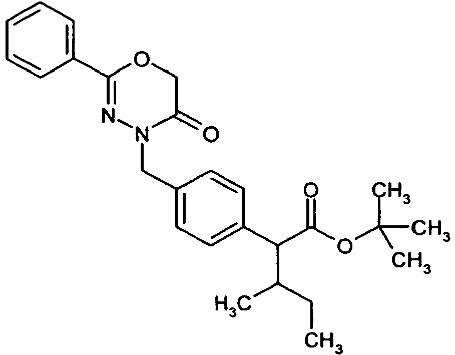 | |
| **45A** | *tert*.-Butyl-cyclopentyl{4-[(6-oxo-3-phenyl-pyridazin-1(6*H*)-yl)methyl]phenyl}acetat | LC-MS (Methode 3): Rₜ = 1.68 min; m/z = 467 (M+Na)⁺. |
| | 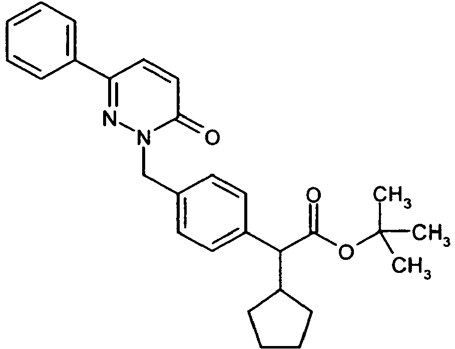 | ¹H-NMR (400 MHz, DMSO-d₆, δ/ppm): 8.08 (1H, d), 7.91-7.85 (2H, m), 7.53-7.42 (3H, m), 7.3 0 (4H, s), 7.09 (1H, d), 5.21 (2H, s), 3.18 (1H, d), 2.44-2.30 (1H, m), 1.85-1.74 (1H, m), 1.65-1.45 (3H, m), 1.44-1.35 (1H, m), 1.34 (9H, s), 1.30-1.15 (2H, m), 1.00-0.88 (1H, m). |
| **46A** | Ethyl-4,4,4-trifluor-3-methyl-2-{4-[(5-oxo-2-phenyl-5,6-dihydro-4*H*-1,3,4-oxadiazin-4-yl)methyl]-phenyl} butanoat | GC-MS (Methode 1): Rₜ = 9.98 min; m/z = 449 (M+H)⁺. |
| | 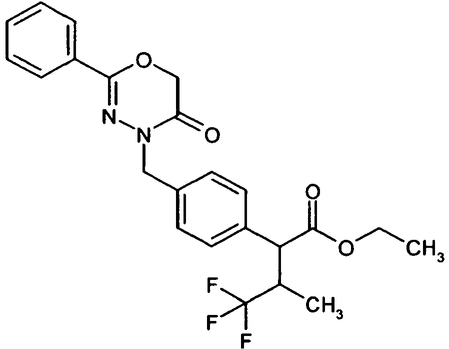 | |

### Beispiel 47A und Beispiel 48A

### tert.-Butyl-cyclopentyl{4-[(6-oxo-3-phenylpyridazin-1(6H)-yl)methyl]phenyl}acetat (Enantiomer 1 und 2)

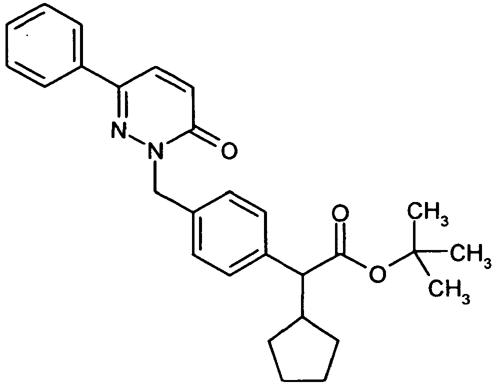

7.42 g (16.69 mmol) des racemischen *tert*.-Butyl-cyclopentyl{4-[(6-oxo-3-phenylpyridazin-1(6*H*)-yl)methyl]phenyl}acetats (Beispiel 45A) wurden mittels präparativer HPLC an chiraler Phase in die Enantiomere aufgetrennt [Säule: Daicel Chiralpak AS-H, 5 µm, 250 mm x 20 mm; Elutionsmittel: Isohexan/Isopropanol 75:25 (v/v); Fluss: 15 ml/min; UV-Detektion: 220 nm; Temperatur: 30°C]:

### Beispiel 47A Enantiomer 1):

Ausbeute: 4.1 g
Rₜ 5.28 min; Reinheit >99%; >99% ee
   [Säule: Daicel Chiralpak AS-H, 5 µm, 250 mm x 4.6 mm; Elutionsmittel: Isohexan/Isopropanol 75:25 (v/v); Fluss: 1 ml/min; UV-Detektion: 220 nm; Temperatur: 40°C].

### Beispiel 48A (Enantiomer 2):

Ausbeute: 2.8 g
R, 5.84 min; Reinheit >98%; >96% ee
   [Säule: Daicel Chiralpak AS-H, 5 µm, 250 mm x 4.6 mm; Elutionsmittel: Isohexan/Isopropanol 75:25 (v/v); Fluss: 1 ml/min; UV-Detektion: 220 nm; Temperatur: 40°C].

### Beispiel 49A

### rac-Cyclopentyl{4-[(5-oxo-2-phenyl-5,6-dihydro-4H-1,3,4-oxadiazin-4-yl)methyl]phenyl}-essigsäure

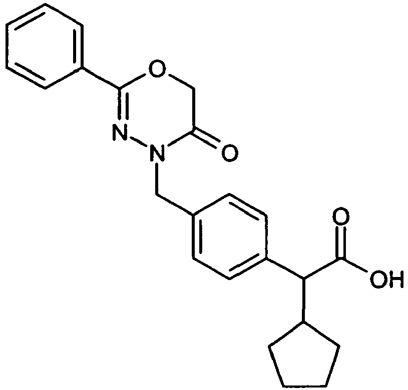

Eine Lösung von 6.6 g (14.7 mmol) *tert*.-Butyl-cyclopentyl{4-[(5-oxo-2-phenyl-5,6-dihydro-4*H-*1,3,4-oxadiazin-4-yl)methyl]phenyl}acetat in 90 ml Dichlormethan wurde bei Raumtemperatur langsam mit 22.67 ml (294.3 mmol) Trifluoressigsäure versetzt und die Mischung über Nacht gerührt. Danach wurde das Lösungsmittel im Vakuum entfernt, der Rückstand in 100 ml Essigsäureethylester aufgenommen und mit 50 ml Wasser ausgeschüttelt. Die organische Phase wurde über Magnesiumsulfat getrocknet. Nach Filtration wurde das Lösungsmittel im Vakuum entfernt. Es wurden 4.8 g (12.23 mmol, 83% d. Th.) eines farblosen Feststoffs erhalten.

¹H-NMR (400 MHz, DMSO-d₆, δ/ppm): 12.35-12.15 (1H, br. s), 7.78 (2H, d), 7.54-7.40 (3H, m), 7.29 (4H, s), 4.91 (2H, s), 4.83 (2H, s), 3.22 (1H, d), 2.48-2.35 (1H, m), 1.89-1.76 (1H, m), 1.68-1.46 (3H, m), 1.45-1.32 (1H, m), 1.32-1.14 (2H, m), 1.01-0.89 (1H, m).
LC-MS (Methode 4): Rₜ = 2.75 min; m/z = 393 (M+H)⁺.

Auf analoge Weise wurden die in der folgenden Tabelle aufgeführten Verbindungen erhalten:

| **Beispiel** | **Name / Struktur** | **Analytische Daten** |
|---|---|---|
| **50A** | 5,5,5-Trifluor-2-{4-[(5-oxo-2-phenyl-5,6-dihydro-4*H*-1,3,4-oxadiazin-4-yl)methyl]phenyl}pentansäure | ¹H-NMR (400 MHz, DMSO-d₆, δ/ppm): 13.0-12.0 (1H, br. s), 7.77 (2H, d), 7.55-7.41 (3H, m), 7.35 (2H, d), 7.26 (2H, d), 4.92 (2H, s), 4.87 (2H, s), 3.67-3.53 (1H, m), 2.31-1.95 (3H, m), 1.89-1.74 (1H, m). |
| | 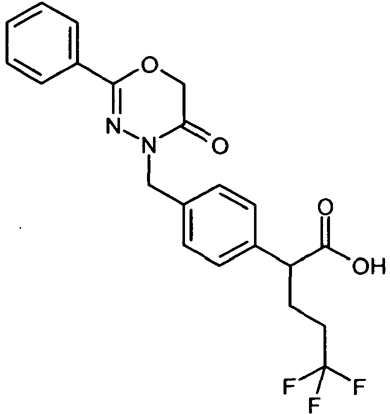 | |
| | | LC-MS (Methode 4): Rₜ = 2.47 min; m/z = 421 (M+H)⁺. |
| **51A** | 3-Methyl-2-{4-[(5-oxo-2-phenyl-5,6-dihydro-4*H-*1,3,4-oxadiazin-4-yl)methyl]phenyl}pentansäure | LC-MS (Methode 2): Rₜ = 2.15 min; m/z = 381 (M+H)⁺. |
| | 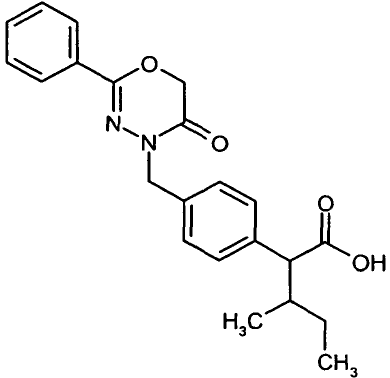 | |
| **52A** | Cyclopentyl {4-[(6-oxo-3-phenylpyridazin-1(6*H*)-yl)methyl]phenyl}essigsäure (*Enantiomer 1*) | LC-MS (Methode 2): Rₜ = 2.11 min; m/z = 389(M+H)⁺. |
| | 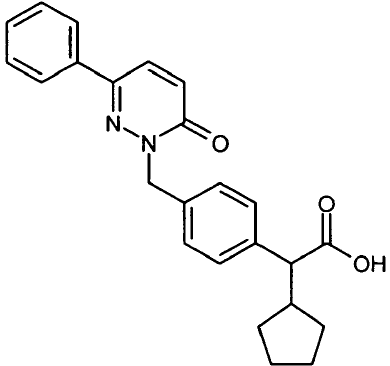 | [α]_{D}²⁰ = +37.3°, c = 0.315, Methanol. |
| | | ¹H-NMR (400 MHz, DMSO-d₆): δ = 12.15-12.31 (br. s, 1H), 8.09 (d, 1H), 7.89 (d, 2H), 7.43-7.53 (m, 3H), 7.25-7.34 (m, 4H), 7.09 (d, 1H), 5.30 (s, 2H), 3.21 (d, 1H), 2.34-2.47 (m, 1H), 1.75-1.89 (m, 1H), 1.32-1.66 (m, 4H), 1.15-1.31 (m, 2H), 0.87-0.99 (m, 1 H). |
| **53A** | Cyclopentyl{4-[(6-oxo-3-phenylpyridazin-1(6*H*)-yl)methyl]phenyl}essigsäure (*Enantiomer 2*) | LC-MS (Methode 2): Rₜ = 2.11 min; m/z = 389 (M+H)⁺. |
| | 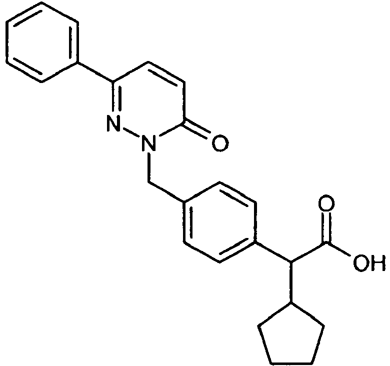 | [α]_{D}²⁰ = -21.0°, c = 0.265, Methanol. |

### Beispiel 54A und Beispiel 55A

### ent-Cyclopentyl{4-[(5-oxo-2-phenyl-5,6-dihydro-4H-1,3,4-oxadiazin-4-yl)methyl]phenyl}essig-säure (Enantiomer 1 und 2)

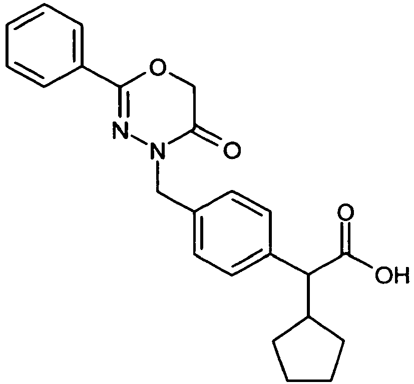

75 g (191.1 mmol) racemische Cyclopentyl{4-[(5-oxo-2-phenyl-5,6-dihydro-4*H*-1,3,4-oxadiazin-4-yl)methyl]phenyl}essigsäure (Beispiel 49A) wurden mittels präparativer HPLC an chiraler Phase in die Enantiomere aufgetrennt [Säule: chirale Kieselgel-Phase basierend auf dem Selektor Poly-(N-methacryloyl-L-isoleucin-3-pentylamid), 430 mm x 40 mm; Elutionsmittel: Isohexan/ Ethylacetat 1:1 (v/v); Fluss: 50 ml/min; Temperatur: 24°C; UV-Detektion: 270 nm]:

### Beispiel 54A (Enantiomer 1):

Ausbeute: 35 g
LC-MS (Methode 4): Rₜ = 2.75 min; m/z = 393 (M+H)⁺
Rₜ 5.73 min; Reinheit >99%; >99% ee
   [Säule: chirale Kieselgel-Phase basierend auf dem Selektor Poly(*N*-methacryloyl-L-isoleucin-3-pentylamid), 250 mm x 4.6 mm; Elutionsmittel: Isohexan/Ethylacetat 1:1 (v/v); Fluss: 2 ml/min; Temperatur: 24°C; UV-Detektion: 270 nm].

### Beispiel 55A (Enantiomer 2):

Ausbeute: 32 g
¹H-NMR (400 MHz, DMSO-d₆, δ/ppm): 12.35-12.15 (1H, breites s), 7.78 (2H, d), 7.54-7.40 (3H, m), 7.29 (4H, s), 4.91 (2H, s), 4.83 (2H, s), 3.22 (1H, d), 2.48-2.35 (1H, m), 1.89-1.76 (1H, m), 1.68-1.46 (3H, m), 1.45-1.32 (1H, m), 1.32-1.14 (2H, m), 1.01-0.89 (1H, m).
LC-MS (Methode 4): Rₜ = 2.75 min; m/z = 393 (M+H)⁺
Rₜ 6.86 min; Reinheit >99%; >99% ee
   [Säule: chirale Kieselgel-Phase basierend auf dem Selektor Poly(*N*-methacryloyl-L-isoleucin-3-pentylamid), 250 mm x 4.6 mm; Elutionsmittel: Isohexan/Ethylacetat 1:1 (v/v); Fluss: 2 ml/min; Temperatur: 24°C; UV-Detektion: 270 nm].
[α]_{D}²⁰ = +37.6°, c = 0.445, Methanol.

### Beispiele 56A - 59A

### 3-Methyl-2-{4-[(5-oxo-2-phenyl-5,6-dihydro-4H-1,3,4-oxadiazin-4-yl)methyl]phenyl}pentansäure (Isomer 1 - 4)

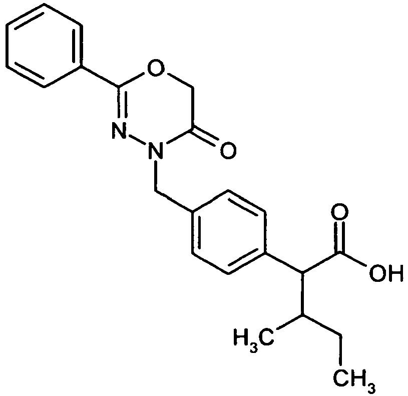

11.8 g (31.02 mmol) des Isomerengemisches von 3-Methyl-2-{4-[(5-oxo-2-phenyl-5,6-dihydro-4*H-*1,3,4-oxadiazin-4-yl)methyl]phenyl}pentansäure (Beispiel 51A) wurden mittels präparativer HPLC an chiraler Phase zunächst in die Diastereomere aufgetrennt [Säule: chirale Kieselgel-Phase basierend auf dem Selektor Poly(*N*-methacryloyl-D-valin-3-pentylamid), 500 mm x 75 mm; Elutionsmittel: Isohexan/Essigsäureethylester 30:70 (v/v); Fluss: 200 ml/min; UV-Detektion: 290 nm; Temperatur: 25°C]. Es wurden 4.11 g bzw. 5.2 g der beiden Diastereomere erhalten.

### Trennung von Diastereomer 1:

4.11 g des Diastereomers 1 wurden mittels präparativer HPLC an chiraler Phase in die Enantiomere *(Isomere 1 und* 2) aufgetrennt [Säule: Daicel Chiralpak AD-H, 5 µm, 250 mm x 20 mm; Elutionsmittel: Isohexan/Isopropanol 95:5 (v/v); Fluss: 25 ml/min; UV-Detektion: 230 nm; Temperatur: 24°C]:

### Beispiel 56A (Isomer 1):

Ausbeute: 865 mg
Rₜ 7.36 min; Reinheit >91%; >93% ee
   [Säule: Daicel Chiralpak AD-H, 5 µm, 250 mm x 4 mm; Elutionsmittel: Isohexan/Isopropanol 80:20 (v/v); Fluss: 1 ml/min; UV-Detektion: 230 nm; Temperatur: 25°C].
LC-MS (Methode 2): R, = 2.16 min; m/z = 381 (M+H)⁺.

### Beispiel 57A (Isomer 2):

Ausbeute: 1662 mg
Rₜ 7.91 min; Reinheit >99%; >97% ee
   [Säule: Daicel Chiralpak AD-H, 5 µm, 250 mm x 4 mm; Elutionsmittel: Isohexan/Isopropanol 80:20 (v/v); Fluss: 1 ml/min; UV-Detektion: 230 nm; Temperatur: 25°C].
LC-MS (Methode 4): Rₜ = 2.53 min; m/z = 381 (M+H)⁺.
¹H-NMR (400 MHz, DMSO-d₆, δ/ppm): 12.35-12.15 (1H, br. s), 7.78 (2H, d), 7.54-7.40 (3H, m), 7.31 (4H, q), 4.92 (2H, s), 4.86 (2H, s), 3.19 (1H, d), 2.09-1.95 (1H, m), 1.59-1.43 (1H, m), 1.25-1.09 (1H, m), 0.89 (3H, t), 0.58 (3H, d).
[α]_{D}²⁰ = +21.7°, c = 0.525, Methanol.

### Trennung von Diastereomer 2:

5.2 g des Diastereomers 2 wurden mittels präparativer HPLC an chiraler Phase in die Enantiomere *(Isomere 3 und 4)* aufgetrennt [Säule: Daicel Chiralcel OJ-H, 5 µm, 250 mm x 20 mm; Elutionsmittel: Isohexan/Isopropanol 95:5 (v/v); Fluss: 25 ml/min; UV-Detektion: 230 nm; Temperatur: 24°C]:

### Beispiel 58A (Isomer 3):

Ausbeute: 2970 mg
Rₜ 7.21 min; Reinheit >94%; >99% ee
   [Säule: Daicel Chiralcel OJ-H, 5 µm, 250 mm x 4 mm; Elutionsmittel: Isohexan/Isopropanol 80:20 (v/v); Fluss: 1 ml/min; UV-Detektion: 230 nm; Temperatur: 25°C].
LC-MS (Methode 4): Rₜ = 2.53 min; m/z = 381 (M+H)⁺.

### Beispiel 59A (Isomer 4):

Ausbeute: 1350 mg
Rₜ 7.77 min; Reinheit >90%; >84% ee
   [Säule: Daicel Chiralcel OJ-H, 5 µm, 250 mm x 4 mm; Elutionsmittel: Isohexan/Isopropanol 80:20 (v/v); Fluss: 1 ml/min; UV-Detektion: 230 nm; Temperatur: 25°C].
LC-MS (Methode 2): Rₜ = 2.17 min; m/z = 381 (M+H)⁺.

### Beispiel 60A

### 4,4,4-Trifluor-3-methyl-2-{4-[(5-oxo-2-phenyl-5,6-dihydro-4H-1,3,4-oxadiazin-4-yl)methyl]-phenyl} butansäure

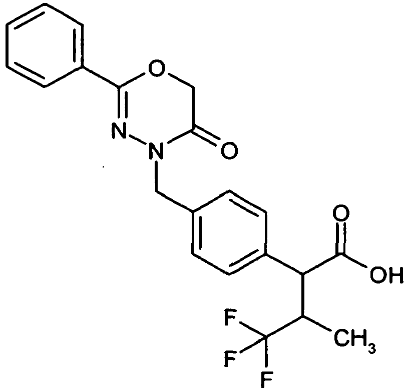

Eine Lösung von 1283 mg (2.86 mmol) Ethyl-4,4,4-trifluor-3-methyl-2-{4-[(5-oxo-2-phenyl-5,6-dihydro-4H-1,3,4-oxadiazin-4-yl)methyl]phenyl}butanoat in 10 ml Dioxan wurde mit 11.4 ml (11.4 mmol) 1 N Natronlauge versetzt und über Nacht bei 80°C gerührt. Nach vollständiger Umsetzung wurde das Dioxan im Vakuum abgezogen, die verbleibende Lösung mit Wasser verdünnt und anschließend mit 1 M Salzsäure auf pH 2 gestellt. Der ausgefallene Feststoff wurde abfiltriert, mit Wasser gewaschen und über Nacht im Vakuum bei 45°C getrocknet. Es wurden 1058 mg (2.52 mmol, 88% d. Th.) der Titelverbindung als Isomerengemisch erhalten.
LC-MS (Methode 5): Rₜ = 1.12 min, m/z = 421 (M+H)⁺ (Diastereomer 1); Rₜ = 1.13 min, m/z = 421 (M+H)⁺ (Diastereomer 2).

### Beispiele 61A - 64A

### 4,4,4-Trifluor-3-methyl-2-{4-[(5-oxo-2-phenyl-5,6-dihydro-4H-1,3,4-oxadiazin-4-yl)methyl]-phenyl}butansäure (Isomer 1 - 4)

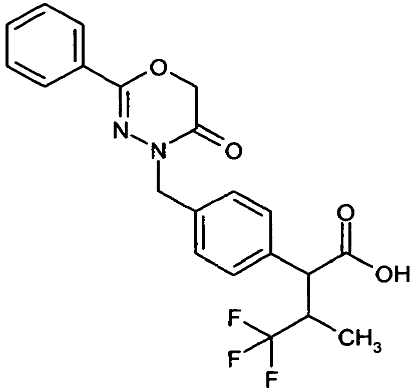

630 mg (1.50 mmol) des Isomerengemisches von 4,4,4-Trifluor-3-methyl-2-{4-[(5-oxo-2-phenyl-5,6-dihydro-4*H*-1,3,4-oxadiazin-4-yl)methyl]phenyl}butansäure wurden mittels präparativer HPLC an chiraler Phase in die Isomere aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 75:25 (v/v); Fluss: 15 ml/min; UV-Detektion: 220 nm; Temperatur: 30°C]:

### Beispiel 61 A (Isomer 1):

Ausbeute: 26 mg
Rₜ 6.17 min; Reinheit >99%; >99% ee
   [Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 25°C].

### Beispiel 62A (Isomer 2):

Ausbeute: 35 mg
Rₜ 6.57 min; Reinheit >98%; >99% ee
   [Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 25°C].

### Beispiel 63A (Isomer 3):

Ausbeute: 236 mg
Rₜ 8.03 min; Reinheit >99%; >99% ee
   [Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 25°C].
LC-MS (Methode 5): Rₜ = 1.12 min; m/z = 421 (M+H)⁺.
¹H-NMR (400 MHz, DMSO-d₆, δ/ppm): 12.60-12.81 (1H, br. s), 7.78 (2H, d), 7.41-7.53 (3H, m), 7.37 (4H, s), 4.93 (2H, s), 4.89 (2H, s), 3.61 (1H, d), 3.18-3.32 (1H, m), 0.77 (3H, d).
[α]_{D}²⁰ = +45.6°, c = 0.565, Methanol.

### Beispiel 64A (Isomer 4):

Ausbeute: 247 mg
Rₜ 9.17 min; Reinheit >99%; >98% ee
   [Säule: Daicel Chiralpak AD-H, 5 µm, 250 mm x 4.6 mm; Elutionsmittel: Isohexan/(Isopropanol + 0.2% Trifluoressigsäure + 1 % Wasser) 75:25 (v/v); Fluss: 1 ml/min; UV-Detektion: 220 nm; Temperatur: 25°C].
[α]_{D}²⁰ = -45.8°, c = 0.305, Methanol.

### Beispiel 65A

### (+/-)-Cyclopentyl{4-[(1-oxo-1,3-dihydro-2H-isoindol-2-yl)methyl]phenyl}essigsäure-tert.-butylester

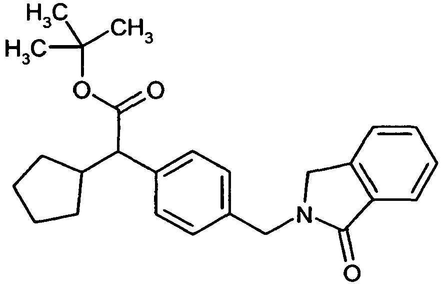

2.035 g (15.3 mmol) 1-Oxoindolin in 12 ml DMF wurden bei 0°C mit 611.3 mg (15.3 mmol, 60%-ig) Natriumhydrid versetzt. Es wurde 25 min gerührt und dann bei 0°C 6.0 g (12.7 mmol, ca. 75% Reinheit) (+/-)-[4-(Brommethyl)phenyl](cyclopentyl)essigsäure-*tert*.-butylester zugegeben. Man rührte das Reaktionsgemisch weitere 4 h unter langsamer Erwärmung auf RT, versetzte dann mit Wasser und extrahierte zweimal mit Dichlormethan. Die vereinigten organischen Phasen wurden mit gesättigter Natriumchlorid-Lösung gewaschen, über Magnesiumsulfat getrocknet und eingeengt. Das Rohprodukt wurde im Ultraschallbad mit Diethylether behandelt und der Feststoff abgesaugt und getrocknet. Es wurden 3.40 g (65.2% d. Th.) der Zielverbindung erhalten.
LC-MS (Methode 4): Rₜ = 1.05 min; m/z = 406 (M+H)⁺.
¹H-NMR (400 MHz, DMSO-*d₆*): δ = 7.73 (d, 1H), 7.63-7.48 (m, 3H), 7.31 (d, 2H), 7.22 (d, 2H), 4.71 (s, 2H), 4.39 (s, 2H), 3.18 (d, 1H), 2.47 (m, 1H), 1.82 (m, 1H), 1.65-1.36 (m, 4H), 1.35 (s, 9H), 1.30-1.20 (m, 2H), 0.95 (m, 1H).

### Beispiel 66A

### (+)-Cyclopentyl{4-[(1-oxo-1,3-dihydro-2H-isoindol-2-yl)methyl]phenyl}essigsäure-tert.-butylester

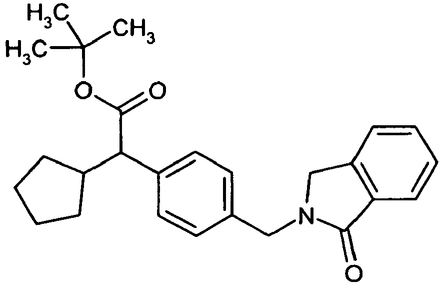

Das in Beispiel 65A erhaltene Racemat wurde durch präparative HPLC an chiraler Phase in die Enantiomere getrennt [Säule: Daicel Chiralpak IA-H, 5 µm, 250 mm x 20 mm; Fluss: 15 ml/min; UV-Detektion: 220 nm; Injektionsvolumen: 0.25 ml; Temperatur: 30°C; Eluent: 20% Acetonitril / 80% Methyl-*tert*.-butylether]. Ausgehend von 3.40 g Racemat wurden 1.50 g des (+)-Enantiomeren erhalten (das andere Enantiomer wurde nicht rein isoliert).
LC-MS (Methode 3): Rₜ = 1.58 min; m/z = 350 (M-C₄H₈+H)⁺, 406 (M+H)⁺.
¹H-NMR (400 MHz, DMSO-*d₆*): δ = 7.73 (d, 1H), 7.63-7.48 (m, 3H), 7.31 (d, 2H), 7.22 (d, 2H), 4.71 (s, 2H), 4.39 (s, 2H), 3.18 (d, 1H), 2.47 (m, 1H), 1.82 (m, 1H), 1.65-1.36 (m, 4H), 1.35 (s, 9H), 1.30-1.20 (m, 2H), 0.95 (m, 1H).
[α]_{D}²⁰ = + 8.2°, c = 0.38, Chloroform.

### Beispiel 67A

### (+)-Cyclopentyl{4-[(1-oxo-1,3-dihydro-2H-isoindol-2-yl)methyl]phenyl}ethansäure

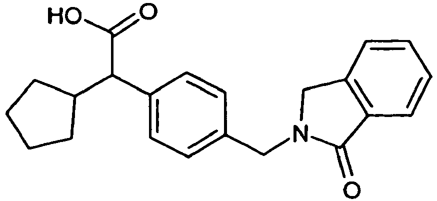

Zu einer Lösung von 300 mg (0.740 mmol) (+)-Cyclopentyl{4-[(1-oxo-1,3-dihydro-2*H*-isoindol-2-yl)methyl]phenyl}ethansäure-*tert*.-butylester in 1.5 ml Dichlormethan wurden 640 µl (7.4 mmol) Trifluoressigsäure getropft. Nach 1 h Rühren wurde das Reaktionsgemisch im Vakuum eingeengt und der Rückstand im Hochvakuum getrocknet. Es wurden 267 mg (100% d. Th.) der Titelverbindung erhalten.
LC-MS (Methode 5): Rₜ = 1.05 min; m/z = 350 (M+H)⁺.
¹H-NMR (400 MHz, DMSO-d₆): δ = 7.73 (d, 1H), 7.57 (q, 2H), 7.50 (t, 1H), 7.31 (d, 2H), 7.22 (d, 2H), 4.71 (s, 2H), 4.37 (s, 2H), 3.22 (d, 1H), 2.41 (m, 1H), 1.83 (m, 1H), 1.65-1.16 (m, 6H), 0.94 (m, 1H).

### Beispiel 68A

### 6-{[2-Cyclopentyl-2-{4-[(5-oxo-2-phenyl-5,6-dihydro-4H-1,3,4-oxadiazin-4-yl)methyl]phenyl}-acetyl]amino}heptansäure-tert.-butylester (Diastereomerengemisch)

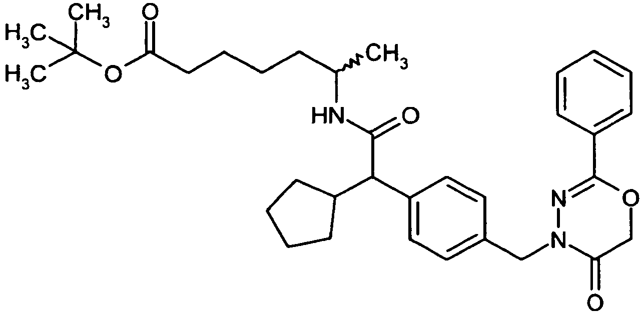

Zu einer Lösung von 100 mg (0.255 mmol) (+)-Cyclopentyl{4-[(5-oxo-2-phenyl-5,6-dihydro-4*H-*1,3,4-oxadiazin-4-yl)methyl]phenyl}essigsäure und 76.9 mg (0.382 mmol) (+/-)-6-Aminoheptan-säure-*tert*.-butylester in 0.3 ml DMF wurden bei RT 41.3 mg (0.306 mmol) HOBt und 126 µl (0.764 mmol) DIEA gegeben. Die resultierende Mischung wurde auf 0°C gekühlt, bevor 116.3 mg (0.306 mmol) HATU hinzugefügt wurden. Die Reaktionsmischung wurde langsam auf RT erwärmt, 1 h bei RT nachgerührt und dann mit Ethylacetat verdünnt. Die organische Phase wurde mit gesättigter Natriumhydrogencarbonat-Lösung und mit gesättigter Natriumchlorid-Lösung gewaschen, über Natriumsulfat getrocknet und im Vakuum eingeengt. Der Rückstand wurde durch präparative RP-HPLC (Acetonitril/Wasser-Gradient) aufgereinigt. Es wurden 118 mg (ca. 83% Reinheit, ca. 67% d. Th.) der Zielverbindung als Diastereomerengemisch erhalten.

LC-MS (Methode 3): Rₜ = 1.59 min; m/z = 576 (M+H)⁺.

### Beispiel 69A

### (+)-1-(4-{[2-Cyclopentyl-2-{4-[(5-oxo-2-phenyl-5,6-dihydro-4H-1,3,4-oxadiazin-4-yl)methyl]-phenyl}acetyl]amino}butyl)cyclopropancarbonsäure-tert.-butylester

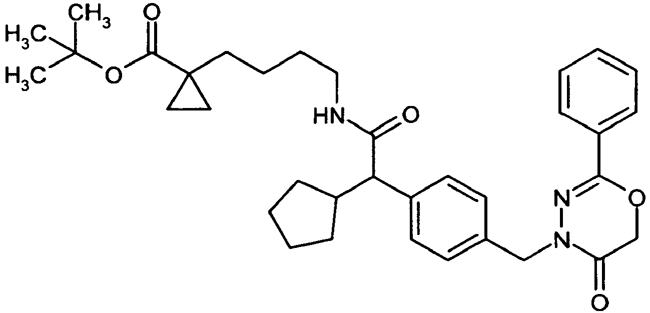

Zu einer Lösung von 545.8 mg (1.39 mmol) (+)-Cyclopentyl{4-[(5-oxo-2-phenyl-5,6-dihydro-4*H-*1,3,4-oxadiazin-4-yl)methyl]phenyl}essigsäure und 445 mg (ca. 2.09 mmol, Rohmaterial) 1-(4-Aminobutyl)cyclopropancarbonsäure-*tert*.-butylester in ca. 5 ml DMF wurden bei RT 266 µl (1.53 mmol) DIEA gegeben. Die resultierende Mischung wurde auf 0°C gekühlt, bevor in vier Portionen insgesamt 687.4 mg (2.09 mmol) HATU hinzugefügt wurden. Die Reaktionsmischung wurde langsam auf RT erwärmt, 1 h bei RT nachgerührt und dann auf Wasser gegeben und dreimal mit Ethylacetat extrahiert. Die vereinigten organischen Phasen wurden über Magnesiumsulfat getrocknet und im Vakuum eingeengt. Das Rohprodukt wurde zunächst durch Chromatographie an Silicagel vorgereinigt (Laufmittel-Gradient Cyclohexan/Ethylacetat 5:1 bis 3:1). Nach anschließender präparativer RP-HPLC (Acetonitril/Wasser-Gradient) wurden 378 mg (46.2% d. Th.) der Zielverbindung erhalten.
LC-MS (Methode 2): Rₜ = 2.73 min; m/z = 588 (M+H)⁺.
¹H-NMR (400 MHz, DMSO-d₆): δ = 7.94 (t, 1H), 7.78 (d, 2H), 7.52-7.44 (m, 3H), 7.30 (d, 2H), 7.27 (d, 2H), 4.90 (s, 2H), 4.83 (s, 2H), 3.10 (d, 1H), 3.10-3.01 (m, 1H), 2.89-2.81 (m, 1H), 2.51-2.45 (m, 1H), 1.73-1.67 (m, 1H), 1.65-1.28 (m, 11H), 1.35 (s, 9H), 1.22-1.15 (m, 1H), 0.95 (m, 2H), 0.94-0.86 (m, 1 H), 0.56 (m, 2H).
[α]_{D}²⁰ = +5.4°, c = 0.525, Chloroform.

### Beispiel 70A

### (+)-6-{[2-Cyclopentyl-2-{4-[(5-oxo-2-phenyl-5,6-dihydro-4H-1,3,4-oxadiazin-4-yl)methyl]-phenyl}acetyl]amino}-2,2-dimethylhexansäure-tert.-butylester

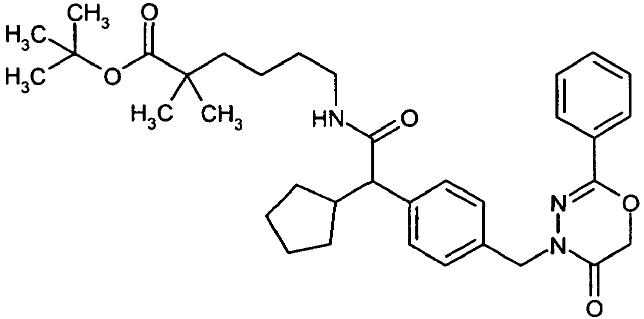

Zu einer Lösung von 328.1 mg (0.836 mmol) (+)-Cyclopentyl{4-[(5-oxo-2-phenyl-5,6-dihydro-4*H-*1,3,4-oxadiazin-4-yl)methyl]phenyl}essigsäure und 270 mg (ca. 1.25 mmol, Rohmaterial) 6-Amino-2,2-dimethylhexansäure-*tert*.-butylester in 3 ml DMF wurden bei RT 160 µl (0.919 mmol) DIEA gegeben. Die resultierende Mischung wurde auf 0°C gekühlt, bevor in vier Portionen insgesamt 413.2 mg (1.09 mmol) HATU hinzugefügt wurden. Die Reaktionsmischung wurde langsam auf RT erwärmt, 16 h bei RT nachgerührt und dann auf Wasser gegeben und dreimal mit Ethylacetat extrahiert. Die vereinigten organischen Phasen wurden über Natriumsulfat getrocknet und im Vakuum eingeengt. Nach präparativer RP-HPLC-Reinigung (Acetonitril/Wasser-Gradient) des Rückstands wurden 179.8 mg (36.5% d. Th.) der Zielverbindung erhalten.
LC-MS (Methode 3): Rₜ = 1.68 min; m/z = 590 (M+H)⁺.
¹H-NMR (400 MHz, DMSO-d₆): δ = 7.94 (t, 1H), 7.79 (d, 2H), 7.51-7.42 (m, 3H), 7.31 (d, 2H), 7.27 (d, 2H), 4.90 (s, 2H), 4.85 (s, 2H), 3.11 (d, 1H), 3.10-3.01 (m, 1H), 2.88-2.80 (m, 1H), 2.51-2.45 (m, 1H), 1.75-1.68 (m, 1H), 1.60-1.07 (m, 12H), 1.36 (s, 9H), 1.22-1.15 (m, 1H), 0.98 (s, 6H), 0.92-0.85 (m, 1H).
[α]_{D}²⁰ = +9.6°, c = 0.570, Chloroform.

### Beispiel 71A

### 6-{[2-Cyclopentyl-2-{4-[(5-oxo-2-phenyl-5,6-dihydro-4H-1,3,4-oxadiazin-4-yl)methyl]phenyl}-acetyl]amino}-2-methylhexansäureethylester (Diastereomerengemisch)

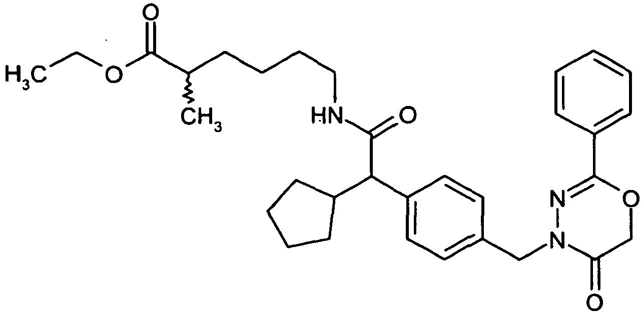

Zu einer Lösung von 148 mg (0.377 mmol) (+)-Cyclopentyl{4-[(5-oxo-2-phenyl-5,6-dihydro-4*H-*1,3,4-oxadiazin-4-yl)methyl]phenyl}essigsäure und 98 mg (ca. 0.66 mmol, Rohmaterial) 6-Amino-2-methylhexansäureethylester in 1.0 ml DMF wurden bei RT 72 µl (0.415 mmol) DIEA gegeben. Die resultierende Mischung wurde auf 0°C gekühlt, bevor in vier Portionen insgesamt 186.4 mg (0.49 mmol) HATU hinzugefügt wurden. Die Reaktionsmischung wurde langsam auf RT erwärmt, 16 h bei RT nachgerührt und dann auf Wasser gegeben und dreimal mit Ethylacetat extrahiert. Die vereinigten organischen Phasen wurden über Natriumsulfat getrocknet und im Vakuum eingeengt. Nach präparativer RP-HPLC-Reinigung (Acetonitril/Wasser-Gradient) des Rückstands wurden 134.0 mg (64.9% d. Th.) der Zielverbindung als Diastereomerengemisch erhalten.
LC-MS (Methode 3): Rₜ = 1.51 min; m/z = 548 (M+H)⁺.
¹H-NMR (400 MHz, DMSO-d₆): δ = 7.93 (t, 1H), 7.78 (d, 2H), 7.53-7.43 (m, 3H), 7.30 (d, 2H), 7.27 (d, 2H), 4.91 (s, 2H), 4.84 (s, 2H), 4.02 (q, 2H), 3.10 (d, 1H), 3.10-3.01 (m, 1H), 2.88-2.80 (m, 1H), 2.51-2.45 (m, 1H), 2.20 (q, 1H), 1.75-1.68 (m, 1H), 1.62-1.39 (m, 5H), 1.36-1.25 (m, 4H), 1.20-1.12 (m, darin t, zus. 5H), 0.99 (d, 3H), 0.93-0.85 (m, 1H).

### Beispiel 72A und Beispiel 73A

### 6-{[2-Cyclopentyl-2-{4-[(5-oxo-2-phenyl-5,6-dihydro-4H-1,3,4-oxadiazin-4-yl)methyl]phenyl}-acetyl]amino}-2-methylhexansäureethylester (Diastereomer 1 und 2)

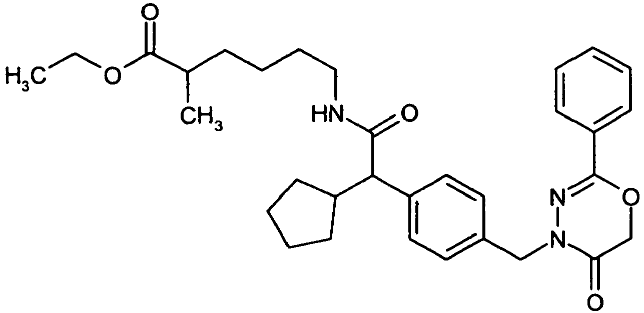

Das oben erhaltene Diastereomerengemisch (95 mg) wurde durch präparative HPLC an chiraler Phase aufgetrennt [Säule: Daicel Chiralpak IA, 5 µm, 250 mm x 20 mm; Injektionsvolumen: 3 ml; Eluent: 90% *tert*.-Butyl-methylether / 10% Methanol; Fluss: 25 ml/min; Temperatur: RT; Detektion: 260 nm]:

### Beispiel 72A (Diastereomer 1):

Ausbeute: 24 mg
   LC-MS (Methode 3): Rₜ = 1.51 min; m/z = 548 (M+H)⁺.
   ¹H-NMR (500 MHz, CDCl₃): δ = 7.82 (d, 2H), 7.48-7.30 (m, 7H), 5.48 (t, 1H), 4.91 (s, 2H), 4.75 (s, 2H), 4.11 (q, 2H), 3.30-3.20 (m, 1H), 3.14-3.08 (m, 1H), 2.94 (d, 1H), 2.61-2.55 (m, 1 H), 2.39-2.34 (m, 1H), 1.96-1.91 (m, 1H), 1.65-1.58 (m, 2H), 1.48-1.35 (m, 4H), 1.20-1.10 (m, darin t, zus. 5H), 1.10 (d, 3H), 0.99-0.84 (m, 2H).
   [α]_{D}²⁰ = +2°, c = 0.280, Chloroform.

### Beispiel 73A (Diastereomer 2):

Ausbeute: 23 mg
   LC-MS (Methode 3): Rₜ = 1.51 min; m/z = 548 (M+H)⁺.
   ¹H-NMR (500 MHz, CDCl₃): δ = 7.83 (d, 2H), 7.47-7.30 (m, 7H), 5.48 (t, 1H), 4.91 (s, 2H), 4.75 (s, 2H), 4.10 (q, 2H), 3.30-3.23 (m, 1H), 3.11-3.05 (m, 1H), 2.92 (d, 1H), 2.61-2.55 (m, 1H), 2.39-2.34 (m, 1H), 1.96-1.90 (m, 1H), 1.65-1.58 (m, 2H), 1.48-1.35 (m, 4H), 1.20-1.10 (m, darin t, zus. 5H), 1.10 (d, 3H), 1.00-0.84 (m, 2H).
   [α]_{D}²⁰ = +13°, c = 0.30, Chloroform.

### Beispiel 74A

### 1-(4-{[2-Cyclopentyl-2-{4-[(5-oxo-2-phenyl-5,6-dihydro-4H-1,3,4-oxadiazin-4-yl)methyl]phenyl}-acetyl]amino}pentyl)cyclopropancarbonsäure-tert.-butylester (Diastereomerengemisch)

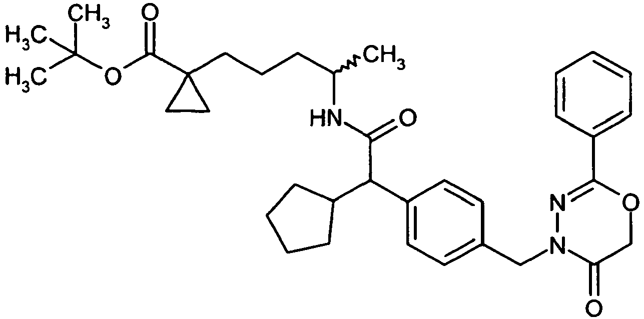

Zu einer Lösung von 517.9 mg (1.32 mmol) (+)-Cyclopentyl{4-[(5-oxo-2-phenyl-5,6-dihydro-4*H-*1,3,4-oxadiazin-4-yl)methyl]phenyl}essigsäure und 450 mg (ca. 1.98 mmol, Rohmaterial) 1-(4-Aminopentyl)cyclopropancarbonsäure-*tert*.-butylester in 2.5 ml DMF wurden bei RT 253 µl (1.45 mmol) DIEA gegeben. Die resultierende Mischung wurde auf 0°C gekühlt, bevor in vier Portionen insgesamt 186.4 mg (0.49 mmol) HATU hinzugefügt wurden. Die Reaktionsmischung wurde langsam auf RT erwärmt, 16 h bei RT nachgerührt und dann auf Wasser gegeben und dreimal mit Ethylacetat extrahiert. Die vereinigten organischen Phasen wurden über Magnesiumsulfat getrocknet und im Vakuum eingeengt. Nach präparativer RP-HPLC-Reinigung (Acetonitril/Wasser-Gradient) des Rohprodukts wurden 540.0 mg (68.0% d. Th.) der Zielverbindung als Diastereomerengemisch erhalten.
LC-MS (Methode 2): Rₜ = 2.79 min, m/z = 602 (M+H)⁺ und Rₜ = 2.83 min, m/z = 602 (M+H)⁺.

### Beispiel 75A und Beispiel 76A

### 1-(4-{[2-Cyclopentyl-2-{4-[(5-oxo-2-phenyl-5,6-dihydro-4H-1,3,4-oxadiazin-4-yl)methyl]phenyl}-acetyl]amino}pentyl)cyclopropancarbonsäure-tert.-butylester (Diastereomer 1 und 2)

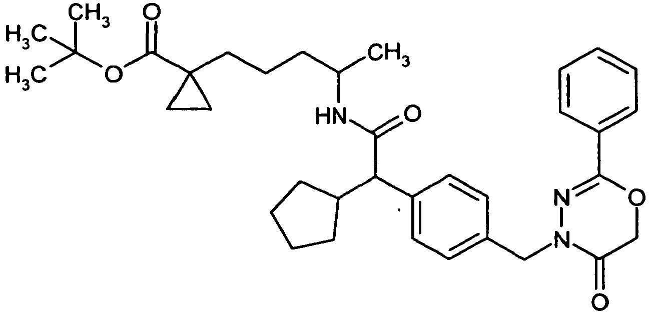

Das oben erhaltene Diastereomerengemisch (536 mg) wurde durch präparative HPLC an chiraler Phase aufgetrennt [Säule: Daicel Chiralpak IA, 5 µm, 250 mm x 20 mm; Injektionsvolumen: 2 ml; Eluent: 90% *tert*.-Butyl-methylether / 10% Methanol; Fluss: 20 ml/min; Temperatur: RT; Detektion: 260 nm]:

### Beispiel 75A (Diastereomer 1):

Ausbeute: 253 mg
LC-MS (Methode 2): Rₜ = 2.87 min; m/z = 602 (M+H)⁺.
¹H-NMR (400 MHz, DMSO-d₆): δ = 7.80-7.75 (m, 3H), 7.54-7.43 (m, 3H), 7.30 (d, 2H), 7.26 (d, 2H), 4.91 (s, 2H), 4.84 (s, 2H), 3.67 (m, 1H), 3.09 (d, 1 H), 2.52-2.43 (m, 1H), 1.76-1.68 (m, 1H), 1.63-1.56 (m, 1H), 1.55-1.28 (m, darin s, zus. 19H), 1.27-1.17 (m, 1H), 0.98 (s, 2H), 0.97-0.85 (m, 1H), 0.88 (d, 3H), 0.62 (d, 2H).
[α]_{D}²⁰ = +3.3°, c = 0.550, Chloroform.

### Beispiel 76A (Diastereomer 2):

Ausbeute: 273 mg
LC-MS (Methode 2): Rₜ = 2.82 min; m/z = 602 (M+H)⁺.
¹H-NMR (400 MHz, DMSO-d₆): δ = 7.80-7.72 (m, 3H), 7.52-7.42 (m, 3H), 7.30 (d, 2H), 7.26 (d, 2H), 4.90 (s, 2H), 4.84 (s, 2H), 3.65 (m, 1H), 3.10 (d, 1H), 2.52-2.43 (m, 1H), 1.75-1.68 (m, 1H), 1.63-1.57 (m, 1H), 1.56-1.10 (m, darin s, zus. 20H), 1.01 (d, 3H), 0.95-0.85 (m, 1H), 0.82 (d, 2H), 0.39 (dq, 2H).
[α]_{D}²⁰ = +5.2°, c = 0.555, Chloroform.

### Beispiel 77A

### (-)-1-(4-{[3-Methyl-2-{4-[(5-oxo-2-phenyl-5,6-dihydro-4H-1,3,4-oxadiazin-4-yl)methyl]phenyl}-pentanoyl]amino}butyl)cyclopropancarbonsäure-tert.-butylester

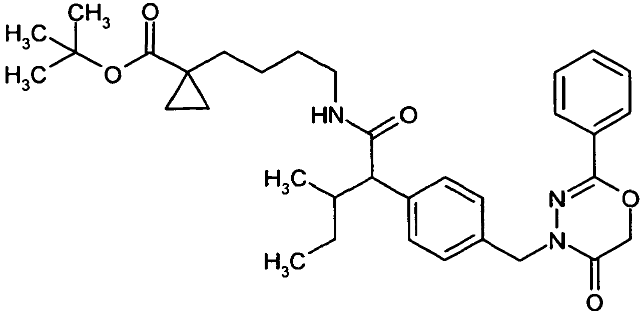

Zu einer Lösung von 210 mg (0.552 mmol) (+)-3-Methyl-2-{4-[(5-oxo-2-phenyl-5,6-dihydro-4*H-*1,3,4-oxadiazin-4-yl)methyl]phenyl}pentansäure (Isomer 2) und 177 mg (0.828 mmol) 1-(4-Aminobutyl)cyclopropancarbonsäure-*tert*.-butylester in 2.0 ml DMF wurden bei RT 106 µl (0.607 mmol) DIEA gegeben. Die resultierende Mischung wurde auf 0°C gekühlt, bevor in vier Portionen insgesamt 273 mg (0.718 mmol) HATU hinzugefügt wurden. Die Reaktionsmischung wurde langsam auf RT erwärmt, 16 h bei RT nachgerührt und dann auf Wasser gegeben und dreimal mit Ethylacetat extrahiert. Die vereinigten organischen Phasen wurden über Natriumsulfat getrocknet und im Vakuum eingeengt. Nach präparativer RP-HPLC-Reinigung (Acetonitril/Wasser-Gradient) des Rückstands wurden 264.0 mg (83.1 % d. Th.) der Zielverbindung erhalten.
LC-MS (Methode 2): Rₜ = 2.75 min; m/z = 576 (M+H)⁺.
¹H-NMR (400 MHz, DMSO-d₆): δ = 7.94 (t, 1H), 7.77 (d, 2H), 7.49 (q, 1H), 7.44 (t, 2H), 7.27 (q, 4H), 4.90 (s, 2H), 4.83 (s, 2H), 3.04 (m, 2H), 2.83 (m, 1H), 2.05 (m, 1H), 1.46 (m, 1H), 1.39-1.23 (m, 15H), 1.08 (m, 1H), 0.90 (s, 2H), 0.86 (t, 3H), 0.53 (m, 5H).
[α]_{D}²⁰ = -1.4°, c = 0.5, Chloroform.

### Beispiel 78A

### 1-[(1E/Z)-4-{[-2-Cyclopentyl-2-{4-[(5-oxo-2-phenyl-5,6-dihydro-4H-1,3,4-oxadiazin-4-yl)methyl]-phenyl}acetyl]amino} but-1-en-1-yl]cyclopropancarbonsäureethylester

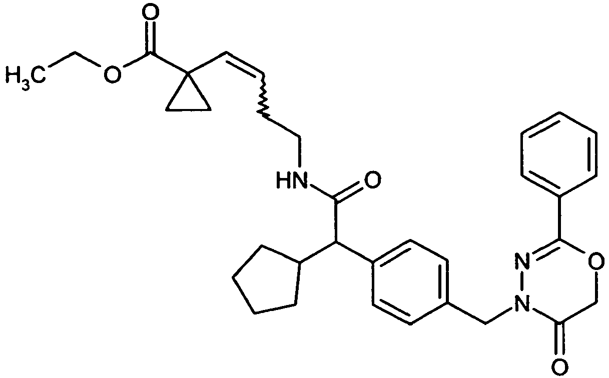

200 mg (0.51 mmol) (+)-Cyclopentyl{4-[(5-oxo-2-phenyl-5,6-dihydro-4*H*-1,3,4-oxadiazin-4-yl)-methyl]phenyl}essigsäure wurden in 0.5 ml DMF und 0.31 ml (3.82 mmol) Pyridin vorgelegt, mit 213.2 mg (0.561 mmol) 1-[Bis-(dimethylamino)methylen]-5-chlor-3-oxy-1*H*-benzotriazol-1-ium-Tetrafluoroborat sowie 93.4 mg (0.51 mmol) 1-[(1*E*/*Z*)-4-Aminobut-1-en-1-yl]cyclopropancarbon-säureethylester versetzt und bei RT über Nacht gerührt. Das Reaktionsgemisch wurde danach mit wenig Acetonitril verdünnt und direkt über präparative RP-HPLC aufgereinigt (Acetonitril/Wasser-Gradient). Es wurden 123 mg (41.8% d. Th.) der Zielverbindung als *E*/*Z*-Isomerengemisch (ca. 1:4) erhalten.
LC-MS (Methode 3): Rₜ = 1.51 min; m/z = 558 (M+H)⁺.
¹H-NMR (400 MHz, DMSO-d₆): δ = 8.00 (t, 1H), 7.77 (d, 2H), 7.47 (m, 3H), 7.28 (q, 4H), 5.47 (m, 1H), 4.90 (s, 2H), 4.83 (s, 2H), 3.98 (m, 2H), 3.09 (d, 2H), 2.92 (m, 1H), 2.12 (m, 2H), 1.70 (m, 1H), 1.64-1.36 (m, 4H), 1.34-1.07 (m, 7H), 0.89 (m, 1H), 0.80 (d, 2H).

### Beispiel 79A

### (+)-1-(4-{[2-Cyclopentyl-2-{4-[(1-oxo-1,3-dihydro-2H-isoindol-2-yl)methyl]phenyl}acetyl]-amino}butyl)cyclopropancarbonsäure-tert.-butylester

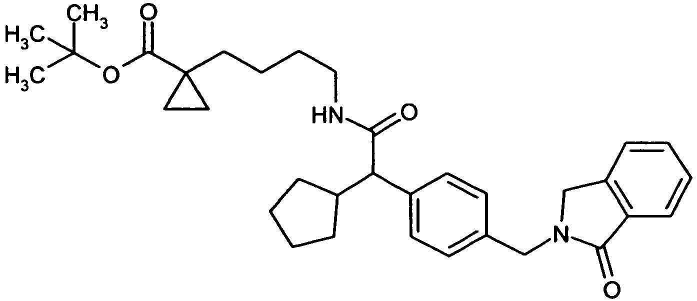

Zu einer Lösung von 260 mg (0.744 mmol) (+)-Cyclopentyl{4-[(1-oxo-1,3-dihydro-2*H*-isoindol-2-yl)methyl]phenyl}ethansäure und 120.7 mg (0.893 mmol) 1-Hydroxy-1*H*-benzotriazol-Hydrat in 2 ml DMF wurden bei 0°C unter Argon 390 µl (2.232 mmol) *N,N*-Diisopropylethylamin, 206.3 mg (0.967 mmol) 1-(4-Aminobutyl)cyclopropancarbonsäure-*tert*.-butylester und dann in Portionen insgesamt 339.5 mg (0.893 mmol) HATU gegeben. Es wurde zunächst 1 h bei 0°C und anschließend 2 h bei RT gerührt. Das Reaktionsgemisch wurde dann auf Wasser gegossen und dreimal mit Ethylacetat extrahiert. Die vereinigten organischen Phasen wurden über Magnesiumsulfat getrocknet und am Rotationsverdampfer eingeengt. Der Rückstand wurde mit wenig Acetonitril versetzt und über präparative RP-HPLC aufgereinigt (Acetonitril/Wasser-Gradient). Es wurden 325 mg (80.3% d. Th.) der Zielverbindung erhalten.
LC-MS (Methode 2): Rₜ = 2.51 min; m/z = 545 (M+H)⁺.
¹H-NMR (400 MHz, DMSO-d₆): δ = 7.93 (t, 1H), 7.72 (d, 1H), 7.67 (q, 2H), 7.50 (t, 1H), 7.30 (d, 2H), 7.17 (d, 2H), 4.68 (s, 2H), 4.36 (s, 2H), 3.09 (d, 1H), 3.05 (m, 1H), 2.85 (m, 1H), 1.69 (m, 1H), 1.62-1.24 (m, 21 H), 1.18 (m, 1H), 0.90 (q, 2H), 0.88 (m, 1H), 0.57 (q, 2H).
[α]_{D}²⁰ = +20.7°, c = 0.345, Chloroform.

### Beispiel 80A

### cis/trans-1-[4-{[2-Cyclopentyl-2-{4-[(5-oxo-2-phenyl-5,6-dihydro-4H-1,3,4-oxadiazin-4-yl)-methyl]phenyl}acetyl]amino}but-2-en-1-yl]cyclopropancarbonsäure-tert.-butylester

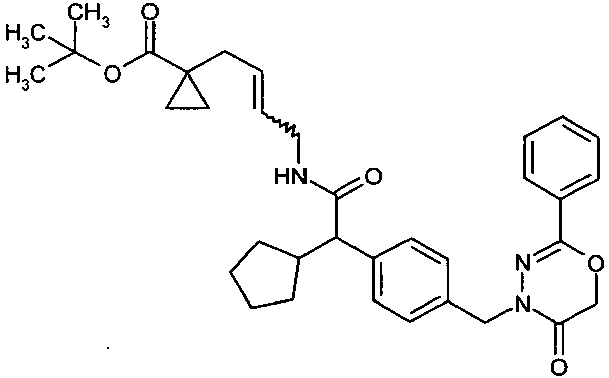

Zu einer Lösung von 107.2 mg (0.273 mmol) (+)-Cyclopentyl{4-[(5-oxo-2-phenyl-5,6-dihydro-4*H-*1,3,4-oxadiazin-4-yl)methyl]phenyl}ethansäure und 44.3 mg (0.328 mmol) 1-Hydroxy-1*H*-benzotriazol-Hydrat in 1.5 ml DMF wurden bei 0°C unter Argon 143 µl (0.819 mmol) *N,N*-Diisopropylethylamin, 75.0 mg (ca. 0.35 mmol, Rohmaterial) *cis*/*trans*-1-[(2*E*/*Z*)-4-Aminobut-2-en-1-yl]cyclopropancarbonsäure-tert.-butylester und dann in Portionen insgesamt 124.9 mg (0.328 mmol) HATU gegeben. Es wurde zunächst 1 h bei 0°C und anschließend 2 h bei RT gerührt. Das Reaktionsgemisch wurde dann auf Wasser gegossen und dreimal mit Ethylacetat extrahiert. Die vereinigten organischen Phasen wurden über Magnesiumsulfat getrocknet und im Vakuum eingeengt. Der Rückstand wurde mit wenig Acetonitril versetzt und über präparative RP-HPLC aufgereinigt (Acetonitril/Wasser-Gradient). Es wurden 121 mg (75.5% d.Th.) der Zielverbindung erhalten (trans-Isomer im deutlichen Überschuss).
LC-MS (Methode 3): Rₜ = 1.63 min; m/z = 530 (M-C₄H₈+H)⁺.

### Beispiel 81A

### trans-1-[(2E)-4-{[2-Cyclopentyl-2-{4-[(5-oxo-2-phenyl-5,6-dihydro-4H-1,3,4-oxadiazin-4-yl)-methyl]phenyl}acetyl]amino}but-2-en-1-yl]cyclopropancarbonsäure-tert.-butylester

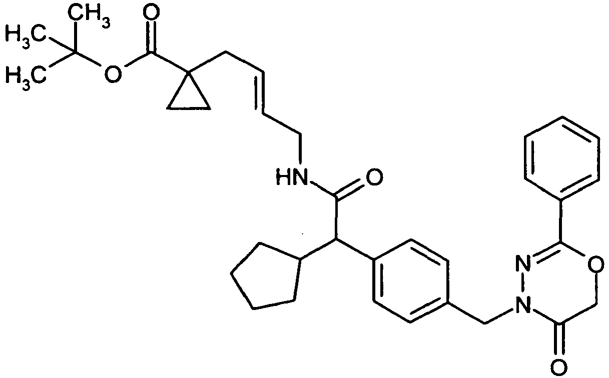

Das oben erhaltene *cis*/*trans*-Isomerengemisch (120 mg) wurde durch präparative HPLC aufgetrennt [Säule: Kromasil 100 C 18, 5 µm, 250 mm x 20 mm; Injektionsvolumen: 2 ml; Eluent: 70% Acetonitril / 30% wässrige Ameisensäure (0.2%); Fluss: 25 ml/min; Temperatur: 35°C; Detektion: 210 nm]:
Ausbeute: 67 mg
LC-MS (Methode 5): Rₜ = 1.43 min; m/z = 530 (M-C₄H₈+H)⁺, 608 (M+Na)⁺.
¹H-NMR (400 MHz, DMSO-d₆): δ = 8.11 (t, 1H), 7.78 (d, 2H), 7.53-7.42 (m, 4H), 7.34-7.26 (m, 4H), 5.50-5.41 (m, 1H), 5.39-5.30 (m, 1H), 4.92 (s, 2H), 4.83 (s, 2H), 3.68-3.61 (m, 1H), 3.51-3.44 (m, 1H), 3.14 (d, 1H), 2.54-2.45 (m, 1H), 2.11 (d, 2H), 1.87-1.37 (m, 6H), 1.35 (s, 9H), 1.34-1.15 (m, 2H), 0.92 (m, 2H), 0.93-0.85 (m, 1H), 0.60 (m, 2H).

### Beispiel 82A

### cis/trans-1-[4-{[2-Cyclopentyl-2-{4-[(6-oxo-3-phenylpyridazin-1(6H)-yl)methyl]phenyl}acetyl]-amino}but-1-en-1-yl]cyclopropancarbonsäureethylester

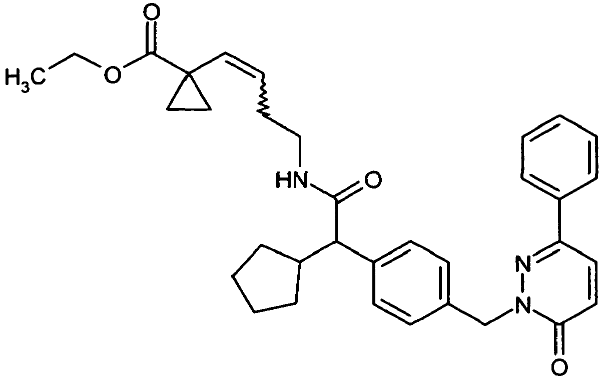

200 mg (0.515 mmol) (+)-(2*S*)-Cyclopentyl{4-[(6-oxo-3-phenylpyridazin-1(6*H*)-yl)methyl]-phenyl}ethansäure wurden in 1.2 ml DMF gelöst, auf 0°C gekühlt und nacheinander mit 83.5 mg (0.618 mmol) HOBt, 0.27 ml (1.55 mmol) DIEA, 113.2 mg (ca. 0.618 mmol, Rohmaterial) 1-[(1*E*/*Z*)-4-Aminobut-1-en-1-yl]cyclopropancarbonsäureethylester sowie portionsweise mit insgesamt 234.9 mg (0.618 mmol) HATU versetzt. Die Reaktionsmischung wurde 1 h bei 0°C gerührt und anschließend langsam auf RT erwärmt. Das Reaktionsgemisch wurde dann auf Wasser gegeben und mehrfach mit Ethylacetat extrahiert. Die vereinigten organischen Phasen wurden über Natriumsulfat getrocknet und im Vakuum eingeengt. Das Rohprodukt wurde mittels präparativer RP-HPLC gereinigt (Acetonitril/Wasser-Gradient). Es wurden 160 mg (56. 1 % d. Th.) der Zielverbindung als *cis*/*trans-*Isomerengemisch erhalten.
LC-MS (Methode 2): Rₜ = 2.41 min, m/z = 554 (M+H)⁺ und Rₜ = 2.45 min, m/z = 554 (M+H)⁺.

### Beispiel 83A

### trans-1-[(1E)-4-{[2-Cyclopentyl-2-{4-[(6-oxo-3-phenylpyridazin-(6H)-yl)methyl]phenyl}acetyl]-amino}but-1-en-1-yl]cyclopropancarbonsäureethylester

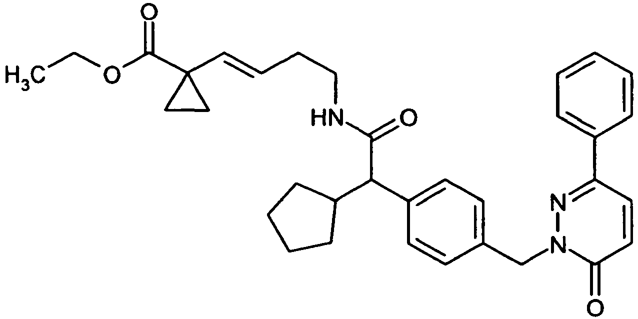

Das oben erhaltene *cis*/*trans*-Isomerengemisch wurde durch präparative HPLC aufgetrennt [Säule: Kromasil 100 C 18, 5 µm, 250 mm x 20 mm; Injektionsvolumen: 0.5 ml; Eluent: 75% Acetonitril / 25% Wasser; Fluss: 25 ml/min; Temperatur: 34.5°C; Detektion: 210 nm]. Ausgehend von 160 mg Gemisch wurden 11 mg des *trans*-Isomeren und 95 mg des cis-Isomeren (siehe Beispiel 84A) erhalten.
LC-MS (Methode 3): Rₜ = 1.46 min; m/z = 554 (M+H)⁺.
¹H-NMR (500 MHz, CDCl₃): δ = 7.78 (d, 2H), 7.49-7.40 (m, 3H), 7.31 (d, 2H), 7.01 (d, 1H), 6.02 (d, 1H), 5.59 (t, 1H), 5.48 (s, 2H), 5.22 (dt, 1H), 4.11 (q, 2H), 3.28 (m, 1H), 3.14 (m, 1H), 2.95 (d, 1H), 2.58 (m, 1H), 2.13 (m, 2H), 1.92 (m, 1H), 1.55-1.38 (m, 5H), 1.35 (s, 2H), 1.24 (t, 3H), 1.24-1.18 (m, 2H), 0.99-0.87 (m, 3H).

### Beispiel 84A

### (-)-cis-1-[(1Z)-4-{[2-Cyclopentyl-2-{4-[(6-oxo-3-phenylpyridazin-1(6H)-yl)methyl]phenyl}acetyl]-amino}but-1-en-1-yl]cyclopropancarbonsäureethylester

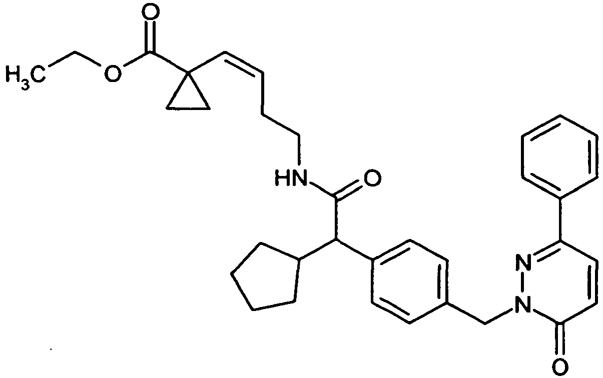

LC-MS (Methode 5): Rₜ = 1.31 min; m/z = 554 (M+H)⁺.
¹H-NMR (400 MHz, DMSO-d₆): δ = 8.08 (d, 1H), 8.01 (t, 1H), 7.89 (d, 2H), 7.51-7.43 (m, 3H), 7.32-7.27 (m, 4H), 7.08 (d, 1H), 5.49-5.43 (m, 2H), 5.30 (s, 2H), 3.99 (q, 2H), 3.13-3.05 (m, 2H), 2.92 (m, 1H), 2.52-2.43 (m, 1H), 2.15-2.10 (m, 2H), 1.75-1.65 (m, 1H), 1.52-1.11 (m, 8H), 1.10 (t, 3H), 0.91-0.82 (m, 1H), 0.79 (m, 2H).
[α]_{D}²⁰ = -21.1°, c = 0.520, Chloroform.

### Beispiel 85A

### Methyl-(1-{3-[(cyclopentyl{4-[(5-oxo-2-phenyl-5,6-dihydro-4H-1,3,4-oxadiazin-4-yl)methyl]-phenyl}acetyl)amino]propyl}cyclopropyl)acetat

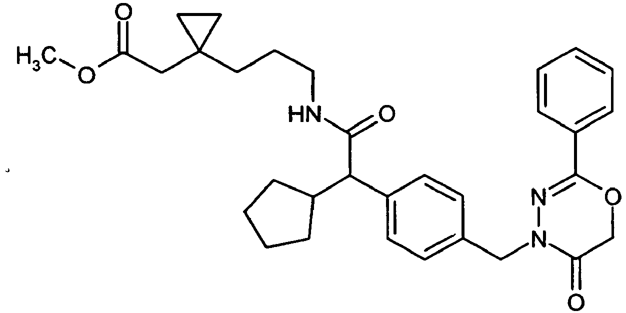

Eine Lösung von 591 mg (1.51 mmol) Cyclopentyl{4-[(5-oxo-2-phenyl-5,6-dihydro-4*H*-1,3,4-oxadiazin-4-yl)methyl]phenyl}essigsäure (Enantiomer 2), 376 mg (1.81 mmol) Methyl-[1-(3-aminopropyl)cyclopropyl]acetat-Hydrochlorid, 859 mg (2.26 mmol) HATU und 1 ml *N,N*-Diisopropylethylamin in 10 ml DMF wurde über Nacht bei Raumtemperatur gerührt. Nach Beendigung der Reaktion wurde der Ansatz auf Eiswasser gegossen, die Phasen getrennt und die wässrige Phase dreimal mit tert.-Butyl-methylether extrahiert. Die vereinigten organischen Phasen wurden über Natriumsulfat getrocknet, und nach Filtration wurde das Lösungsmittel im Vakuum bis zur Trockene entfernt. Das erhaltene Rohprodukt wurde über präparative RP-HPLC gereinigt. Es wurden 233 mg (0.43 mmol, 28.5% d. Th.) der Titelverbindung als farbloses Öl erhalten.
LC-MS (Methode 2): Rₜ = 2.41 min; m/z = 546 (M+H)⁺.

### Beispiel 86A

### tert.-Butyl-1-{4-[(cyclopentyl{4-[(6-oxo-3-phenylpyridazin-1(6H)-yl)methyl]phenyl}acetyl)amino]butyl}cyclopropancarboxylat

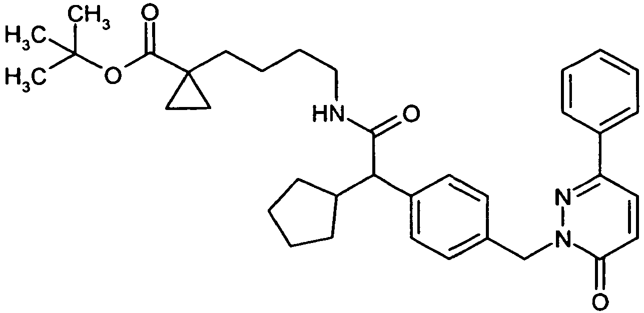

Eine Lösung von 113 mg (0.29 mmol) Cyclopentyl{4-[(6-oxo-3-phenylpyridazin-1(6*H*)-yl)-methyl]phenyl}essigsäure (Enantiomer 1), 75 mg (0.35 mmol) *tert*.-Butyl-1-(4-aminobutyl)cyclopropancarboxylat, 167 mg (0.44 mmol) HATU und 0.15 ml (0.88 mmol) *N,N*-Diisopropylethylamin in 3.5 ml DMF wurde über Nacht bei Raumtemperatur gerührt. Nach Beendigung der Reaktion wurde der Ansatz auf Eiswasser gegossen, die Phasen getrennt und die wässrige Phase dreimal mit *tert*.-Butyl-methylether extrahiert. Die vereinigten organischen Phasen wurden über Natriumsulfat getrocknet, und nach Filtration wurde das Lösungsmittel im Vakuum bis zur Trockene entfernt. Es wurden 197 mg der rohen Titelverbindung erhalten, welche ohne weitere Aufreinigung in der Folgeraktion eingesetzt wurde.
LC-MS (Methode 5): Rₜ = 1.42 min; m/z = 584 (M+H)⁺, 528 (M-C₄H₈+H)⁺.

### Beispiel 87A

### Methyl-7-[(cyclopentyl{4-[(5-oxo-2-phenyl-5,6-dihydro-4H-1,3,4-oxadiazin-4-yl)methyl]phenyl}-acetyl)amino]heptanoat

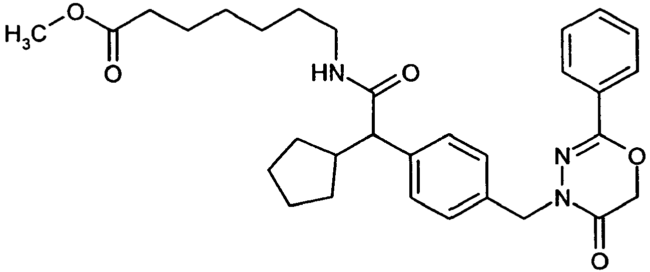

Eine Lösung von 72 mg (0.18 mmol) Cyclopentyl{4-[(5-oxo-2-phenyl-5,6-dihydro-4*H*-1,3,4-oxadiazin-4-yl)methyl]phenyl}essigsäure (Enantiomer 2), 30 mg (0.15 mmol) Methyl-7-aminoheptanoat-Hydrochlorid, 87 mg (0.23 mmol) HATU und 0.8 ml Pyridin in 3.2 ml DMF wurde über Nacht bei Raumtemperatur gerührt. Nach Beendigung der Reaktion wurde der Ansatz auf Eiswasser gegossen, die Phasen getrennt und die wässrige Phase dreimal mit tert.-Butyl-methylether extrahiert. Die vereinigten organischen Phasen wurden über Natriumsulfat getrocknet, und nach Filtration wurde das Lösungsmittel im Vakuum bis zur Trockene entfernt. Das erhaltene Rohprodukt wurde über präparative RP-HPLC gereinigt. Es wurden 13 mg (0.02 mmol, 13% d. Th.) der Titelverbindung als farbloses Öl erhalten.
LC-MS (Methode 4): Rₜ = 2.79 min; m/z = 534 (M+H)⁺.

In Analogie zu Beispiel 85A wurden die in der folgenden Tabelle aufgeführten Verbindungen erhalten:

| **Beispiel** | **Name / Struktur / Edukte** | **Analytische Daten** |
|---|---|---|
| **88A** | Methyl-4-[(cyclopentyl {4-[(5-oxo-2-phenyl-5,6-dihydro-4*H*-1,3,4-oxadiazin-4-yl)methyl]phenyl}-acetyl)amino]butanoat | LC-MS (Methode 4): Rₜ = 2.62 min; m/z = 492 (M+H)⁺. |
| | 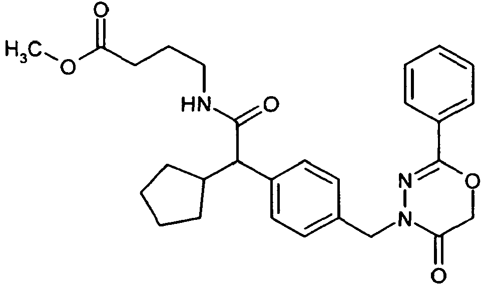 | |
| | (aus Cyclopentyl{4-[(5-oxo-2-phenyl-5,6-dihydro-4*H*-1,3,4-oxadiazin-4-yl)methyl]phenyl}essigsäure (*Enantiomer 2*) und Methyl-4-aminobutanoat-Hydrochlorid) | |
| **89A** | Methyl-5-[(cyclopentyl{4-[(5-oxo-2-phenyl-5,6-dihydro-4*H-*1,3,4-oxadiazin-4-yl)methyl]phenyl}-acetyl)amino]pentanoat | LC-MS (Methode 4): Rₜ = 2.62 min; m/z = 506 (M+H)⁺. |
| | 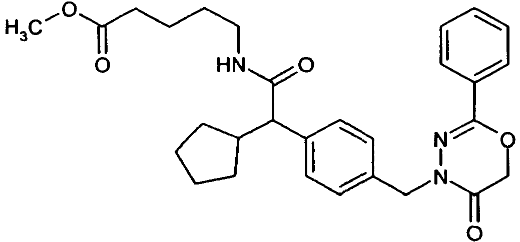 | |
| | (aus Cyclopentyl{4-[(5-oxo-2-phenyl-5,6-dihydro-4*H*-1,3,4-oxadiazin-4-yl)methyl]phenyl}essigsäure (*Enantiomer 2*) und Methyl-5-aminopentanoat-Hydrochlorid) | |
| **90A** | Methyl-6-[(cyclopentyl{4-[(5-oxo-2-phenyl-5,6-dihydro-4*H*-1,3,4-oxadiazin-4-yl)methyl]phenyl}-acetyl)amino]hexanoat | LC-MS (Methode 4): Rₜ = 2.70 min; m/z = 520 (M+H)⁺. |
| | 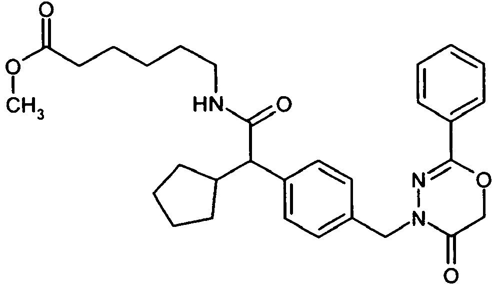 | |
| | (aus Cyclopentyl{4-[(5-oxo-2-phenyl-5,6-dihydro-4*H*-1,3,4-oxadiazin-4-yl)methyl]phenyl}essigsäure (*Enantiomer 2*) und Methyl-6-aminohexanoat-Hydrochlorid) | |
| **91A** | Methyl-6-[(5,5,5-trifluor-2-{4-[(5-oxo-2-phenyl-5,6-dihydro-4*H*-1,3,4-oxadiazin-4-yl)methyl]phenyl}-pentanoyl)amino]hexanoat | LC-MS (Methode 2): Rₜ = 2.24 min; m/z = 548 (M+H)⁺. |
| | 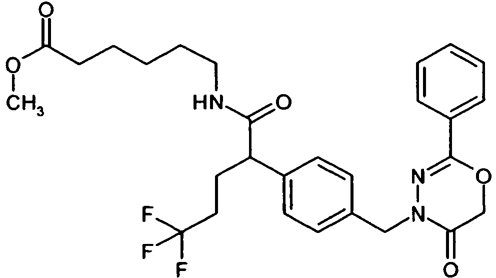 | |
| | (aus 5,5,5-Trifluor-2-{4-[(5-oxo-2-phenyl-5,6-dihydro-4*H*-1,3,4-oxadiazin-4-yl)methyl]phenyl}pentansäure und Methyl-6-aminohexanoat-Hydrochlorid) | |
| **92A** | *tert*.-Butyl-1-{4-[(4,4,4-trifluor-3-methyl-2-{4-[(5-oxo-2-phenyl-5,6-dihydro-4*H*-1,3,4-oxadiazin-4-yl)methyl]-phenyl}butanoyl)amino]butyl}cyclopropancarboxylat | LC-MS (Methode 5): Rₜ = 1.39 min; m/z = 616 (M+H)⁺. |
| | 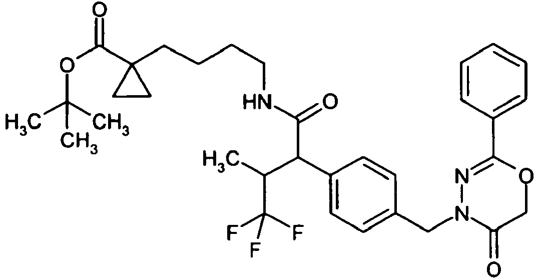 | ¹H-NMR (400 MHz, DMSO-d₆, δ/ppm): 8.11 (1H, t), 7.77 (2H, d), 7.40-7.54 (3H, m), 7.33 (4H, s), 4.93 (2H, s), 4.88 (2H, s), 3.55 (1H, d), 3.17-3.29 (1H, m), 2.99-3.10 (1H, m), 2.76-2.88 (1H, m), 1.30-1.39 (2H, m), 1.35 (9H, s), 1.21-1.30 (4H, m), 0.87-0.96 (2H, m), 0.71 (3H, d), 0.51-0.60 (2H, m). |
| | (aus 4,4,4-Trifluor-3-methyl-2-{4-[(5-oxo-2-phenyl-5,6-dihydro-4*H*-1,3,4-oxadiazin-4-yl)methyl]phenyl}-butansäure (*Isomer 3*) und *tert*.-Butyl-1-(4-aminobutyl)cyclopropancarboxylat) | |

### Ausführungsbeispiele:

### Allgemeine Vorschrift 3: Spaltung von tert.-Butylestern zu den entsprechenden Carbonsäuren

Zu einer Lösung des *tert*.-Butylesters in Dichlormethan (Konzentration 0.1 bis 1.0 mol/l; zusätzlich optional ein Tropfen Wasser) wird bei 0°C bis RT tropfenweise Trifluoressigsäure (TFA) hinzugefügt, bis ein Verhältnis Dichlormethan/TFA von ca. 2:1 bis 1:2 erreicht ist. Die Reaktionsmischung wird 1-18 h bei RT gerührt (gegebenenfalls wird die Mischung auf 40°C erwärmt, bis vollständiger Umsatz erreicht ist) und dann im Vakuum eingeengt. Das Reaktionsprodukt kann, falls erforderlich, durch Kristallisation aus Wasser/Acetonitril-Gemischen oder durch präparative RP-HPLC (Eluent: Acetonitril/Wasser-Gradient) gereinigt werden.

Die folgenden Beispiele wurden gemäß der Allgemeinen Vorschrift 3 hergestellt:

| **Beispiel** | **Name / Struktur / Ausgangsmaterial** | **Analytische Daten** |
|---|---|---|
| **1** | (+)-1-(4-{[2-Cyclopentyl-2-{4-[(5-oxo-2-phenyl-5,6-dihydro-4*H*-1,3,4-oxadiazin-4-yl)methyl]phenyl}acetyl]amino} butyl)cyclopropancarbonsäure | LC-MS (Methode 2): Rₜ = 2.15 min; m/z = 532 (M+H)⁺. |
| | 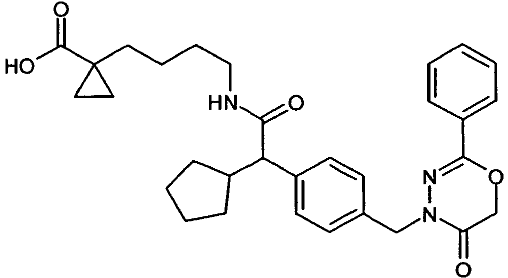 | ¹H-NMR (400 MHz, DMSO-d₆): δ = 11.98 (s, 1H), 7.93 (t, 1H), 7.78 (d, 2H), 7.53-7.42 (m, 3H), 7.30 (d, 2H), 7.27 (d, 2H), 4.90 (s, 2H), 4.84 (s, 2H), 3.10 (d, 1H), 3.09-3.01 (m, 1H), 2.88-2.80 (m, 1H), 2.51-2.45 (m, 1H), 1.76-1.26 (m, 12H), 1.24-1.15 (m, 1H), 0.98 (d, 2H), 0.95-0.85 (m, 1H), 0.60 (d, 2H). [α]_{D}²⁰ = +11.5°, c = 0.500, Chloroform. |
| | aus (+)-1-(4-{[2-Cyclopentyl-2-{4-[(5-oxo-2-phenyl-5,6-dihydro-4*H*-1,3,4-oxadiazin-4-yl)methyl]phenyl}acetyl]amino} butyl)cyclopropancarbonsäure-*tert*.-butylester | |

| **Beispiel** | **Name / Struktur / Ausgangsmaterial** | **Analytische Daten** |
|---|---|---|
| **2** | (+)-6-{[2-Cyclopentyl-2-{4-[(5-oxo-2-phenyl-5,6-dihydro-4*H*-1,3,4-oxadiazin-4-yl)methyl]phenyl}-acetyl]amino}-2,2-dimethylhexansäure | LC-MS (Methode 3): Rₜ = 1.37 min; m/z = 534 (M+H)⁺. |
| | 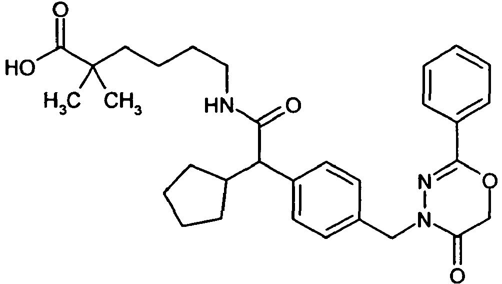 | ¹H-NMR (400 MHz, DMSO-d₆): δ = 12.00 (br. s, 1H), 7.95 (t, 1H), 7.79 (d, 2H), 7.51-7.42 (m, 3H), 7.31 (d, 2H), 7.27 (d, 2H), 4.90 (s, 2H), 4.34 (s, 2H), 3.11 (d, 1H), 3.09-3.02 (m, 1H), 2.88-2.80 (m, 1 H), 2.51-2.45 (m, 1H), 1.76-1.66 (m, 1H), 1.63-1.24 (m, 10H), 1.23-1.09 (m, 4H), 1.01 (s, 6H), 0.95-0.80 (m, 2H). |
| | aus (+)-6-{[2-Cyclopentyl-2-{4-[(5-oxo-2-phenyl-5,6-dihydro-4*H*-1,3,4-oxadiazin-4-yl)methyl]phenyl}-acetyl]amino}-2,2-dimethylhexansäure-*tert*.-butylester | |
| | | [α]_{D}²⁰ = +15.2°, c = 0.520, Chloroform. |
| **3** | 1-(4-{[2-Cyclopentyl-2-{4-[(5-oxo-2-phenyl-5,6-dihydro-4*H*-1,3,4-oxadiazin-4-yl)methyl]phenyl}-acetyl]amino}pentyl)cyclopropancarbonsäure | LC-MS (Methode 2): Rₜ = 2.22 min; m/z = 546 (M+H)⁺. |
| | 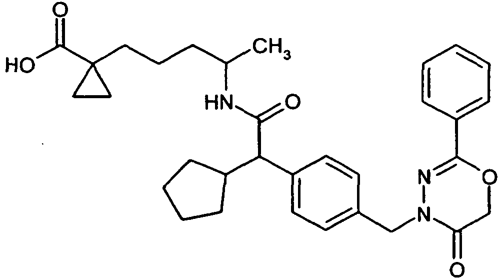 | ¹H-NMR(400 MHz, DMSO-d₆): δ = 11.99 (s, 1H), 7.80-7.75 (m, 3H), 7.53-7.43 (m, 3H), 7.31 (d, 2H), 7.27 (d, 2H), 4.90 (s, 2H), 4.84 (s, 2H), 3.67 (m, 1H), 3.09 (d, 1H), 2.52-2.46 (m, 1H), 1.77-1.67 (m, 1H), 1.66-1.28 (m, 11H), 1.25-1.15 (m, 1H), 1.01 (m, 2H), 0.94-0.85 (m, 1H), 0.89 (d, 3 H), 0.64 (m, 2H). |
| | aus (+)-1-(4-{[2-Cyclopentyl-2-{4-[(5-oxo-2-phenyl-5,6-dihydro-4*H*-1,3,4-oxadiazin-4-yl)methyl]phenyl}-acetyl]amino}pentyl)cyclopropancarbonsäure-*tert*.-butylester (*Diastereomer 1*) | |

| **Beispiel** | **Name / Struktur / Ausgangsmaterial** | **Analytische Daten** |
|---|---|---|
| **4** | (+)-1-(4-{[2-Cyclopentyl-2-{4-[(5-oxo-2-phenyl-5,6-dihydro-4*H*-1,3,4-oxadiazin-4-yl)methyl]phenyl}-acetyl]amino} pentyl)cyclopropancarbonsäure | LC-MS (Methode 2): Rₜ = 2.25 min; m/z = 546 (M+H)⁺. |
| | 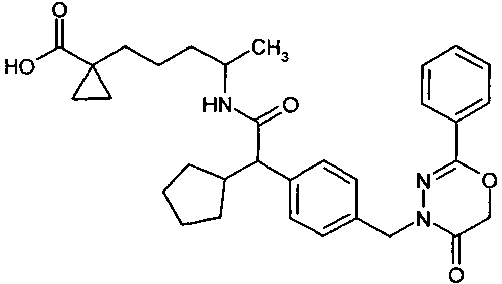 | ¹H-NMR (400 MHz, DMSO-d₆): δ = 11.92 (s, 1H), 7.80-7.74 (m, 3H), 7.53-7.43 (m, 3H), 7.31 (d, 2H), 7.28 (d, 2H), 4.90 (s, 2H), 4.84 (s, 2H), 3.65 (m, 1H), 3.09 (d, 1H), 2.52-2.46 (m, 1H), 1.75-1.65 (m, 1H), 1.62-1.10 (m, 12H), 0.99 (d, 3H), 0.94-0.85(m, 1H), 0.87 (s, 2H), 0.59(dq, 2H). |
| | aus (+)-1-(4-{[2-Cyclopentyl-2-{4-[(5-oxo-2-phenyl-5,6-dihydro-4*H*-1,3,4-oxadiazin-4-yl)methyl]phenyl}-acetyl]amino} pentyl)cyclopropancarbonsäure-*tert*.-butylester (*Diastereomer 2*) | [α]_{D}²⁰ = +22.1°, c = 0.490, Chloroform. |
| **5** | (+)-1-(4-{[3-Methyl-2-{4-[(5-oxo-2-phenyl-5,6-dihydro-4*H*-1,3,4-oxadiazin-4-yl)methyl]phenyl}-pentanoyl]amino}butyl)cyclopropancarbonsäure | LC-MS (Methode 2): Rₜ = 2.15 min; m/z = 520 (M+H)⁺. |
| | 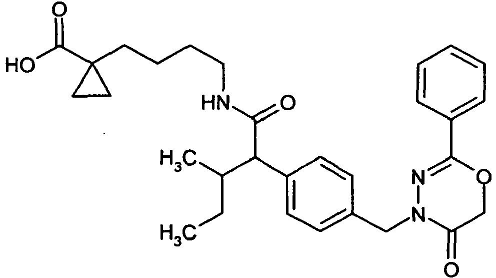 | ¹H-NMR (400 MHz, DMSO-d₆): δ = 7.96 (t, 1H), 7.77 (d, 2H), 7.47 (m, 3H), 7.28 (t, 4H), 4.90 (s, 2H), 4.83 (s, 2H), 3.06 (d, 2H), 2.80 (m, 1H), 2.06 (m, 1), 1.47 (m, 1 H), 1.36 (m, 2H), 1.29 (m, 4H), 1.09 (m, 1H), 0.97 (m, 2H), 0.87 (t, 3H), 0.56 (m,5H). |
| | aus (+)-1-(4-{[3-Methyl-2-{4-[(5-oxo-2-phenyl-5,6-dihydro-4*H*-1,3,4-oxadiazin-4-yl)methyl]phenyl}-pentanoyl]amino}butyl)cyclopropancarbonsäure-*tert*.-butylester | [α]_{D}²⁰ = +5.5°, c = 0.495, Chloroform. |

| **Beispiel** | **Name / Struktur / Ausgangsmaterial** | **Analytische Daten** |
|---|---|---|
| **6** | (+)-1-(4-{[2-Cyclopentyl-2-{4-[(1-oxo-1,3-dihydro-2*H*-isoindol-2-yl)methyl]phenyl}acetyl]amino}butyl)-cyclopropancarbonsäure | LC-MS (Methode 3): Rₜ = 1.21 min; m/z = 489 (M+H)⁺. |
| | 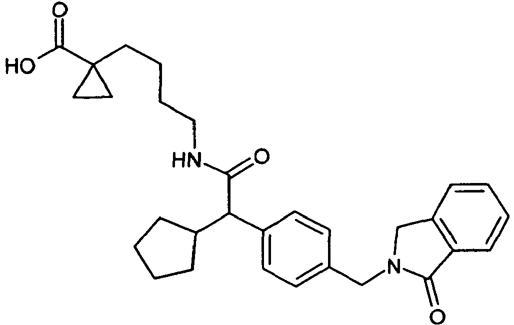 | ¹H-NMR (400 MHz, DMSO-d₆): δ = 11.97 (s, 1H), 7.91 (t, 1H), 7.72 (d, 1H), 7.56 (m, 2H), 7.50 (t, 1H), 7.30 (d, 2H), 7.19 (d, 2H), 4.68 (s, 2H), 4.35 (s, 2H), 3.09 (d, 1H), 3.05 (m, 1H), 2.85 (m, 1H), 2.45 (m, 1H), 1.70 (m, 1H), 1.62-1.24 (m, 10H), 1.17 (m, 2H), 0.96 (d, 2H),0.88 (m, 1H), 0.59 (d, 2H). |
| | aus (+)-1-(4-{[2-Cyclopentyl-2-{4-[(1-oxo-1,3-dihydro-2*H*-isoindol-2-yl)methyl]phenyl}acetyl]-amino}butyl)cyclopropancarbonsäure-*tert*.-butylester | [α]_{D}²⁰ = +24.5°, c = 0.360, Chloroform. |
| **7** | 6-{[(2*S*)-2-Cyclopentyl-2-{4-[(5-oxo-2-phenyl-5,6-dihydro-4*H*-1,3,4-oxadiazin-4-yl)methyl]phenyl}-acetyl]amino}heptansäure (*Diastereomerengemisch*) | LC-MS (Methode 3): Rₜ = 1.30 min, m/z = 520 (M+H)⁺ und Rₜ = 1.31 min, m/z = 520(M+H)⁺. |
| | 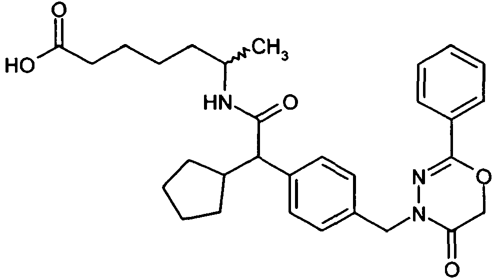 | |
| | aus 6-{[(2*S*)-2-Cyclopentyl-2-{4-[(5-oxo-2-phenyl-5,6-dihydro-4*H*-1,3,4-oxadiazin-4-yl)methyl]phenyl}-acetyl]amino} heptansäure-*tert*.-butylester *(Diastereomerengemisch)* | |

### Beispiel 8 und Beispiel 9

### (+)-6-{[2-Cyclopentyl-2-{4-[(5-oxo-2-phenyl-5,6-dihydro-4H-1,3,4-oxadiazin-4-yl)methyl]-phenyl}acetyl]amino}heptansäure (Diastereomer 1 und 2)

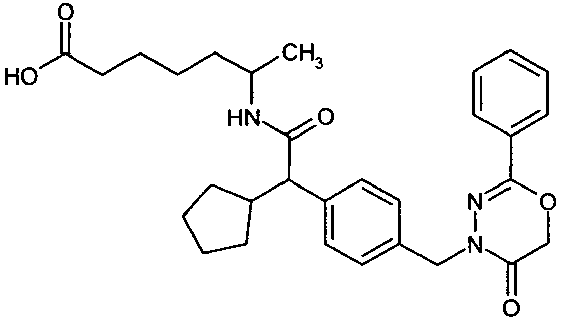

Das in Beispiel 7 erhaltene Diastereomerengemisch (50 mg) wurde durch präparative HPLC an chiraler Phase aufgetrennt [Säule: Daicel Chiralpak OJ-H, 5 µm, 250 mm x 20 mm; Injektionsvolumen: 0.6 ml; Eluent: 70% *iso*-Hexan / 30% Ethanol; Fluss: 20 ml/min; Temperatur: RT; Detektion: 230 nm]:

### Beispiel 8 (Diastereomer 1):

Ausbeute: 21.8 mg
LC-MS (Methode 3): Rₜ = 1.32 min; m/z = 520 (M+H)⁺.
¹H-NMR (400 MHz, DMSO-d₆): δ = 11.91 (br. s, 1H), 7.79-7.71 (m, 3H), 7.52-7.42 (m, 3H), 7.31 (d, 2H), 7.27 (d, 2H), 4.91 (s, 2H), 4.84 (s, 2H), 3.63 (m, 1H), 3.10 (d, 1H), 2.52-2.46 (m, 1H), 2.00 (t, 2H), 1.75-1.68 (m, 1H), 1.65-1.25 (m, 10H), 1.05-0.98 (m, 2H), 1.00 (d, 3H), 0.93-0.82 (m, 1H).
[α]_{D}²⁰ = +13.0°, c = 0.250, Chloroform.

### Beispiel 9 (Diastereomer 2):

Ausbeute: 22.7 mg
LC-MS (Methode 4): Rₜ = 2.53 min; m/z = 520 (M+H)⁺.
¹H-NMR (500 MHz, CDCl₃): δ = 7.83 (d, 2H), 7.48-7.29 (m, 7H), 5.22 (br. d, 1H), 4.93 (s, 2H), 4.78 (s, 2H), 3.92 (m, 1H), 2.98 (d, 1H), 2.58-2.50 (m, 1H), 1.95-1.88 (m, 1H), 1.70-1.51 (m, 5H), 1.50-1.37 (m, 4H), 1.33-1.20 (m, 4H), 1.00 (d, 3H), 1.00-0.95 (m, 1H).
[α]_{D}²⁰ = +5.0°, c = 0.265, Chloroform.

### Allgemeine Vorschrift 4: Hydrolyse von Methyl- oder Ethylestern zu den entsprechenden Carbonsäuren

Zu einer Lösung des Methyl- oder Ethylesters in THF, THF/Methanol oder THF/Ethanol (Konzentration ca. 0.05 bis 0.5 mol/l) werden bei 0°C bis RT 1.5 bis 5 eq. Lithiumhydroxid gegeben. Die Mischung wird für einen Zeitraum von 0.5-18 h gerührt (dabei Erwärmung auf RT) und dann mit 1 N Salzsäure neutralisiert oder schwach angesäuert. Kommt es dabei zum Ausfallen eines Feststoffs, kann das Produkt durch Filtration, Waschen mit Wasser und Trocknen im Hochvakuum isoliert werden. Alternativ wird die Zielverbindung direkt aus dem Rohprodukt oder nach extraktiver Aufarbeitung mit Dichlormethan oder Ethylacetat durch präparative RP-HPLC isoliert (Eluent: Wasser/Acetonitril-Gradient).

Die folgenden Beispiele wurden gemäß der Allgemeinen Vorschrift 4 hergestellt:

| **Beispiel** | **Name / Struktur / Ausgangsmaterial** | **Analytische Daten** |
|---|---|---|
| **10** | (+)-6-{[2-Cyclopentyl-2- {4-[(5-oxo-2-phenyl-5,6-dihydro-4*H*-1,3,4-oxadiazin-4-yl)methyl]phenyl}-acetyl]amino}-2-methylhexansäure (*Diastereomer 1*) | LC-MS (Methode 4): Rₜ = 2.51 min; m/z = 520 (M+H)⁺. |
| | 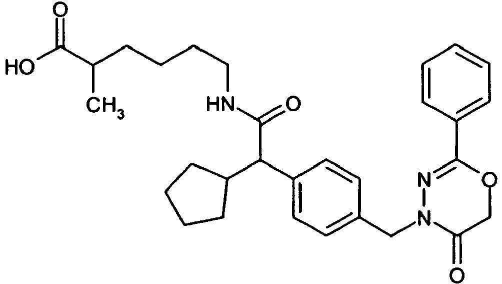 | ¹H-NMR (400 MHz, DMSO-d₆): 12.00 (br. s, 1H), 7.95 (t, 1H), 7.78 (d, 2H), 7.53-7.43 (m, 3H), 7.30 (d, 2H), 7.27 (d, 2H), 4.91 (s, 2H), 4.84 (s, 2H), 3.11 (d, 1H), 3.10-3.02 (m, 1H), 2.53-2.46 (m, 1H), 2.22 (m, 2H), 1.75-1.65 (m, 1H), 1.62-1.15 (m, 12H),0.99 (d, 3H), 0.95-0.84 (m,1H). |
| | aus (+)-6-{[2-Cyclopentyl-2-{4-[(5-oxo-2-phenyl-5,6-dihydro-4*H*-1,3,4-oxadiazin-4-yl)methyl]phenyl} - acetyl]amino}-2-methylhexansäureethylester (*Diastereomer 1*) | [α]_{D}²⁰ = +11 °, c = 0.405, Chloroform. |

| **Beispiel** | **Name / Struktur / Ausgangsmaterial** | **Analytische Daten** |
|---|---|---|
| **11** | (+)-6-{[2-Cyclopentyl-2-{4-[(5-oxo-2-phenyl-5,6-dihydro-4*H*-1,3,4-oxadiazin-4-yl)methyl]phenyl}-acetyl]amino}-2-methylhexansäure (*Diastereomer 2*) | LC-MS (Methode 2): Rₜ = 2.12 min; m/z = 520 (M+H)⁺. |
| | 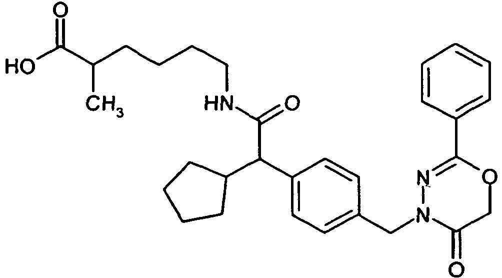 | ¹H-NMR (500 MHz, CDCl₃): δ = 7.85 (d, 2H), 7.48-7.23 (m, 7H), 5.50 (br. s, 1H), 4.94 (s, 2H), 4.79 (s, 2H), 3.28-3.12 (m, 2H), 2.99 (d, 1H), 2.63-2.52 (m, 1H), 2.43-2.33 (m, 1H), 2.00-1.91 (m, 1H), 1.68-1.31 (m, 8H), 1.30-1.05 (m, 6H), 1.04-0.93 (m,1H). |
| | aus (+)-6-{[2-Cyclopentyl-2-{4-[(5-oxo-2-phenyl-5,6-dihydro-4*H*-1,3,4-oxadiazin-4-yl)methyl]phenyl}-acetyl]amino}-2-methylhexansäureethylester (*Diastereomer 2*) | [α]_{D}²⁰ = +20°, c = 0.255, Chloroform. |

### Beispiel 12

### cis/trans-1-[(4-{[2-Cyclopentyl-2-{4-[(5-oxo-2-phenyl-5,6-dihydro-4H-1,3,4-oxadiazin-4-yl)-methyl]phenyl}acetyl]amino}but-1-en-1-yl]cyclopropancarbonsäure

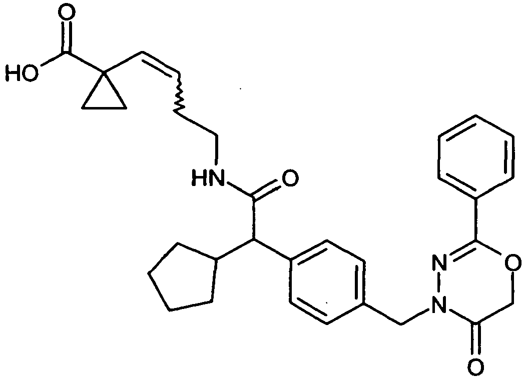

Zu einer Lösung von 120 mg (0.215 mmol) *cis*/*trans*-1-[4-{[(+)-2-Cyclopentyl-2-{4-[(5-oxo-2-phenyl-5,6-dihydro-4*H*-1,3,4-oxadiazin-4-yl)methyl]phenyl}acetyl]amino}but-1-en-1-yl]cyclopropancarbonsäureethylester in 0.23 ml THF und 0.56 ml Ethanol wurden 86 mg (2.15 mmol) Natriumhydroxid gegeben. Die Suspension wurde 20 min bei RT gerührt und dann mit 1 N Salzsäure schwach angesäuert. Es wurde dreimal mit Ethylacetat extrahiert. Die vereinigten organischen Phasen wurden mit gesättigter wässriger Ammoniumchlorid-Lösung gewaschen, über Magnesiumsulfat getrocknet und im Vakuum eingeengt. Es wurden 112 mg der Zielverbindung als *cis*/*trans*-Isomerengemisch isoliert.

Das erhaltene *cis*/*trans*-Gemisch wurde anschließend durch präparative HPLC aufgetrennt [Säule: Kromasil 100 C 18, 5 µm, 250 mm x 20 mm; Injektionsvolumen: 0.7 ml; Eluent: 40% 0.2%-ige Trifluoressigsäure / 60% Acetonitril; Fluss: 25 ml/min; Temperatur: 40°C; Detektion: 210 nm]. Ausgehend von 112 mg Stereoisomerengemisch wurden 66 mg des *cis*-Isomeren (siehe Beispiel 13) und 11 mg des *trans*-Isomeren (siehe Beispiel 14) erhalten.

### Beispiel 13

### cis-1-[(1Z)-4-{[2-Cyclopentyl-2-{4-[(5-oxo-2-phenyl-5,6-dihydro-4H-1,3,4-oxadiazin-4-yl)-methyl]phenyl}acetyl]amino}but-1-en-1-yl]cyclopropancarbonsäure

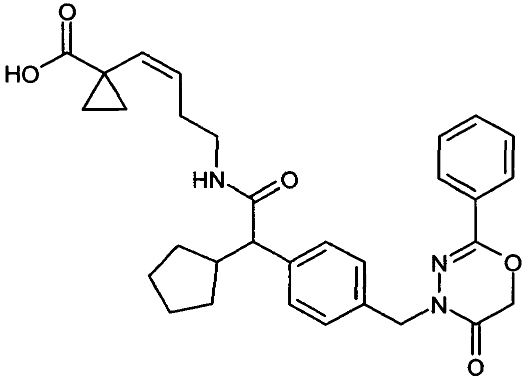

LC-MS (Methode 4): Rₜ = 2.52 min; m/z = 530 (M+H)⁺.
¹H-NMR (400 MHz, DMSO-d₆): δ = 7.98 (t, 1H), 7.77 (d, 2H), 7.46 (m, 3H), 7.28 (q, 4H), 5.50 (d, 1H), 5.40 (m, 1H), 4.90 (s, 2H), 4.82 (s, 2H), 3.09 (m, 2H), 2.90 (m, 1H), 2.47 (m, 1H), 2.15 (m, 2H), 1.69 (m, 1H), 1.62-1.34 (m, 4H), 1.28 (m, 1H), 1.22 (q, 2H), 1.16 (m, 1H), 0.88 (m, 1H), 0.77 (q, 2H).

### Beispiel 14

### trans-1-[(1E)-4-{[2-Cyclopentyl-2-{4-[(5-oxo-2-phenyl-5,6-dihydro-4H-1,3,4-oxadiazin-4-yl)-methyl]phenyl }acetyl]amino} but-1-en-1-yl]cyclopropancarbonsäure

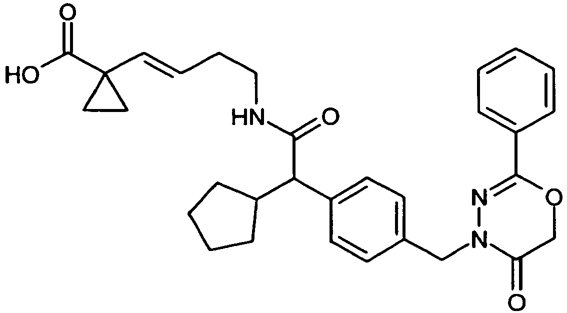

LC-MS (Methode 4): Rₜ = 2.50 min; m/z = 530 (M+H)⁺.
¹H-NMR (400 MHz, DMSO-d₆): δ = 7.91 (t, 1H), 7.77 (d, 2H), 7.46 (m, 3H), 7.28 (q, 4H), 5.99 (d, 1H), 5.17 (m, 1H), 4.90 (s, 2H), 4.83 (s, 2H), 3.07 (m, 2H), 2.87 (m, 1H), 2.45 (m, 1H), 2.03 (q, 2H), 1.69 (m, 1H), 1.62-1.26 (m, 5H), 1.20 (d, 2H), 1.18 (m, 1H), 0.88 (m, 1H), 0.85 (q, 2H).

### Beispiel 15

### (-)-trans-1-[(2E)-4-{[(2-Cyclopentyl-2-{4-[(5-oxo-2-phenyl-5,6-dihydro-4H-1,3,4-oxadiazin-4-yl)-methyl]phenyl}acetyl]amino}but-2-en-1-yl]cyclopropancarbonsäure

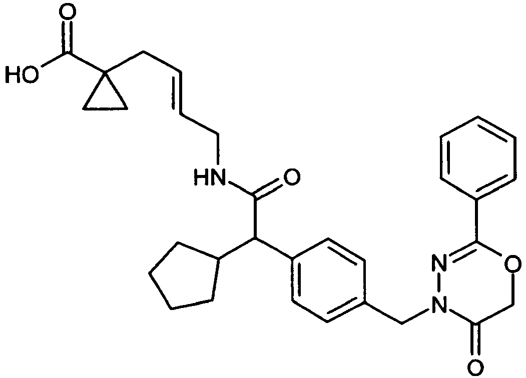

Zu einer Lösung von 61 mg (0.104 mmol) (-)-*trans*-1-[(2*E*)-4-{[2-Cyclopentyl-2-{4-[(5-oxo-2-phenyl-5,6-dihydro-4*H*-1,3,4-oxadiazin-4-yl)methyl]phenyl}acetyl]amino}but-2-en-1-yl]cyclopropancarbonsäure-*tert*.-butylester in 0.1 ml Dichlormethan wurden bei RT 0.12 ml Trifluoressigsäure getropft. Die Mischung wurde 1 h bei RT gerührt und danach nochmals 0.12 ml Trifluoressigsäure hinzugefügt. Nach weiteren 2 h bei RT wurde die Reaktionsmischung im Vakuum eingeengt und der Rückstand durch präparative RP-HPLC (Acetonitril/Wasser-Gradient) gereinigt. Es wurden 42 mg (76.2% d. Th.) der Zielverbindung erhalten.
LC-MS (Methode 3): Rₜ = 1.33 min; m/z = 530 (M+H)⁺.
¹H-NMR (400 MHz, DMSO-d₆): δ = 12.08 (br. s, 1H), 8.09 (t, 1H), 7.88 (d, 2H), 7.53-7.42 (m, 3H), 7.33-7.26 (m, 4H), 5.53-5.45 (m, 1H), 5.38-5.30 (m, 1H), 4.91 (s, 2H), 4.83 (s, 2H), 3.70-3.60 (m, 1H), 3.49-3.40 (m, 1H), 3.18 (m, 1H), 2.12 (d, 2H), 1.77-1.15 (m, 7H), 0.98 (m, 2H), 0.96-0.86 (m, 1 H), 0.62 (m, 2H).
[α]_{D}²⁰ = -1.1 °, c = 0.53, Chloroform.

### Beispiel 16

### (-)-cis-1-[(1Z)-4-{[2-Cyclopentyl-2-{4-[(6-oxo-3-phenylpyridazin-1(6H)-yl)methyl]phenyl}-acetyl]amino}but-1-en-1-yl]cyclopropancarbonsäure

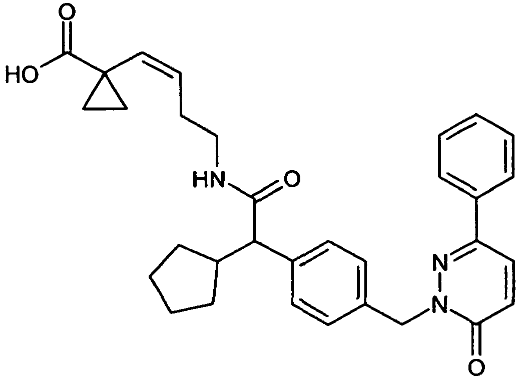

90 mg (0.163 mmol) *cis*-1-[(1*Z*)-4-{[2-Cyclopentyl-2-{4-[(6-oxo-3-phenylpyridazin-1(6*H*)-yl)-methyl]phenyl}acetyl]amino}but-1-en-1-yl]cyclopropancarbonsäureethylester wurden in einer Mischung aus 100 µl Wasser, 100 µl THF und 100 µl Methanol gelöst und bei 0°C mit 17.1 mg (0.406 mmol) Lithiumhydroxid versetzt. Die Mischung wurde auf RT erwärmt. Da keine Umsetzung erkennbar war, wurde das Reaktionsgemisch mit einem größeren Überschuss an Natriumhydroxid versetzt und über Nacht bei RT gerührt. Die Reaktionsmischung wurde dann auf Eiswasser gegeben und mit 1 N Salzsäure schwach angesäuert. Der ausgefallene Feststoff wurde abgesaugt, mehrmals mit Wasser gewaschen und im Hochvakuum getrocknet. Es wurden 78 mg (91.3% d. Th.) der Zielverbindung erhalten.
LC-MS (Methode 5): Rₜ = 1.13 min; m/z = 526 (M+H)⁺.
¹H-NMR (400 MHz, DMSO-d₆): δ = 12.09 (s, 1H), 8.09 (d, 1H), 7.98 (t, 1H), 7.89 (m, 2H), 7.52-7.44 (m, 3H), 7.33-7.26 (m, 4H), 7.08 (d, 1H), 5.52-5.48 (m, 1H), 5.43-5.35 (m, 1H), 5.29 (s, 2H), 3.12-3.05 (m, 2H), 2.90 (m, 1H), 2.49 (m, 1H), 2.20-2.10 (m, 2H), 1.74-1.23 (m, 6H), 1.21 (m, 2H), 1.20-1.12 (m, 1H), 0.91-0.84 (m, 1H), 0.78 (m, 2H).
[α]_{D}²⁰ = -22.9°, c = 0.520, Chloroform.

### Beispiel 17

### (-)-trans-1-[(1E)-4-{[2-Cyclopentyl-2-{4-[(6-oxo-3-phenylpyridazin-1(6H)-yl)methyl]phenyl}-acetyl]amino}but-1-en-1-yl]cyclopropancarbonsäure

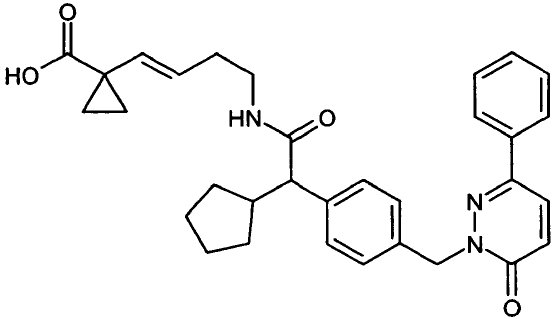

11.0 mg (0.020 mmol) *trans*-1-[(1*E*)-4-{[2-Cyclopentyl-2-{4-[(6-oxo-3-phenylpyridazin-1(6*H*)-yl)-methyl]phenyl}acetyl]amino}but-1-en-1-yl]cyclopropancarbonsäureethylester wurden in einer Mischung aus 50 µl Wasser, 50 µl THF und 50 µl Methanol gelöst und mit 8 mg (0.2 mmol) Natriumhydroxid versetzt. Die Reaktionsmischung wurde 3 h bei RT gerührt, bevor mit Wasser verdünnt und mit 1 N Salzsäure auf pH 2 eingestellt wurde. Die wässrige Phase wurde zweimal mit Ethylacetat extrahiert. Die vereinigten organischen Phasen wurden über Natriumsulfat getrocknet und im Vakuum eingeengt. Der Rückstand wurde in ca. 0.5 ml 1,4-Dioxan aufgenommen, bei -78°C eingefroren und im Hochvakuum lyophilisiert. Es wurden 9.6 mg (91.9% d. Th.) der Zielverbindung erhalten.
LC-MS (Methode 5): Rₜ = 1.12 min; m/z = 526 (M+H)⁺.
¹H-NMR (400 MHz, DMSO-d₆): δ = 12.18 (br. s, 1H), 8.07 (d, 1H), 7.95-7.88 (m, 3H), 7.53-7.44 (m, 3H), 7.34-7.27 (m, 4H), 7.09 (d, 1H), 5.99 (d, 1H), 5.29 (s, 2H), 5.18 (dt, 1H), 3.12-3.05 (m, 1H), 3.07 (d, 1H), 2.48 (m, 1H), 2.54-2.46 (m, 1H), 2.08-2.02 (m, 2H), 1.75-1.24 (m, 8H), 1.24-1.16 (m, 2H), 0.91-0.82 (m, 3H).
[α]_{D}²⁰ = -12.0°, c = 0.235, Chloroform.

### Beispiel 18

### (1-{3-[(Cyclopentyl{4-[(5-oxo-2-phenyl-5,6-dihydro-4H-1,3,4-oxadiazin-4-yl)methyl]phenyl}-acetyl)amino]propyl}cyclopropyl)essigsäure

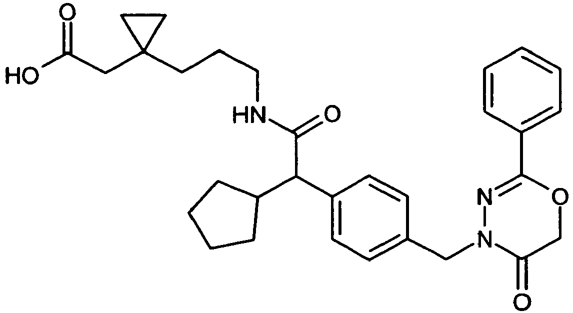

Eine Lösung von 230 mg (0.42 mmol) Methyl-(1-{3-[(cyclopentyl{4-[(5-oxo-2-phenyl-5,6-di-hydro-4*H*-1,3,4-oxadiazin-4-yl)methyl]phenyl}acetyl)amino]propyl}cyclopropyl)acetat in 2.8 ml THF und 1.4 ml Wasser wurde mit 71 mg (1.69 mmol) Lithiumhydroxid-Monohydrat versetzt und über Nacht bei RT gerührt. Nach vollständiger Umsetzung wurde das THF im Vakuum abgezogen, die Reaktionslösung mit Wasser verdünnt und anschließend mit 1 M Salzsäure auf pH 2 gestellt. Der ausgefallene Feststoff wurde abfiltriert, mit Wasser gewaschen und über Nacht im Vakuum bei 45°C getrocknet. Es wurden 217 mg (0.41 mmol, 97% d. Th.) der Titelverbindung als weißer Feststoff erhalten.
LC-MS (Methode 2): Rₜ = 2.14 min; m/z = 532 (M+H)⁺.
¹H-NMR (400 MHz, DMSO-d₆, δ/ppm): 12.20-11.80 (1H, br. s), 7.92 (1H, t), 7.77 (2H, d), 7.53-7.42 (3H, m), 7.28 (4H, q), 4.90 (2H, s), 4.83 (2H, s), 3.09 (1H, d), 3.06-2.97 (1H, m), 2.88-2.77 (1H, m), 2.56-2.44 (1H, m), 2.07 (2H, s), 1.75-1.65 (1H, m), 1.64-1.25 (7H, m), 1.23-1.10 (3H, m), 0.94-0.82 (1H, m), 0.34-0.25 (2H, m), 0.20-0.11 (2H, m).

### Beispiel 19

### 6-[(Cyclopentyl{4-[(5-oxo-2-phenyl-5,6-dihydro-4H-1,3,4-oxadiazin-4-yl)methyl]phenyl}acetyl)-amino]hexansäure

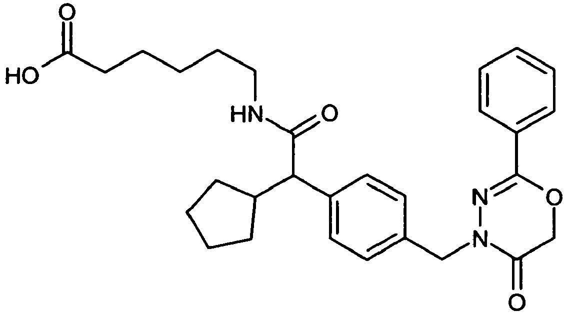

Eine Lösung von 160 mg (0.31 mmol) Methyl-6-[(cyclopentyl{4-[(5-oxo-2-phenyl-5,6-dihydro-4*H*-1,3,4-oxadiazin-4-yl)methyl]phenyl}acetyl)amino]hexanoat in 4 ml THF und 4 ml Wasser wurde mit 15 mg (0.62 mmol) Lithiumhydroxid-Monohydrat versetzt und über Nacht bei 60°C gerührt. Der Ansatz wurde danach mit 1M Salzsäure auf pH 4 gestellt und zweimal mit Essigsäureethylester extrahiert. Die vereinigten organischen Phasen wurden über Magnesiumsulfat getrocknet und anschließend im Vakuum bis zur Trockene eingeengt. Es wurden 120 mg (0.24 mmol, 77% d. Th.) der Titelverbindung als weißer Feststoff erhalten.
LC-MS (Methode 3): Rₜ = 1.26 min; m/z = 506 (M+H)⁺.
¹H-NMR (400 MHz, DMSO-d₆, δ/ppm): 11.96 (1H, s), 7.92 (1H, t), 7.77 (2H, d), 7.54-7.41 (3H, m), 7.28 (4H, q), 4.90 (2H, s), 4.83 (2H, s), 3.10 (1H, d), 3.07-2.99 (1H, m), 2.89-2.77 (1H, m), 2.52-2.41 (1H, m), 2.12 (2H, t), 1.76-1.65 (1H, m), 1.65-1.25 (9H, m), 1.25-1.10 (3H, m), 0.95-0.82 (1H, m).

### Beispiel 20

### 1-{4-[(Cyclopentyl{4-[(6-oxo-3-phenylpyridazin-1(6H)-yl)methyl]phenyl}acetyl)amino]butyl}-cyclopropancarbonsäure

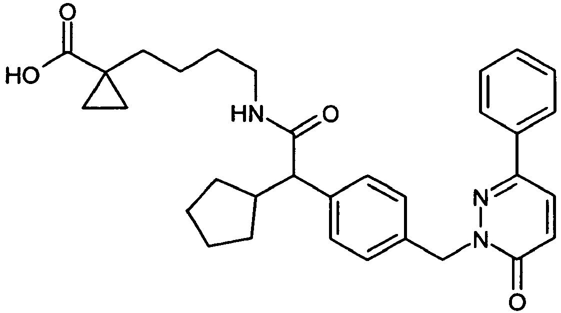

Zu einer Lösung von 197 mg (0.34 mmol) *tert*.-Butyl-1-{4-[(cyclopentyl{4-[(6-oxo-3-phenyl-pyridazin-1(6*H*-yl)methyl]phenyl}acetyl)amino]butyl}cyclopropancarboxylat in 10 ml Dichlormethan wurden 0.52 ml (6.74 mmol) Trifluoressigsäure getropft und die Mischung über Nacht bei RT gerührt. Anschließend wurde die Reaktionslösung im Vakuum bis zur Trockene eingeengt. Der Rückstand wurde über präparative RP-HPLC gereinigt. Es wurden 99 mg (0.19 mmol, 56% d. Th.) der Titelverbindung erhalten.
LC-MS (Methode 3): Rₜ = 1.31 min; m/z = 528 (M+H)⁺.
¹H-NMR (400 MHz, DMSO-d₆, δ/ppm): 12.10-11.75 (1H, br. s), 8.07 (1H, d), 7.95-7.85 (3H, m), 7.53-7.42 (3H, m), 7.28 (4H, q), 7.08 (1H, d), 5.29 (2H, s), 3.09 (1H, d), 3.07-2.97 (1H, m), 2.89-2.76 (1H, m), 2.52-2.39 (1H, m), 1.75-1.64 (1H, m), 1.64-1.24 (11H, m), 1.21-1.10 (1H, m), 0.99-0.93 (2H, m), 0.93-0.82 (1H, m), 0.62-0.50 (2H, m).

Auf analoge Weise wurden die in der folgenden Tabelle aufgeführten Verbindungen erhalten:

| **Beispiel** | **Name / Struktur / Edukt** | **Analytische Daten** |
|---|---|---|
| **21** | 4-[(Cyclopentyl{4-[(5-oxo-2-phenyl-5,6-dihydro-4*H-*1,3,4-oxadiazin-4-yl)methyl]phenyl}acetyl)amino]-butansäure | LC-MS (Methode 2): Rₜ = 1.94 min; m/z = 478 (M+H)⁺. |
| | 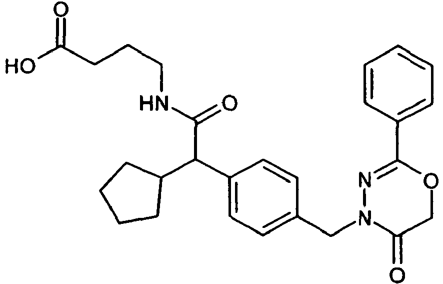 | |
| | (aus Methyl-4-[(cyclopentyl{4-[(5-oxo-2-phenyl-5,6-dihydro-4*H*-1,3,4-oxadiazin-4-yl)methyl]phenyl}-acetyl)amino]butanoat) | |
| **22** | 5-[(Cyclopentyl{4-[(5-oxo-2-phenyl-5,6-dihydro-4*H-*1,3,4-oxadiazin-4-yl)methyl]phenyl}acetyl)amino]-pentansäure | LC-MS (Methode 2): Rₜ = 1.98 min; m/z = 492 (M+H)⁺. |
| | 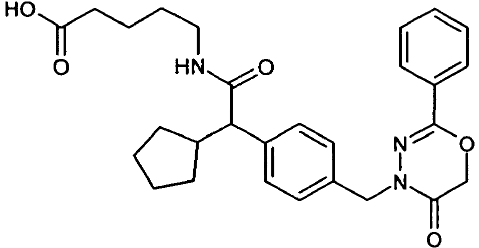 | ¹H-NMR (400 MHz, DMSO-d₆, δ/ppm): 11.97 (1H, s), 7.96 (1H, t), 7.77 (2H, d), 7.53-7.42 (3H, m), 7.28 (4H, q), 4.91 (2H, s), 4.83 (2H, s), 3.14-3.02 (2H, m), 2.88-2.77 (1H, m), 2.52-2.41 (1H, m), 2.16 (2H, t), 1.77-1.65 (1H, m), 1.65-1.25 (9H, m), 1.25-1.10 (1H, m), 0.95-0.82 (1H, m). |
| | (aus Methyl-5-[(cyclopentyl{4-[(5-oxo-2-phenyl-5,6-dihydro-4*H*-1,3,4-oxadiazin-4-yl)methyl]phenyl}-acetyl)amino]pentanoat) | |
| **23** | 7-[(Cyclopentyl{4-[(S-oxo-2-phenyl-5,6-dihydro-4*H-*1,3,4-oxadiazin-4-yl)methyl]phenyl}acetyl)amino]-heptansäure | LC-MS (Methode 2): Rₜ = 2.10 min; m/z = 520 (M+H)⁺. |
| | 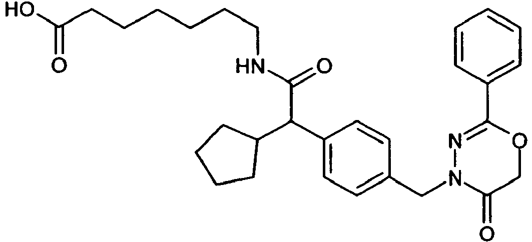 | |
| | (aus Methyl-7-[(cyclopentyl{4-[(5-oxo-2-phenyl-5,6-dihydro-4*H*-1,3,4-oxadiazin-4-yl)methyl]phenyl}-acetyl)amino]heptanoat) | |
| **24** | 1-{4-[(4,4,4-Trifluor-3-methyl-2-{4-[(5-oxo-2-phenyl-5,6-dihydro-4*H*-1,3,4-oxadiazin-4-yl)methyl]phenyl}-butanoyl)amino]butyl}cyclopropancarbonsäure | LC-MS (Methode 3): Rₜ = 1.31 min; m/z = 560 (M+H)⁺. |
| | 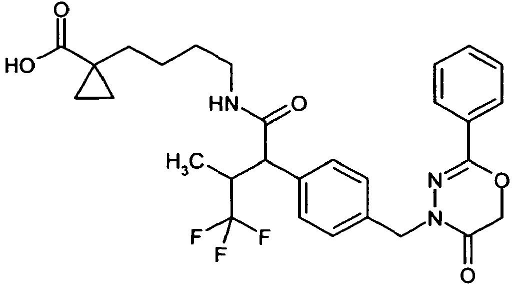 | ¹H-NMR (400 MHz, DMSO-d₆, δ/ppm): 11.97 (1H, br. s), 8.09 (1H, t), 7.77 (2H, d), 7.41-7.54 (3H, m), 7.31 (4H, s), 4.92 (2H, s), 4.87 (2H, s), 3.55 (1H, d), 3.17-3.30 (1H, m), 2.97-3.08 (1H, m), 2.75-2.87 (1H, m), 1.31-1.41 (2H, m), 1.20-1.31 (4H, m), 0.91-1.00 (2H, m), 0.71 (3H, d), 0.51-0.61 (2H, m). |
| | (aus *tert*.-Butyl-1-{4-[(4,4,4-trifluor-3-methyl-2-{4-[(5-oxo-2-phenyl-5,6-dihydro-4*H*-1,3,4-oxadiazin-4-yl)-methyl]phenyl}butanoyl)amino]butyl}cyclopropan-carboxylat) | |

### Beispiel 25

### (+/-)-6-[(5,5,5-Trifluor-2-{4-[(5-oxo-2-phenyl-5,6-dihydro-4H-1,3,4-oxadiazin-4-yl)methyl]-phenyl}pentanoyl)amino]hexansäure

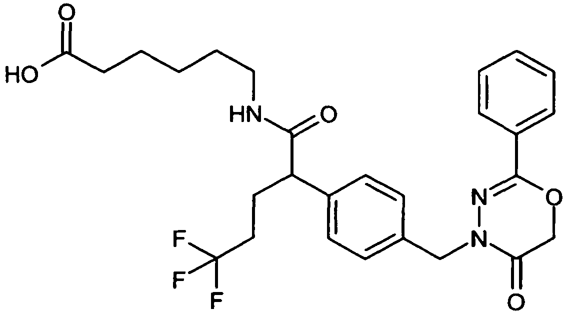

Eine Lösung von 109 mg (0.20 mmol) Methyl-6-[(5,5,5-trifluor-2-(4-[(5-oxo-2-phenyl-5,6-dihydro-4*H*-1,3,4-oxadiazin-4-yl)methyl]phenyl)pentanoyl)amino]hexanoat in 2 ml THF und 1 ml Wasser wurde mit 33 mg (0.80 mmol) Lithiumhydroxid-Monohydrat versetzt und über Nacht bei RT gerührt. Der Ansatz wurde danach mit 1 M Salzsäure auf pH 2 gestellt und zweimal mit Essigsäureethylester extrahiert. Die vereinigten organischen Phasen wurden über Magnesiumsulfat getrocknet und anschließend im Vakuum bis zur Trockene eingeengt. Das Rohprodukt wurde über präparative RP-HPLC gereinigt. Es wurden 59 mg (0.11 mmol, 56% d. Th.) der Titelverbindung als farbloses Öl erhalten.
LC-MS (Methode 2): Rₜ = 2.00 min; m/z = 534 (M+H)⁺.
¹H-NMR (400 MHz, DMSO-d₆, δ/ppm): 12.20-11.75 (1H, br. s), 8.04 (1H, t), 7.77 (2H, d), 7.53-7.41 (3H, m), 7.34-7.25 (4H, m), 4.91 (2H, s), 4.85 (2H, s), 3.48 (1H, t), 3.09-2.98 (1H, m), 2.97-2.85 (1H, m), 2.18-2.00 (5H, m), 1.84-1.71 (1H, m), 1.47-1.36 (2H, m), 1.36-1.27 (2H, m), 1.21-1.10 (2H, m).

### Beispiel 26 und Beispiel 27

### 6-[(5,5,5-Trifluor-2-{4-[(5-oxo-2-phenyl-5,6-dihydro-4H-1,3,4-oxadiazin-4-yl)methyl]phenyl}-pentanoyl)amino]hexansäure (Enantiomer 1 und 2)

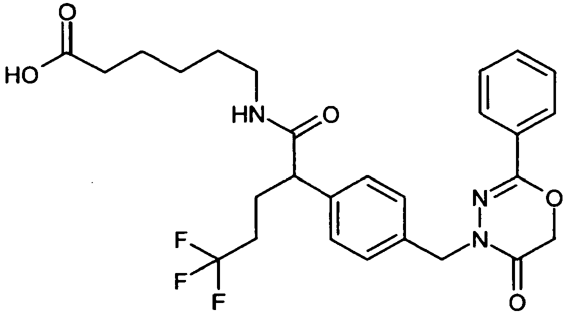

52 mg (0.097 mmol) der oben erhaltenen racemischen (+/-)-6-[(5,5,5-Trifluor-2-{4-[(5-oxo-2-phenyl-5,6-dihydro-4*H*-1,3,4-oxadiazin-4-yl)methyl]phenyl}pentanoyl)amino]hexansäure wurden mittels präparativer HPLC an chiraler Phase weiter aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 75:25 (v/v); Fluss: 15 ml/min; UV-Detektion: 220 nm; Temperatur: 30°C]:

### Beispiel 26 (Enantiomer 1):

Ausbeute: 10 mg
Rₜ 6.78 min; Reinheit >99%; >99% ee
   [Säule: Daicel Chiralpak AD-H, 5 µm, 250 mm x 4.6 mm; Elutionsmittel: Isohexan/(lsopropanol + 0.2% Trifluoressigsäure + 1% Wasser) 75:25 (v/v); Fluss: 2 ml/min; UV-Detektion: 220 nm; Temperatur: 25°C]
¹H-NMR (400 MHz, DMSO-d₆, δ/ppm): 12.20-11.75 (1H, br. s), 8.04 (1H, t), 7.77 (2H, d), 7.53-7.41 (3H, m), 7.34-7.25 (4H, m), 4.91 (2H, s), 4.85 (2H, s), 3.48 (1H, t), 3.09-2.98 (1H, m), 2.97-2.85 (1H, m), 2.18-2.00 (5H, m), 1.84-1.71 (1H, m), 1.47-1.36 (2H, m), 1.36-1.27 (2H, m), 1.21-1.10(2H,m).

### Beispiel 27 (Enantiomer 2):

Ausbeute: 26 mg
Rₜ 7.41 min; Reinheit >98%; >99% ee (analytische Säule s.o.)
LC-MS (Methode 6): Rₜ = 2.28 min; m/z = 534 (M+H)⁺.

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro:

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCI 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 1 aufgeführt:

**Tabelle 1: Gefäßrelaxierende Wirkung in vitro**

| **Beispiel Nr.** | **IC₅₀ [nM]** |
|---|---|
| 1 | 284 |
| 5 | 297 |
| 20 | 70 |

### B-2. Stimulation der rekombinanten löslichen Guanylatcyclase (SGC) in vitro:

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazolo[4,3-a]chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als n-fache Stimulation der Basalaktivität angegeben. Das Ergebnis für Beispiel 1 ist in Tabelle 2 gezeigt:

**Tabelle 2: Stimulation (n-fach) der rekombinanten löslichen Guanylatcyclase (sGC) in vitro durch Beispiel 1**

| **Konzentration Beispiel 1 [µM]** | **Häm-haltige sGC** | | | **Häm-freie sGC Basal** |
|---|---|---|---|---|
| | **Basal** | **+ 0.1 µM DEA/NO** | **+ 10 µM ODQ** | |
| 0 | 1.0 | 22.2 | 3.0 | 1.0 |
| 0.1 | 1.5 | 21.9 | 3.0 | 1.4 |
| 1 | 1.4 | 19.3 | 3.7 | 1.7 |
| 10 | 2.6 | 20.0 | 10.3 | 8.4 |
| 100 | 14.0 | 37.8 | 46.6 | 111.8 |

| | | | | |
|---|---|---|---|---|
| [DEA/NO = 2-(*N,N-*Diethylamino)diazenolat-2-oxid; ODQ = 1*H*-1,2,4-Oxadiazolo[4,3-a]chinoxa-lin-1-on]. | | | | |

Aus Tabelle 2 ist ersichtlich, dass eine Stimulation sowohl des Häm-haltigen als auch des Hämfreien Enzyms erreicht wird. Weiterhin zeigt die Kombination aus Beispiel 1 und 2-(*N,N*-Diethyl-amino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch den Häm-abhängigen Inhibitor der löslichen Guanylatcyclase ODQ nicht blockiert, sondern sogar gesteigert. Die Ergebnisse aus Tabelle 2 belegen somit den Wirkmechanismus der erfindungsgemäßen Verbindungen als Aktivatoren der löslichen Guanylatcyclase.

### B-3. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Ergebnisse zu den erfindungsgemäßen Verbindungen sind in Tabelle 3 aufgeführt:

**Tabelle 3: sGC-aktivierende Wirkung in der CHO-Reporterzelle in vitro**

| **Beispiel Nr.** | **MEC [nM]** |
|---|---|
| 1 | 10 |
| 5 | 6.5 |
| 10 | 100 |
| 11 | 100 |
| is | 20 |
| 18 | 30 |
| 19 | 100 |
| 20 | 3 |
| 24 | 1 |

| | |
|---|---|
| (MEC = minimale effektive Konzentration). | |

### B-4. Stimulation der sGC-Enzymaktivität

Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzym-aktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77,14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus pyralis*-Luciferase, Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1% BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM Mach, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-a]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

Repräsentative Ergebnisse zu den erfindungsgemäßen Verbindungen sind in Tabelle 4 aufgeführt:

**Tabelle 4: Aktivierende Wirkung am sGC-Enzym in vitro**

| **Beispiel Nr.** | **MEC [nM]** | **EC₅₀ [nM]** |
|---|---|---|
| 1 | 51 | n.d. |
| 5 | 58 | 1500 |
| 20 | 13 | 650 |
| 24 | 5.5 | 184 |

| | | |
|---|---|---|
| (MEC = minimale effektive Konzentration; EC₅₀ = Konzentration bei 50% der maximalen Wirksamkeit; n.d. = nicht bestimmt). | | |

### B-5. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (1) Implantierbare Sender, (2) Empfänger, die über einen Multiplexer mit einem (3) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer AG, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (1) systolischer Blutdruck (SBP), (2) diastolischer Blutdruck (DBP), (3) arterieller Mitteldruck (MAP) und (4) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### C. Ausführungsbeispiele für pharmazeutische Zusammensetzunaen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 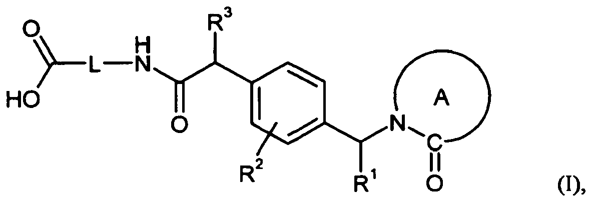 in welcher
der Ring A für einen N-verknüpften 5- bis 7-gliedrigen, gesättigten oder partiell ungesättigten, oxo-substituierten Azaheterocyclus steht,
der (*i*) ein oder zwei weitere Heteroatome aus der Reihe N, O und/oder S als Ringglieder enthalten kann,
der (*ii*) mit einem Rest ausgewählt aus der Reihe Fluor, Chlor, (C₁-C₆)-Alkyl, Trifluormethyl, (C₃-C₇)-Cy_{C}loalkyl, 4- bis 7-gliedriges Heterocyclyl und Phenyl substituiert ist oder der benzo-anelliert ist,
wobei der Phenyl-Substituent und der anellierte Phenylring ihrerseits bis zu zweifach, gleich oder verschieden, mit einem Rest ausgewählt aus der Reihe Halogen, Cyano, (C₁-C₄)-Alkyl, (C₂-C₄)-Alkenyl, Trifluormethyl, (C₁-C₄)-Alkoxy und Trifluormethoxy substituiert sein können,
und
der (*iii*) darüber hinaus bis zu zweifach, gleich oder verschieden, mit weiteren Resten ausgewählt aus der Reihe Fluor, Chlor, (C₁-C₆)-Alkyl, Trifluormethyl, Oxo, (C₃-C₇)-Cy_{C}loalkyl, 4- bis 7-gliedriges Heterocyclyl und Phenyl substituiert sein kann,
wobei Phenyl seinerseits bis zu zweifach, gleich oder verschieden, mit einem Rest ausgewählt aus der Reihe Halogen, Cyano, (C₁-C₄)-Alkyl, (C₂-C₄)-Alkenyl, Trifluormethyl, (C₁-C₄)-Alkoxy und Trifluormethoxy substituiert sein kann,
R¹ für Wasserstoff, (C₁-C₄)-Alkyl oder Cyclopropyl steht,
R² für Wasserstoff, Halogen, Cyano, (C₁-C₄)-Alkyl oder Trifluormethyl steht,
R³ für (C₃-C₆)-Alkyl oder (C₃-C₆)-Alkenyl, welche jeweils mit Cyano, (C₁-C₄)-Alkoxy oder Trifluormethoxy und bis zu sechsfach mit Fluor substituiert sein können,
oder
für (C₃-C₇)-Cycloalkyl oder (C₃-C₇)-Cycloalkenyl, welche jeweils bis zu zweifach, gleich oder verschieden, mit (C₁-C₄)-Alkyl, Trifluormethyl und (C₁-C₄)-Alkoxy sowie bis zu vierfach mit Fluor substituiert sein können,
oder
für Oxetanyl, Tetrahydrofuranyl oder Tetrahydropyranyl steht,
und
L für geradkettiges (C₃-C₇)-Alkandiyl oder (C₃-C₇)-Alkendiyl steht, welche jeweils bis zu vierfach, gleich oder verschieden, mit einem Rest R⁴ substituiert sein können, worin
R⁴ Fluor, Trifluormethyl oder (C₁-C₄)-Alkyl bedeutet
oder
zwei an dasselbe Kohlenstoffatom gebundene Reste R⁴ miteinander verknüpft sind und zusammen mit diesem Kohlenstoffatom einen (C₃-C₆)-Cycloalkan-1,1-diyl-Ring bilden,
sowie ihre Salze, Solvate und Solvate der Salze.

2. Verbindung der Formel (I) nach Anspruch 1, in welcher
der Ring A für einen oxo-substituierten Azaheterocyclus der Formel 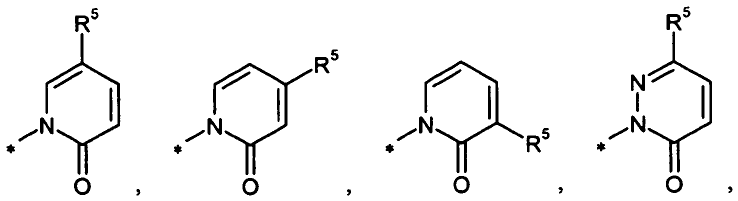 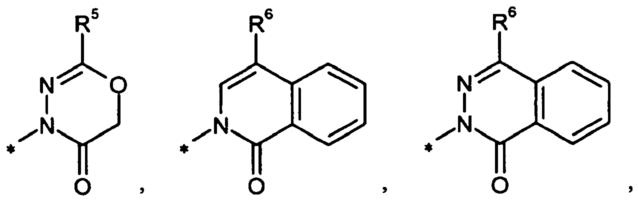 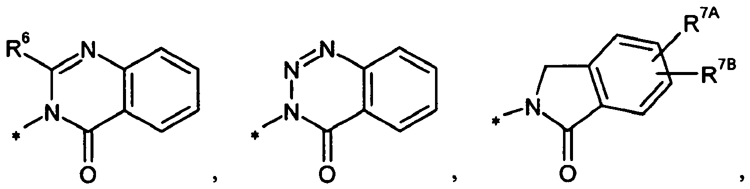 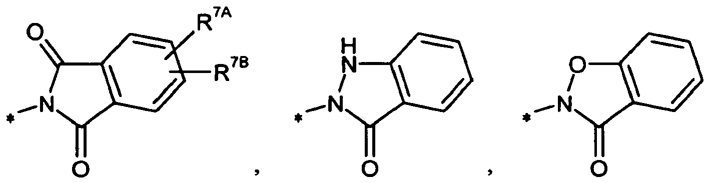 oder 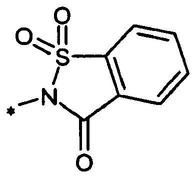 steht, worin
* die Verknüpfungsposition mit dem restlichen Teil des Moleküls kennzeichnet,
R⁵ Chlor, (C₁-C₆)-Alkyl, Trifluormethyl, (C₃-C₆)-Cycloalkyl, 4- bis 6-gliedriges Heterocyclyl oder Phenyl bedeutet, wobei Phenyl seinerseits bis zu zweifach, gleich oder verschieden, mit einem Rest ausgewählt aus der Reihe Fluor, Chlor, Brom, Cyano, (C₁-C₄)-Alkyl, Vinyl, Trifluormethyl, (C₁-C₄)-Alkoxy und Trifluormethoxy substituiert sein kann,
R⁶ Wasserstoff bedeutet oder die oben genannte Bedeutung von R⁵ hat
und
R^{7A} und R^{7B} unabhängig voneinander Wasserstoff, Fluor oder Chlor bedeuten,
sowie ihre Salze, Solvate und Solvate der Salze.

3. Verbindung der Formel (I) nach Anspruch 1 oder 2, in welcher
der Ring A für einen oxo-substituierten Azaheterocyclus der Formel 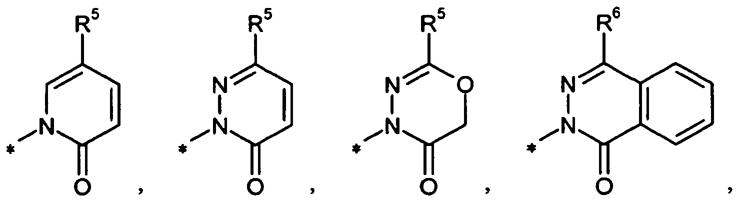 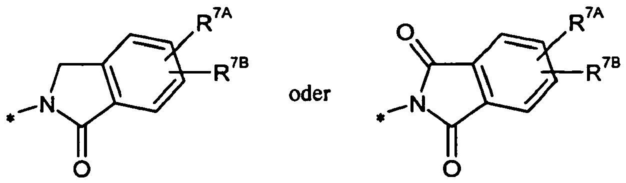 steht, worin
* die Verknüpfungsposition mit dem restlichen Teil des Moleküls kennzeichnet,
R⁵ Chlor, (C₁-C₆)-Alkyl, Trifluormethyl, (C₃-C₆)-Cycloalkyl oder Phenyl bedeutet, wobei Phenyl seinerseits bis zu zweifach, gleich oder verschieden, mit einem Rest ausgewählt aus der Reihe Fluor, Chlor, Cyano, (C₁-C₄)-Alkyl, Trifluormethyl, (C₁-C₄)-Alkoxy und Trifluormethoxy substituiert sein kann,
R⁶ Wasserstoff bedeutet oder die zuvor genannte Bedeutung von R⁵ hat
und
R^{7A} und R^{7B} unabhängig voneinander Wasserstoff, Fluor oder Chlor bedeuten,
R¹ für Wasserstoff oder (C₁-C₄)-Alkyl steht,
R² für Wasserstoff, Fluor, Chlor oder Trifluormethyl steht,
R³ für (C₃-C₆)-Alkyl oder (C₃-C₆)-Alkenyl, welche jeweils mit Cyano, Methoxy, Ethoxy oder Trifluormethoxy und bis zu sechsfach mit Fluor substituiert sein können,
oder
für (C₃-C₆)-Cycloalkyl oder (C₄-C₆)-Cycloalkenyl, welche jeweils bis zu zweifach, gleich oder verschieden, mit Methyl, Ethyl und Trifluormethyl sowie bis zu vierfach mit Fluor substituiert sein können,
oder
für Oxetanyl steht,
und
L für geradkettiges (C₃-C₆)-Alkandiyl oder (C₃-C₆)-Alkendiyl steht, welche jeweils bis zu vierfach, gleich oder verschieden, mit einem Rest R⁴ substituiert sein können, worin
R⁴ Fluor, Trifluormethyl, Methyl oder Ethyl bedeutet
oder
zwei an dasselbe Kohlenstoffatom gebundene Reste R⁴ miteinander verknüpft sind und zusammen mit diesem Kohlenstoffatom einen Cyclopropan-1,1-diyl- oder Cyclobutan-1,1-diyl-Ring bilden,
sowie ihre Salze, Solvate und Solvate der Salze.

4. Verbindung der Formel (I) nach Anspruch 1, 2 oder 3, in welcher
der Ring A für einen oxo-substituierten Azaheterocyclus der Formel 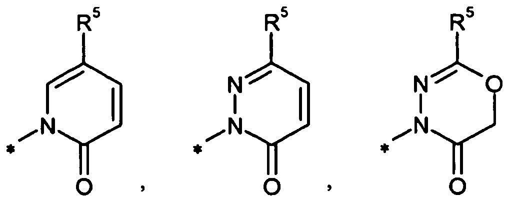 oder 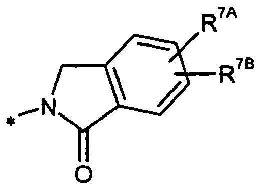 steht, worin
* die Verknüpfungsposition mit dem restlichen Teil des Moleküls kennzeichnet,
R⁵ Chlor, Trifluormethyl oder Phenyl bedeutet, wobei Phenyl seinerseits bis zu zweifach, gleich oder verschieden, mit einem Rest ausgewählt aus der Reihe Fluor, Chlor, Methyl und Trifluormethyl substituiert sein kann,
und
R^{7A} und R^{7B} unabhängig voneinander Wasserstoff oder Fluor bedeuten,
R¹ für Wasserstoff steht,
R² für Wasserstoff steht,
R³ für Propan-2-yl, Butan-2-yl, Pentan-2-yl, 3,3,3-Trifluorpropan-1-yl, 1,1,1-Trifluorpropan-2-yl, 1,1,1-Trifluorbutan-2-yl, 4,4,4-Trifluorbutan-2-yl, 4,4,4-Trifluor-2-methylbutan-1-yl, Cyclopentyl oder 3,3-Difluorcyclopentyl steht,
und
L für geradkettiges (C₃-C₆)-Alkandiyl oder (C₃-C₆)-Alkendiyl steht, welche jeweils bis zu vierfach, gleich oder verschieden, mit einem Rest R⁴ substituiert sein können, worin
R⁴ Methyl darstellt
oder
zwei an dasselbe Kohlenstoffatom gebundene Reste R⁴ miteinander verknüpft sind und zusammen mit diesem Kohlenstoffatom einen Cyclopropan-1,1-diyl-Ring bilden,
sowie ihre Salze, Solvate und Solvate der Salze.

5. Verfahren zur Herstellung einer Verbindung der Formel (I), wie in den Ansprüchen 1 bis 4 definiert, **dadurch gekennzeichnet, dass** man zunächst entweder
[A] eine Verbindung der Formel (II) 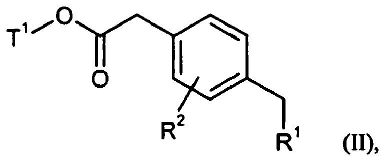 in welcher R¹ und R² die in den Ansprüchen 1 bis 4 angegebenen Bedeutungen haben
und
T¹ für (C₁-C₄)-Alkyl steht,
in einem inerten Lösungsmittel in Gegenwart einer Base mit einer Verbindung der Formel (III) 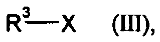 in welcher R³ die in den Ansprüchen 1 bis 4 angegebene Bedeutung hat und
X für eine Abgangsgruppe wie beispielsweise Halogen, Mesylat, Tosylat oder Triflat steht,
in eine Verbindung der Formel (IV) 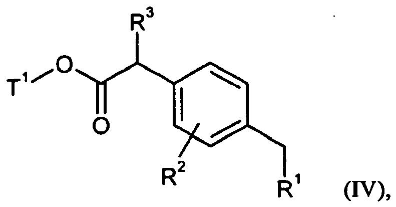 in welcher R¹, R², R³ und T¹ jeweils die oben angegebenen Bedeutungen haben, überführt
oder
[B] eine Verbindung der Formel (V) 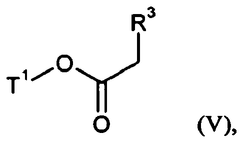 in welcher R³ die in den Ansprüchen 1 bis 4 angegebene Bedeutung hat
und
T¹ für (C₁-C₄)-Alkyl steht,
in einem inerten Lösungsmittel, nach Deprotonierung mittels einer Base, mit einer Verbindung der Formel (VI) 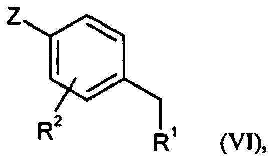 in welcher R¹ und R² die in den Ansprüchen 1 bis 4 angegebenen Bedeutungen haben
und
Z für Chlor, Brom oder Iod steht,
in Gegenwart eines geeigneten Palladium-Katalysators gleichfalls zu einer Verbindung der Formel (IV) 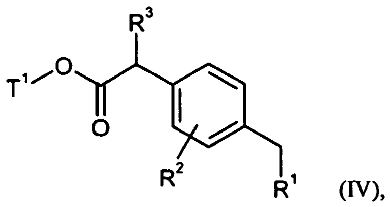 in welcher R¹, R², R³ und T' jeweils die oben angegebenen Bedeutungen haben, umsetzt,
die Verbindung der Formel (IV) dann in einem inerten Lösungsmittel mit elementarem Brom oder mit N-Bromsuccinimid zu einer Verbindung der Formel (VII) 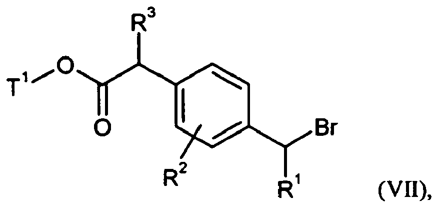 in welcher R¹, R², R³ und T¹ jeweils die oben angegebenen Bedeutungen haben,
bromiert, anschließend in einem inerten Lösungsmittel in Gegenwart einer Base mit einer Verbindung der Formel (VIII) 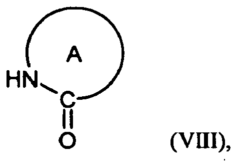 in welcher der Ring A für einen oxo-substituierten Azaheterocyclus, wie in den Ansprüchen 1 bis 4 definiert, steht,
zu einer Verbindung der Formel (IX) 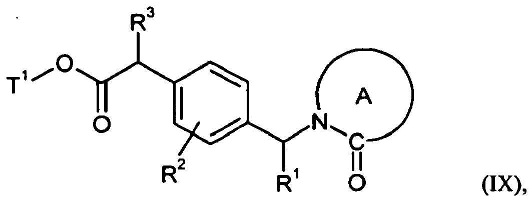 in welcher der Ring A, R¹, R², R³ und T¹ jeweils die oben angegebenen Bedeutungen haben,
umsetzt, nachfolgend den Ester-Rest T¹ in (IX) unter basischen oder sauren Bedingungen abspaltet, die resultierende Carbonsäure der Formel (X) 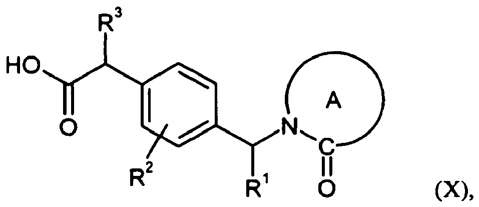 in welcher der Ring A, R¹, R² und R³ jeweils die oben angegebenen Bedeutungen haben, dann in einem inerten Lösungsmittel in Gegenwart eines Kondensationsmittels oder über die Zwischenstufe des entsprechenden Carbonsäurechlorids in Gegenwart einer Base mit einem Amin der Formel (XI) 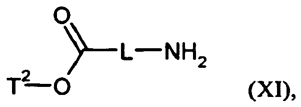 in welcher L die in den Ansprüchen 1 bis 4 angegebene Bedeutung hat und
T² für (C₁-C₄)-Alkyl steht,
zu einer Verbindung der Formel (XII) 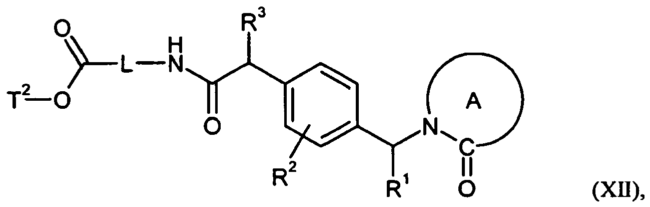 in welcher der Ring A, R¹, R², R³, L und T² jeweils die oben angegebenen Bedeutungen haben,
kuppelt und abschließend den Ester-Rest T² in (XII) durch erneute basische oder saure Solvolyse zur Carbonsäure der Formel (I) abspaltet
und die Verbindungen der Formel (I) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

6. Verbindung, wie in einem der Ansprüche 1 bis 4 definiert, zur Verwendung in der Behandlung und/oder Prävention von Krankheiten.

7. Verbindung, wie in einem der Ansprüche 1 bis 4 definiert, zur Verwendung in einem Verfahren zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

8. Verwendung einer Verbindung, wie in einem der Ansprüche 1 bis 4 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

9. Arzneimittel enthaltend eine Verbindung, wie in einem der Ansprüche 1 bis 4 definiert, in Kombination mit einem oder mehreren inerten, nicht-toxischen, pharmazeutisch geeigneten Hilfsstoffen.

10. Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

11. Arzneimittel nach Anspruch 9 oder 10 zur Verwendung in der Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

## Claims

1. Compound of the formula (I) 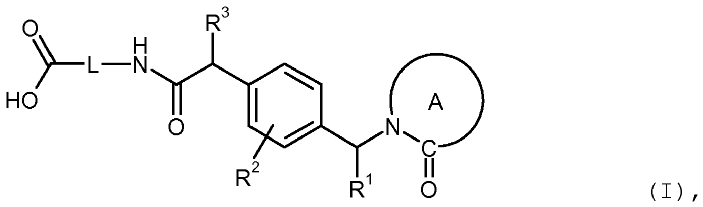 in which
ring A represents a 5- to 7-membered saturated or partially unsaturated oxo-substituted azaheterocycle attached via nitrogen,
which (*i*) may contain one or two further heteroatoms from the group consisting of N, O and S as ring members,
which (*ii*) is substituted by a radical selected from the group consisting of fluorine, chlorine, (C₁-C₆) -alkyl, trifluoromethyl, (C₃-C₇)-cycloalkyl, 4- to 7-membered heterocyclyl and phenyl or is benzofused,
where the phenyl substituent and the fused phenyl ring for their part may be substituted up to two times by identical or different radicals selected from the group consisting of halogen, cyano, (C₁-C₄) -alkyl, (C₂-C₄) -alkenyl, trifluoromethyl, (C₁-C₄) -alkoxy and trifluoromethoxy,
and
which (*iii*) may additionally be substituted up to two times by identical or different further radicals selected from the group consisting of fluorine, chlorine, (C₁-C₆) - alkyl, trifluoromethyl, oxo, (C₃-C₇) -cyclo-alkyl, 4- to 7-membered heterocyclyl and phenyl,
where phenyl for its part may be substituted up to two times by identical or different radicals selected from the group consisting of halogen, cyano, (C₁-C₄) -alkyl, (C₂-C₄) -alkenyl, trifluoromethyl, (C₁-C₄) -alkoxy and trifluoromethoxy,
R¹ represents hydrogen, (C₁-C₄) -alkyl or cyclopropyl,
R² represents hydrogen, halogen, cyano, (C₁-C₄) - alkyl or trifluoromethyl,
R³ represents (C₃-C₆) -alkyl or (C₃-C₆) -alkenyl, each of which may be substituted by cyano, (C₁-C₄) -alkoxy or trifluoromethoxy and up to six times by fluorine,
or
represents (C₃-C₇)-cycloalkyl or (C₃-C₇) - cycloalkenyl, each of which may be substituted up to two times by identical or different radicals from the group consisting of (C₁-C₄)-alkyl, trifluoromethyl and (C₁-C₄) - alkoxy and also up to four times by fluorine,
or
represents oxetanyl, tetrahydrofuranyl or tetrahydropyranyl,
and
L represents straight-chain (C₃-C₇)-alkanediyl or (C₃-C₇)-alkenediyl, each of which may be substituted up to four times by identical or different radicals R⁴ where
R⁴ represents fluorine, trifluoromethyl or (C₁-C₄)-alkyl
or
two radicals R⁴ attached to the same carbon atom are linked to each other and together with this carbon atom form a (C₃-C₆)-cycloalkane-1,1-diyl ring,
and the salts, solvates and solvates of the salts thereof.

2. Compound of the formula (I) according to Claim 1 in which
ring A represents an oxo-substituted azaheterocycle of the formula 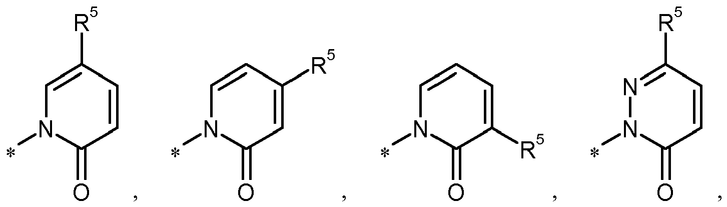 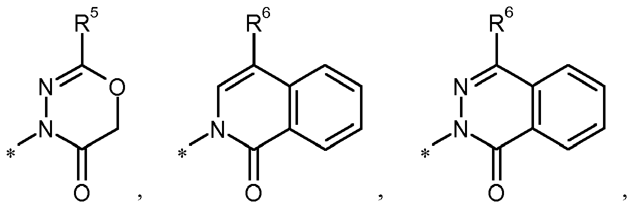 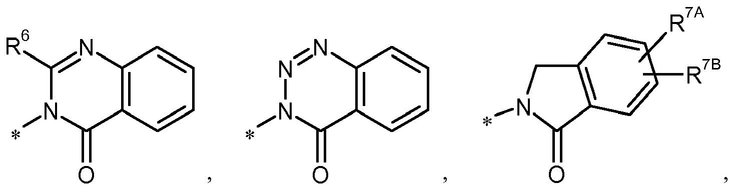 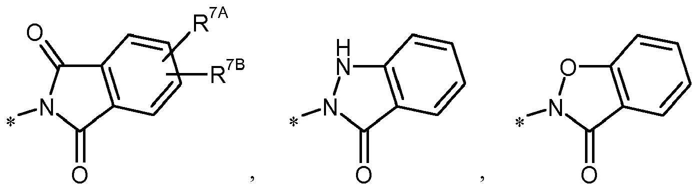 or 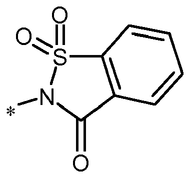 in which
* denotes the point of attachment to the remainder of the molecule,
R⁵ represents chlorine, (C₁-C₆) -alkyl, trifluoromethyl, (C₃-C₆)-cycloalkyl, 4-to 6-membered heterocyclyl or phenyl, where phenyl for its part may be substituted up to two times by identical or different radicals selected from the group consisting of fluorine, chlorine, bromine, cyano, (C₁-C₄)-alkyl, vinyl, trifluoromethyl, (C₁-C₄)-alkoxy and trifluoromethoxy,
R⁶ represents hydrogen or has the meaning of R⁵ given above
and
R^{7A} and R^{7B} independently of one another represent hydrogen, fluorine or chlorine,
and the salts, solvates and solvates of the salts thereof.

3. Compound of the formula (I) according to Claim 1 or 2 in which
ring A represents an oxo-substituted azaheterocycle of the formula 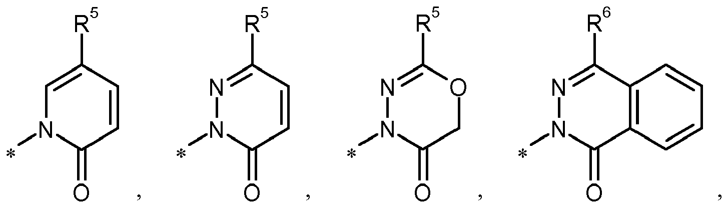 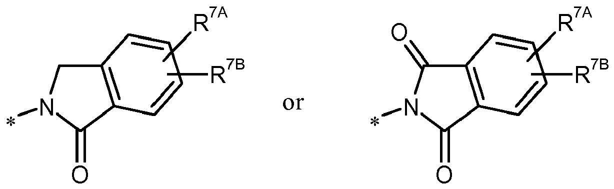 in which
* denotes the point of attachment to the remainder of the molecule,
R⁵ represents chlorine, (C₁-C₆)-alkyl, trifluoromethyl, (C₃-C₆) -cycloalkyl or phenyl, where phenyl for its part may be substituted up to two times by identical or different radicals selected from the group consisting of fluorine, chlorine, cyano, (C₁-C₄)-alkyl, trifluoromethyl, (C₁-C₄)-alkoxy and trifluoromethoxy,
R⁶ represents hydrogen or has the meaning of R⁵ given above
and
R^{7A} and R^{7B} independently of one another represent hydrogen, fluorine or chlorine,
R¹ represents hydrogen or (C₁-C₄)-alkyl,
R² represents hydrogen, fluorine, chlorine or trifluoromethyl,
R³ represents (C₃-C₆) -alkyl or (C₃-C₆) -alkenyl, each of which may be substituted by cyano, methoxy, ethoxy or trifluoromethoxy and up to six times by fluorine,
or
represents (C₃-C₆)-cycloalkyl or (C₄-C₆)-cycloalkenyl, each of which may be substituted up to two times by identical or different radicals from the group consisting of methyl, ethyl and trifluoromethyl and also up to four times by fluorine,
or
represents oxetanyl,
and
L represents straight-chain (C₃-C₆)-alkanediyl or (C₃-C₆)-alkenediyl, each of which may be substituted up to four times by identical or different radicals R⁴ where
R⁴ represents fluorine, trifluoromethyl, methyl or ethyl
or
two radicals R⁴ attached to the same carbon atom are linked to each other and together with this carbon atom form a cyclopropane-1,1-diyl or cyclobutane-1,1-diyl ring,
and the salts, solvates and solvates of the salts thereof.

4. Compound of the formula (I) according to any of Claims 1 to 3 in which
ring A represents an oxo-substituted azaheterocycle of the formula 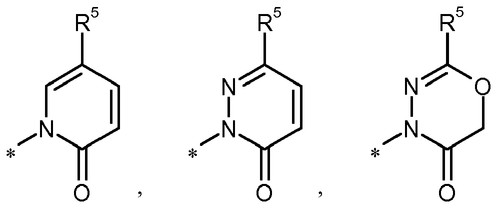 or 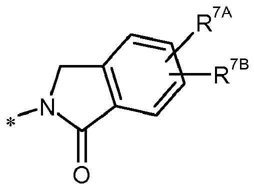 in which
* denotes the point of attachment to the remainder of the molecule,
R⁵ represents chlorine, trifluoromethyl or phenyl, where phenyl for its part may be substituted up to two times by identical or different radicals selected from the group consisting of fluorine, chlorine, methyl and trifluoromethyl,
and
R^{7A} and R^{7B} independently of one another represent hydrogen or fluorine,
R¹ represents hydrogen,
R² represents hydrogen,
R³ represents propan-2-yl, butan-2-yl, pentan-2-yl, 3,3,3-trifluoropropan-1-yl, 1,1,1-trifluoropropan-2-yl, 1,1,1-trifluorobutan-2-yl, 4,4,4-trifluorobutan-2-yl, 4,4,4-trifluoro-2-methylbutan-1-yl, cyclopentyl or 3,3-difluorocyclopentyl,
and
L represents straight-chain (C₃-C₆)-alkanediyl or (C₃-C₆)-alkenediyl, each of which may be substituted up to four times by identical or different radicals R⁴ where
R⁴ represents methyl
or
two radicals R⁴ attached to the same carbon atom are linked to each other and together with this carbon atom form a cyclopropane-1,1-diyl ring,
and the salts, solvates and solvates of the salts thereof.

5. Process for preparing a compound of the formula (I) as defined in any of Claims 1 to 4, **characterized in that** initially either
[A] a compound of the formula (II) 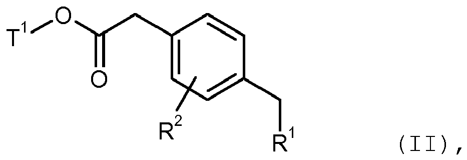 in which R¹ and R² have the meanings given in any of Claims 1 to 4
and
T¹ represents (C₁-C₄)-alkyl,
is converted in an inert solvent in the presence of a base with a compound of the formula (III) 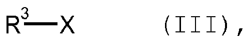 in which R³ has the meaning given in any of Claims 1 to 4
and
X represents a leaving group such as, for example, halogen, mesylate, tosylate or triflate,
into a compound of the formula (IV) 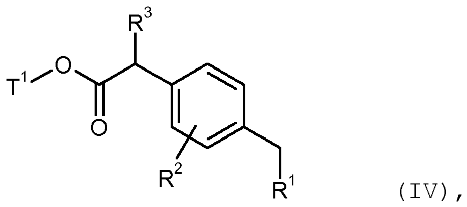 in which R¹, R², R³ and T¹ each have the meanings given above,
or
[B] a compound of the formula (V) 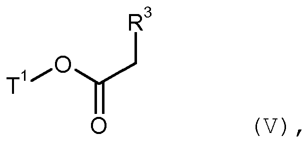 in which R³ has the meaning given in any of Claims 1 to 4
and
T¹ represents (C₁-C₄)-alkyl,
is, in an inert solvent, after deprotonation with a base, reacted with a compound of the formula (VI) 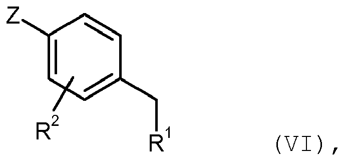 in which R¹ and R² have the meanings given in any of Claims 1 to 4
and
Z represents chlorine, bromine or iodine,
in the presence of a suitable palladium catalyst, likewise to give a compound of the formula (IV) 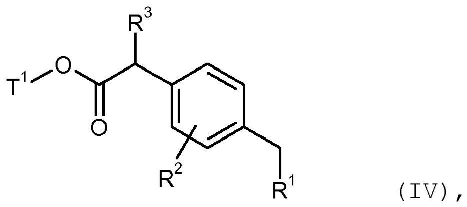 in which R¹, R², R³ and T¹ each have the meanings given above,
the compound of the formula (IV) is then brominated in an inert solvent with elemental bromine or with N-bromosuccinimide to give a compound of the formula (VII) 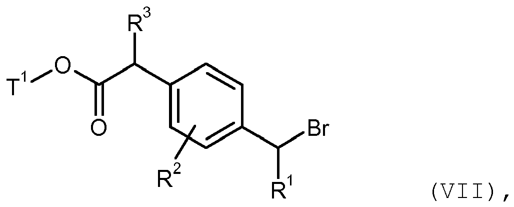 in which R¹, R², R³ and T¹ each have the meanings given above,
and then reacted in an inert solvent in the presence of a base with a compound of the formula (VIII) 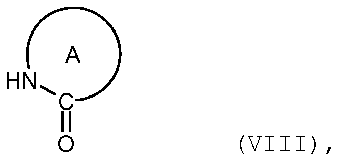 in which ring A represents an oxo-substituted azaheterocycle, as defined in any of Claims 1 to 4,
to give a compound of the formula (IX) 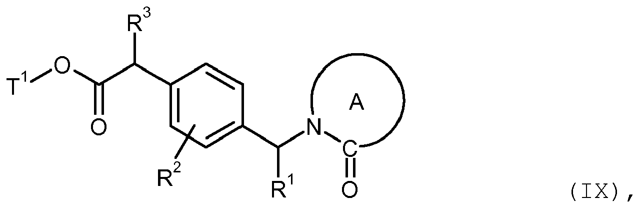 in which ring A, R¹, R², R³ and T¹ each have the meanings given above,
the ester radical T¹ in (IX) is then removed under basic or acidic conditions, the resulting carboxylic acid of the formula (X) 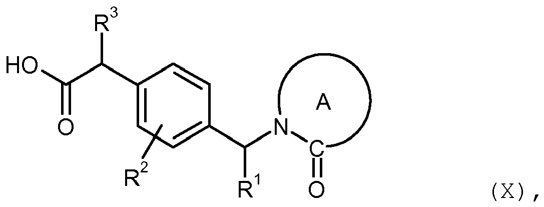 in which ring A, R¹, R² and R³ each have the meanings given above,
is then coupled in an inert solvent in the presence of a condensing agent or via the intermediate of the corresponding carbonyl chloride in the presence of a base with an amine of the formula (XI) 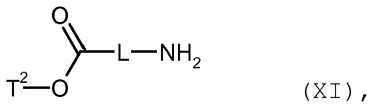 in which L has the meaning given in any of Claims 1 to 4
and
T² represents (C₁-C₄)-alkyl,
to give a compound of the formula (XII) 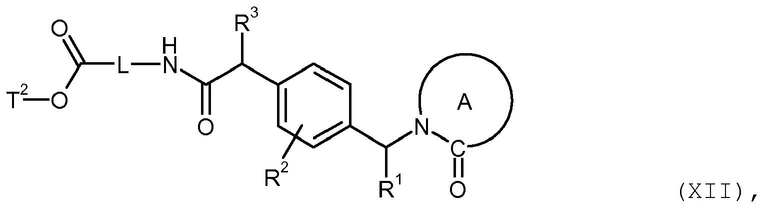 in which ring A, R¹, R², R³, L and T² each have the meanings given above,
and the ester radical T² in (XII) is then removed by further basic or acidic solvolysis to give the carboxylic acid of the formula (I)
and the compounds of the formula (I) are separated where appropriate by methods known to the skilled person into their enantiomers and/or diastereomers, and/or where appropriate reacted with the appropriate (i) solvents and/or (ii) bases or acids to give the solvates, salts and/or solvates of the salts thereof.

6. Compound as defined in any of Claims 1 to 4 for use in the treatment and/or prevention of diseases.

7. Compound as defined in any of Claims 1 to 4 for use in a method for the treatment and/or prevention of heart failure, angina pectoris, hypertension, pulmonary hypertension, ischemias, vascular disorders, thromboembolic disorders and arteriosclerosis.

8. Use of a compound as defined in any of Claims 1 to 4 for producing a medicament for the treatment and/or prevention of heart failure, angina pectoris, hypertension, pulmonary hypertension, ischemias, vascular disorders, thromboembolic disorders and arteriosclerosis.

9. Medicament comprising a compound as defined in any of Claims 1 to 4 in combination with one or more inert, non-toxic, pharmaceutically suitable excipients.

10. Medicament comprising a compound as defined in any of Claims 1 to 4 in combination with one or more further active ingredients selected from the group consisting of organic nitrates, NO donors, cGMP-PDE inhibitors, stimulators of guanylate cyclase, agents having antithrombotic activity, agents lowering blood pressure, and agents altering lipid metabolism.

11. Medicament according to Claim 9 or 10 for use in the treatment and/or prevention of heart failure, angina pectoris, hypertension, pulmonary hypertension, ischemias, vascular disorders, thromboembolic disorders and arteriosclerosis.

## Revendications

1. Composé de formule (I) 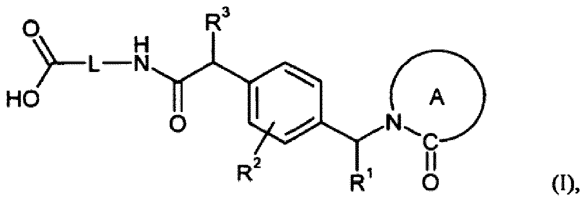 dans laquelle
le cycle A représente un azahétérocycle à substitution oxo, saturé ou partiellement insaturé, de 5 à 7 éléments, relié à N, qui
(i) peut contenir un ou deux hétéroatomes supplémentaires de la série N, O et/ou S en tant qu'éléments de cycle,
(ii) est substitué avec un radical choisi dans la série fluor, chlore, alkyle en (C₁-C₆), trifluorométhyle, cycloalkyle en (C₃-C₇), hétérocyclyle de 4 à 7 éléments et phényle, ou qui est benzo-annelé,
le substituant phényle et le cycle phényle annelé pouvant être substitués de leur côté jusqu'à deux fois, de manière identique ou différente, avec un radical choisi dans la série halogène, cyano, alkyle en (C₁-C₄), alcényle en (C₂-C₄), trifluorométhyle, alcoxy en (C₁-C₄) et trifluorométhoxy,
et
(iii) peut également être substitué jusqu'à deux fois, de manière identique ou différente, avec des radicaux supplémentaires choisis dans la série fluor, chlore, alkyle en (C₁-C₆), trifluorométhyle, oxo, cycloalkyle en (C₃-C₇), hétérocyclyle de 4 à 7 éléments et phényle,
le phényle pouvant de son côté être substitué jusqu'à deux fois, de manière identique ou différente, avec un radical choisi dans la série halogène, cyano, alkyle en (C₁-C₄), alcényle en (C₂-C₄), trifluorométhyle, alcoxy en (C₁-C₄) et trifluorométhoxy,
R¹ représente hydrogène, alkyle en (C₁-C₄) ou cyclopropyle,
R² représente hydrogène, halogène, cyano, alkyle en (C₁-C₄) ou trifluorométhyle,
R³ représente alkyle en (C₃-C₆) ou alcényle en (C₃-C₆), qui peuvent chacun être substitués avec cyano, alcoxy en (C₁-C₄) ou trifluorométhoxy et jusqu'à six fois avec fluor,
ou
cycloalkyle en (C₃-C₇) ou cycloalcényle en (C₃-C₇), qui peuvent chacun être substitués jusqu'à deux fois, de manière identique ou différente, avec alkyle en (C₁-C₄), trifluorométhyle et alcoxy en (C₁-C₄), et jusqu'à quatre fois avec fluor,
ou
oxétanyle, tétrahydrofuranyle ou tétrahydropyranyle,
et
L représente alcanediyle en (C₃-C₇) ou alcènediyle en (C₃-C₇) linéaire, qui peuvent chacun être substitués jusqu'à quatre fois, de manière identique ou différente, avec un radical R⁴,
R⁴ signifiant fluor, trifluorométhyle ou alkyle en (C₁-C₄),
ou
deux radicaux R⁴ reliés au même atome de carbone étant reliés l'un avec l'autre et formant ensemble avec cet atome de carbone un cycle cycloalcane-1,1-diyle en (C₃-C₆),
ainsi que leurs sels, solvates et solvates des sels.

2. Composé de formule (I) selon la revendication 1, dans lequel
le cycle A représente un azahétérocycle à substitution oxo de formule 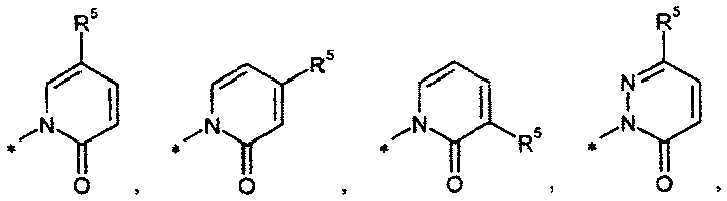 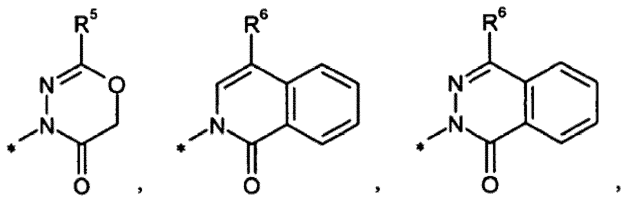 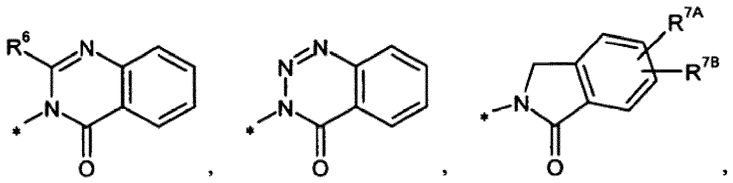 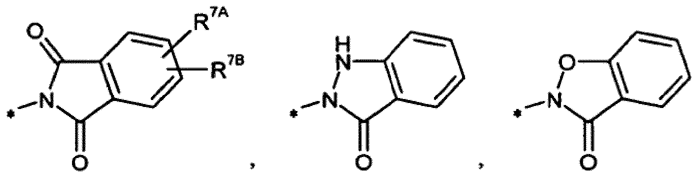 ou 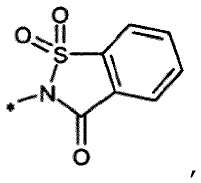 dans lesquelles
* désigne la position de liaison avec la partie restante de la molécule,
R⁵ signifie chlore, alkyle en (C₁-C₆), trifluorométhyle, cycloalkyle en (C₃-C₆), hétérocyclyle de 4 à 6 éléments ou phényle, le phényle pouvant de son côté être substitué jusqu'à deux fois, de manière identique ou différente, avec un radical choisi dans la série fluor, chlore, brome, cyano, alkyle en (C₁-C₄), vinyle, trifluorométhyle, alcoxy en (C₁-C₄) et trifluorométhoxy,
R⁶ signifie hydrogène ou a la signification indiquée précédemment pour R⁵
et
R^{7A} et R^{7B} signifient indépendamment l'un de l'autre hydrogène, fluor ou chlore,
ainsi que leurs sels, solvates ou solvates des sels.

3. Composé de formule (I) selon la revendication 1 ou 2, dans lequel
le cycle A représente un azahétérocycle à substitution oxo de formule 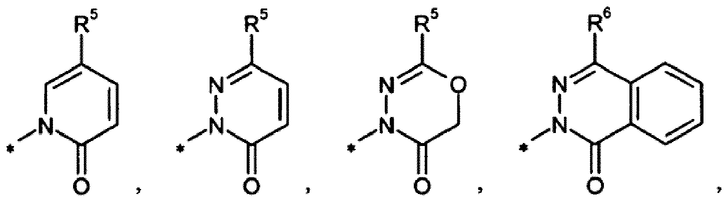 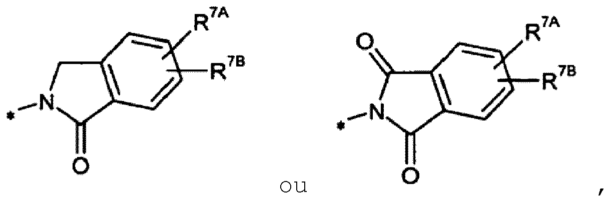 dans lesquelles
* désigne la position de liaison avec la partie restante de la molécule,
R⁵ signifie chlore, alkyle en (C₁-C₆), trifluorométhyle, cycloalkyle en (C₃-C₆) ou phényle, le phényle pouvant de son côté être substitué jusqu'à deux fois, de manière identique ou différente, avec un radical choisi dans la série fluor, chlore, cyano, alkyle en (C₁-C₄), trifluorométhyle, alcoxy en (C₁-C₄) et trifluorométhoxy,
R⁶ signifie hydrogène ou a la signification indiquée précédemment pour R⁵
et
R^{7A} et R^{7B} signifient indépendamment l'un de l'autre hydrogène, fluor ou chlore,
R¹ représente hydrogène ou alkyle en (C₁-C₄),
R² représente hydrogène, fluor, chlore ou trifluorométhyle,
R³ représente alkyle en (C₃-C₆) ou alcényle en (C₃-C₆), qui peuvent chacun être substitués avec cyano, méthoxy, éthoxy ou trifluorométhoxy, et jusqu'à six fois avec fluor,
ou
cycloalkyle en (C₃-C₆) ou cycloalcényle en (C₄-C₆), qui peuvent chacun être substitués jusqu'à deux fois, de manière identique ou différente, avec méthyle, éthyle et trifluorométhyle, et jusqu'à quatre fois avec fluor,
ou oxétanyle, et
L représente alcanediyle en (C₃-C₆) ou alcènediyle en (C₃-C₆) linéaire, qui peuvent chacun être substitués jusqu'à quatre fois, de manière identique ou différente, avec un radical R⁴,
R⁴ signifiant fluor, trifluorométhyle, méthyle ou éthyle,
ou
deux radicaux R⁴ reliés au même atome de carbone étant reliés l'un avec l'autre et formant ensemble avec cet atome de carbone un cycle cyclopropane-1,1-diyle ou cyclobutane-1,1-diyle,
ainsi que leurs sels, solvates ou solvates des sels.

4. Composé de formule (I) selon la revendication 1, 2 ou 3, dans lequel
le cycle A représente un azahétérocycle à substitution oxo de formule 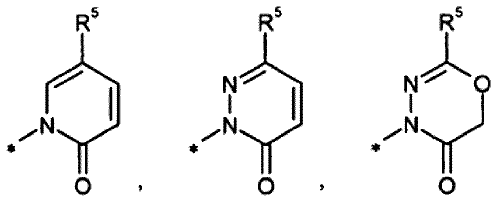 ou 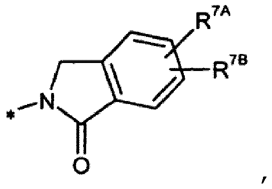 dans lesquelles
* désigne la position de liaison avec la partie restante de la molécule,
R⁵ signifie chlore, trifluorométhyle ou phényle, phényle pouvant de son côté être substitué jusqu'à deux fois, de manière identique ou différente, avec un radical choisi dans la série fluor, chlore, méthyle et trifluorométhyle,
et
R^{7A} et R^{7B} signifient indépendamment l'un de l'autre hydrogène ou fluor,
R¹ représente hydrogène,
R² représente hydrogène,
R³ représente propan-2-yle, butan-2-yle, pentan-2-yle, 3,3,3-trifluoropropan-1-yle, 1,1,1-trifluoropropan-2-yle, 1,1,1-trifluorobutan-2-yle, 4,4,4-trifluorobutan-2-yle, 4,4,4-trifluoro-2-méthylbutan-1-yle, cyclopentyle ou 3,3-difluorocyclopentyle,
et
L représente alcanediyle en (C₃-C₆) ou alcènediyle en (C₃-C₆) linéaire, qui peuvent chacun être substitués jusqu'à quatre fois, de manière identique ou différente, avec un radical R⁴,
R⁴ représentant méthyle,
ou
deux radicaux R⁴ reliés au même atome de carbone étant reliés l'un avec l'autre et formant ensemble avec cet atome de carbone un cycle cyclopropane-1,1-diyle,
ainsi que leurs sels, solvates ou solvates des sels.

5. Procédé de fabrication d'un composé de formule (I), tel que défini dans les revendications 1 à 4, **caractérisé en ce que** tout d'abord soit
[A] un composé de formule (II) 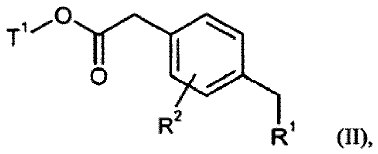 dans laquelle R¹ et R² ont les significations indiquées dans les revendications 1 à 4,
et
T¹ représente alkyle en (C₁-C₄),
est transformé dans un solvant inerte en présence d'une base avec un composé de formule (III)
R³-X (III),
dans laquelle R³ a la signification indiquée dans les revendications 1 à 4,
et
X représente un groupe partant tel que par exemple halogène, mésylate, tosylate ou triflate, en un composé de formule (IV) 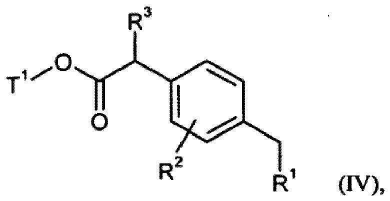
dans laquelle R¹, R², R³ et T¹ ont chacun les significations indiquées précédemment,
soit
[B] un composé de formule (V) 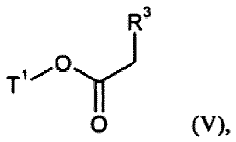 dans laquelle R³ a la signification indiquée dans les revendications 1 à 4
et
T¹ représente alkyle en (C₁-C₄),
est transformé dans un solvant inerte, après déprotonation au moyen d'une base, avec un composé de formule (VI) 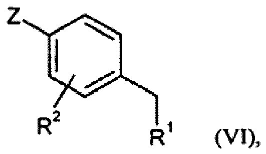 dans laquelle R¹ et R² ont les significations indiquées dans les revendications 1 à 4,
et
Z représente chlore, brome ou iode,
en présence d'un catalyseur de palladium approprié également en un composé de formule (IV) 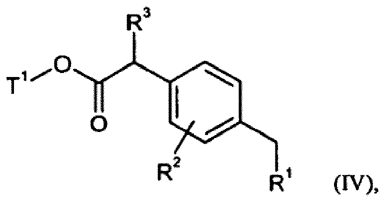 dans laquelle R¹, R², R³ et T¹ ont chacun les significations indiquées précédemment,
le composé de formule (IV) est ensuite bromé dans un solvant inerte avec du brome élémentaire ou du N-bromosuccinimide en un composé de formule (VII) 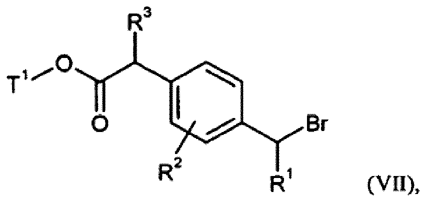 dans laquelle R¹, R², R³ et T¹ ont chacun les significations indiquées précédemment,
puis transformé dans un solvant inerte en présence d'une base avec un composé de formule (VIII) 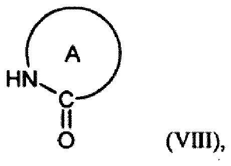 dans laquelle le cycle A représente un azahétérocycle à substitution oxo tel que défini dans les revendications 1 à 4,
en un composé de formule (IX) 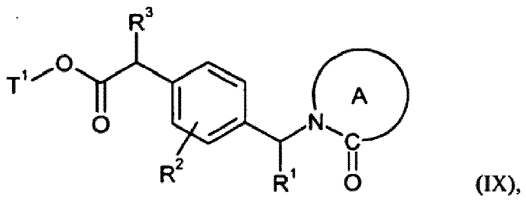 dans laquelle le cycle A, R¹, R², R³ et T¹ ont chacun les significations indiquées précédemment,
puis le radical ester T¹ dans (IX) est clivé en conditions basiques ou acides, l'acide carboxylique résultant de formule (X) 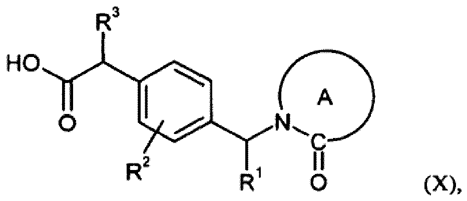 dans laquelle le cycle A, R¹, R² et R³ ont chacun les significations indiquées précédemment,
est ensuite couplé dans un solvant inerte en présence d'un agent de condensation ou par l'intermédiaire du chlorure d'acide carboxylique correspondant en présence d'une base avec une amine de formule (XI) 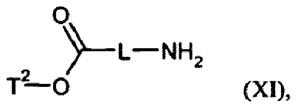 dans laquelle L a la signification indiquée dans les revendications 1 à 4
et
T² représente alkyle en (C₁-C₄),
en un composé de formule (XII) 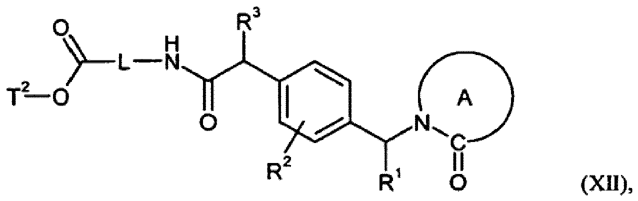 dans laquelle le cycle A, R¹, R², R³, L et T² ont chacun les significations indiquées précédemment,
puis le radical ester T² dans (XII) est clivé par une nouvelle solvolyse basique ou acide pour obtenir l'acide carboxylique de formule (I)
et les composés de formule (I) sont éventuellement séparés par des procédés connus de l'homme du métier en leurs énantiomères et/ou diastéréomères, et/ou éventuellement transformés avec les (i) solvants et/ou (ii) bases ou acides appropriés en leurs solvates, sels et/ou solvates des sels.

6. Composé, tel que défini dans l'une quelconque des revendications 1 à 4, destiné à être utilisé pour le traitement et/ou la prévention de maladies.

7. Composé, tel que défini dans l'une quelconque des revendications 1 à 4, destiné à être utilisé dans un procédé de traitement et/ou de prévention de l'insuffisance cardiaque, de l'angine de poitrine, de l'hypertension, de l'hypertension pulmonaire, des ischémies, des maladies vasculaires, des maladies thromboemboliques et de l'artériosclérose.

8. Utilisation d'un composé, tel que défini dans l'une quelconque des revendications 1 à 4, pour la fabrication d'un médicament pour le traitement et/ou la prévention de l'insuffisance cardiaque, de l'angine de poitrine, de l'hypertension, de l'hypertension pulmonaire, des ischémies, des maladies vasculaires, des maladies thromboemboliques et de l'artériosclérose.

9. Médicament contenant un composé, tel que défini dans l'une quelconque des revendications 1 à 4, en combinaison avec un ou plusieurs adjuvants inertes, non toxiques et pharmaceutiquement appropriés.

10. Médicament contenant un composé, tel que défini dans l'une quelconque des revendications 1 à 4, en combinaison avec un ou plusieurs agents actifs supplémentaires choisis dans le groupe constitués par les nitrates organiques, les donneurs de NO, les inhibiteurs de cGMP-PDE, les stimulateurs de guanylate cyclase, les agents antithrombotiques, les agents abaissant la tension artérielle et les agents modifiant le métabolisme lipidique.

11. Médicament selon la revendication 9 ou 10, destiné à être utilisé pour le traitement et/ou la prévention de l'insuffisance cardiaque, de l'angine de poitrine, de l'hypertension, de l'hypertension pulmonaire, des ischémies, des maladies vasculaires, des maladies thromboemboliques et de l'artériosclérose.
